(19) Europäisches Patentamt
European Patent Office
Office européen des brevets

(11) EP 2 295 446 A1

(12) EUROPÄISCHE PATENTANMELDUNG

(43) Veröffentlichungstag:
**16.03.2011 Patentblatt 2011/11**

(21) Anmeldenummer: **10181802.9**

(22) Anmeldetag: **27.05.2004**

(51) Int Cl.:
***C07K 14/435*** (2006.01) ***C07K 14/47*** (2006.01)
***C12N 15/09*** (2006.01) ***C12N 15/62*** (2006.01)

(84) Benannte Vertragsstaaten:
**AT BE BG CH CY CZ DE DK EE ES FI FR GB GR HU IE IT LI LU MC NL PL PT RO SE SI SK TR**

(30) Priorität: **28.05.2003 DE 10324447**

(62) Dokumentnummer(n) der früheren Anmeldung(en) nach Art. 76 EPÜ:
**04735010.3 / 1 626 985**

(71) Anmelder: **Scil Proteins GmbH
06120 Halle/Saale (DE)**

(72) Erfinder:
- **Fiedler, Markus
06120, Halle (DE)**
- **Fiedler, Ulrike
06114, Halle (DE)**
- **Rudolph, Rainer
06120, Halle (DE)**

(74) Vertreter: **Behnisch, Werner
Reinhard, Skuhra, Weise & Partner GbR
Patent- und Rechtsanwälte
Friedrichstrasse 31
80801 München (DE)**

Bemerkungen:
Diese Anmeldung ist am 29-09-2010 als Teilanmeldung zu der unter INID-Code 62 erwähnten Anmeldung eingereicht worden.

# (54) Generierung künstlicher Bindungsproteine auf der Grundlage von Ubiquitin-Proteinen

(57) Die vorliegende Erfindung beschreibt modifizierte Proteine der Protein-Superfamilie "ubiquitin-like proteins", Proteine, die ein Ubiquitin-artiges Faltungsmotiv aufweisen sowie Fragmente oder Fusionsproteine hiervon, wobei die Proteine aufgrund dieser Modifikation eine vorher nicht vorhandene Bindungsaffinität gegenüber einem vorbestimmten Bindungspartner aufweist, sowie ein Verfahren zu ihrer Herstellung und ihre Verwendung.

EP 2 295 446 A1

SEQUENCE LISTING

<110> Scil Proteins GmbH

<120> Generierung künstlicher Bindungsproteine auf der Grundlage von Ubiquitin

<130> P28025-EP01

<150> DE 103 24 447.6
<151> 2003-05-23

<160> 34

<170> PatentIn version 3.1

<210> 1
<211> 291
<212> DNA
<213> Artificial Sequence

<220>
<223> DNA-Sequenz für ein modifiziertes Ubiquitin-Proteingerüst
<400> 1
```
atgaaatacc tattgcctac ggcagccgct ggattgttat tactcgcggc ccagccggcc    60
atggccatgc aaatcttcgt taaaaccctg acgggaaaga ctatcaccct ggaggtagaa   120
ccgtccgaca ccatcgaaaa tgtcaaagct aaaatccaag acaaagaagg aattccacct   180
gaccagcaac gcctagcttt cgcaggacga caactagagg acgggctcac cctgtctgac   240
tacaacatcc aaaaagaatc caccctccac ctggcactcc tcctgcgggc c            291
```

<210> 2
<211> 50
<212> DNA
<213> Artificial Sequence

<220>

<223> Template

<400> 2
atgcaaatct tcgttaaaac cctgacggga aagactatca ccctggaggt 50

<210> 3

<211> 53

<212> DNA

<213> Artificial Sequence

<220>

<223> Template

<400> 3
ggattttagc tttgacattt tcgatggtgt cggacggttc tacctccagg gtg 53

<210> 4

<211> 53

<212> DNA

<213> Artificial Sequence

<220>

<223> Template

<400> 4
gtcaaagcta aaatccaaga caaagaagga attccacctg accagcaacg cct 53

<210> 5

<211> 50

<212> DNA

<213> Artificial Sequence

<220>

<223> Template

<400> 5
gggtgagccc gtcctctagt tgtcgtcctg cgaaagctag gcgttgctgg 50

<210> 6

<211> 50

<212> DNA

<213> Artificial Sequence

<220>

<223> Template

<400> 6
gacgggctca ccctgtctga ctacaacatc caaaaagaat ccaccctcca 50

<210> 7

<211> 39

<212> DNA

<213> Artificial Sequence

<220>

<223> Template

<400> 7
gagtgctcgc agcaggagtg ccaggtggag ggtggattc 39

<210> 8

<211> 22

<212> DNA

<213> Artificial Sequence

<220>

<223> Primer

<400> 8
gatatacata tgcaaatctt cg 22

<210> 9

<211> 18

<212> DNA

<213> Artificial Sequence

<220>

<223> Primer

<400> 9
gtggtgctcg agtgctcg 18

<210> 10

<211> 55

<212> DNA
<213> Artificial Sequence

<220>
<223> Primer
<220>
<221> misc_feature
<222> (20)..(21)
<223> a, g, c or t

<220>
<221> misc_feature
<222> (26)..(27)
<223> a, g, c or t

<220>
<221> misc_feature
<222> (32)..(33)
<223> a, g, c or t

<400> 10
ccagccggcc atggccatgn nkatcnnkgt tnnkaccctg acgggaaaga ctatc 55

<210> 11
<211> 55
<212> DNA
<213> Artificial Sequence

<220>
<223> Primer
<220>
<221> misc_feature
<222> (23)..(24)
<223> a, g, c or t

<220>

<221> misc_feature

<222> (29)..(30)

<223> a, g, c or t

<220>

<221> misc_feature

<222> (32)..(33)

<223> a, g, c or t

<220>

<221> misc_feature

<222> (35)..(36)

<223> a, g, c or t

<220>

<221> misc_feature

<222> (26)..(27)

<223> a, g, c or t

<400> 11
caggaggagt gccaggtgga gmnnmnnmnn mnnmnngatg ttgtagtcag acagg 55

<210> 12

<211> 34

<212> DNA

<213> Artificial Sequence

<220>

<223> Primer

<400> 12
gttattactc gcggcccagc cggccatggc catg 34

<210> 13

<211> 48

<212> DNA

<213> Artificial Sequence

<220>

<223> Primer

<400> 13
gagtttttgt tcggcctcga gggcccgcag gaggagtgcc aggtggag 48

<210> 14

<211> 23

<212> DNA

<213> Artificial Sequence

<220>

<223> Primer

<400> 14
accactccct atcagtgata gag 23

<210> 15

<211> 228

<212> DNA

<213> Artificial Sequence

<220>

<223> modifizierte Ubiquitin-Sequenz als Grundlage für Bibliothek

<400> 15
atgcagatct tcgtgaagac cctgaccggc aagaccatca ctctggaggt ggagcccagt 60

gacaccatcg aaaatgtgaa ggccaagatc caagataaag aaggcattcc ccccgaccag 120

cagaggctca tctgggcagg caagcagctg gaagatggcc gcactctttc tgactacaac 180

atccagaaag agtcgaccct gcacctggtc ctccgcctga ggggcggc 228

<210> 16

<211> 54

<212> DNA

<213> Artificial Sequence

<220>

<223> Primer

<220>
<221> misc_feature
<222> (19)..(20)
<223> a, c, g or t

<220>
<221> misc_feature
<222> (25)..(26)
<223> a, c, g or t

<220>
<221> misc_feature
<222> (31)..(32)
<223> a, c, g or t

<400> 16
gaaggagata tacatatgnn katcnnkgtt nnkaccctga cgggaaagac tatc 54

<210> 17
<211> 55
<212> DNA
<213> Artificial Sequence

<220>
<223> Primer
<220>
<221> misc_feature
<222> (23)..(24)
<223> a, c, g or t

<220>
<221> misc_feature
<222> (26)..(27)
<223> a, c, g or t

<220>
<221> misc_feature
<222> (29)..(30)
<223> a, c, g or t

<220>
<221> misc_feature
<222> (32)..(33)
<223> a, c, g or t

<220>
<221> misc_feature
<222> (35)..(36)
<223> a, c, g or t

<400> 17
caggaggagt gccaggtgga gmnnmnnmnn mnnmnngatg ttgtagtcag acagg 55

<210> 18
<211> 54
<212> DNA
<213> Artificial Sequence

<220>
<223> Primer
<220>
<221> misc_feature
<222> (19)..(20)
<223> a, c, g or t

<220>
<221> misc_feature
<222> (25)..(26)
<223> a, c, g or t

<220>

<221> misc_feature

<222> (31)..(32)

<223> a, c, g or t

<400> 18
gaaggagata tacatatgnn katcnnkgtt nnkaccctga ccggcaagac catc 54

<210> 19

<211> 61

<212> DNA

<213> Artificial Sequence

<220>

<223> Primer

<220>

<221> misc_feature

<222> (23)..(24)

<223> a, c, g or t

<220>

<221> misc_feature

<222> (26)..(27)

<223> a, c, g or t

<220>

<221> misc_feature

<222> (29)..(30)

<223> a, c, g or t

<220>

<221> misc_feature

<222> (32)..(33)

<223> a, c, g or t

<220>

<221> misc_feature

<222> (35)..(36)

<223> a, c, g or t

<400> 19
caggaggagt gccaggtgga gmnnmnnmnn mnnmnngatg ttgtagtcag aaagagtgcg 60

g 61

<210> 20

<211> 33

<212> DNA

<213> Artificial Sequence

<220>

<223> Primer

<400> 20
caccaccacc accaccaccc tcctgtcaat gct 33

<210> 21

<211> 35

<212> DNA

<213> Artificial Sequence

<220>

<223> Primer

<400> 21
ggcccacccg tgaaggtgag cctcagtagc gacag 35

<210> 22

<211> 45

<212> DNA

<213> Artificial Sequence

<220>

<223> Primer

<400> 22
ctggcactcc tcctgcgggc cctcgagcac caccaccacc accac 45

<210> 23

<211> 63

<212> DNA

<213> Artificial Sequence

<220>

<223> Primer

<400> 23
agaccacaac ggtttccctc tagaaataat tttgtttaac tttaagaagg agatatacat 60

atg 63

<210> 24

<211> 42

<212> DNA

<213> Artificial Sequence

<220>

<223> Primer

<400> 24
atacgaaatt aatacgactc actataggga gacccacaac gg 42

<210> 25

<211> 55

<212> DNA

<213> Artificial Sequence

<220>

<223> Primer

<220>

<221> misc_feature

<222> (32)..(33)

<223> a, c, g or t

<220>

<221> misc_feature

<222> (35)..(36)

<223> a, c, g or t

<400> 25
caggaggagt gccaggtgga gattcctacc mnnmnngatg ttgtagtcag acagg 55

<210> 26

<211> 55

<212> DNA

<213> Artificial Sequence

<220>

<223> Primer

<220>

<221> misc_feature

<222> (26)..(27)

<223> a, c, g or t

<220>

<221> misc_feature

<222> (29)..(30)

<223> a, c, g or t

<400> 26
caggaggagt gccaggtgga gattmnnmnn cggcgggatg ttgtagtcag acagg 55

<210> 27

<211> 55

<212> DNA

<213> Artificial Sequence

<220>

<223> Oligodesoxynukleotid

<400> 27
ctcgagcacc accaccacca ccactgtccg tgagatccgg ctgctaacaa agccc 55

<210> 28

<211> 55

<212> DNA

<213> Artificial Sequence

<220>

<223> Oligodesoxynukleotid

<400> 28
gggctttgtt agcagccgga tctcacggac agtggtggtg gtggtggtgc tcgag 55

<210> 29

<211> 34

<212> DNA

<213> Artificial Sequence

<220>

<223> Konstruktion einer Bibliothek von Ubiquitin-Varianten

<400> 29
gttattactc gcggcccagc cggccatggc catg 34

<210> 30

<211> 55

<212> DNA

<213> Artificial Sequence

<220>

<223> Konstruktion einer Bibliothek von Ubiquitin-Varianten

<220>

<221> misc_feature

<222> (20)..(21)

<223> a, c, g or t

<220>

<221> misc_feature

<222> (26)..(27)

<223> a, c, g or t

<220>

<221> misc_feature

<222> (32)..(33)

<223> a, c, g or t

<400> 30
ccagccggcc atggccatgn nkatcnnkgt tnnkaccctg acgggaaaga ctatc 55

<210> 31

<211> 114

<212> PRT

<213> Artificial Sequence

<220>

<223> Konstruktion einer Bibliothek von Ubiquitin-Varianten

<220>

<221> MISC_FEATURE

<222> (24)..(24)

<223> any amino acid

<220>

<221> MISC_FEATURE

<222> (26)..(26)

<223> any amino acid

<220>

<221> MISC_FEATURE

<222> (28)..(28)

<223> any amino acid

<220>

<221> MISC_FEATURE

<222> (84)..(88)

<223> any amino acid

<400> 31

Met Lys Tyr Leu Leu Pro Thr Ala Ala Ala Gly Leu Leu Leu Leu Ala
1               5                   10                  15

Ala Gln Pro Ala Met Ala Met Xaa Ile Xaa Val Xaa Thr Leu Thr Gly
            20                  25                  30

Lys Thr Ile Thr Leu Glu Val Glu Pro Ser Asp Thr Ile Glu Asn Val
        35                  40                  45

Lys Ala Lys Ile Gln Asp Lys Glu Gly Ile Pro Pro Asp Gln Gln Arg
    50                  55                  60

Leu Ala Phe Ala Gly Arg Gln Leu Glu Asp Gly Leu Thr Leu Ser Asp
65                  70                  75                  80

Tyr Asn Ile Xaa Xaa Xaa Xaa Xaa Leu His Leu Ala Leu Leu Leu Arg
                85                  90                  95

Ala Leu Glu Ala Glu Gln Lys Leu Ile Ser Glu Glu Asn Leu Tyr Phe
            100                 105                 110

Gln Gly

<210> 32

<211> 345

<212> DNA

<213> Artificial Sequence

<220>

<223> Konstruktion einer Bibliothek von Ubiquitin-Varianten

<400> 32
atgaaatacc tattgcctac ggcagccgct ggattgttat tactcgcggc ccagccggcc 60

atggccatgc aaatcttcgt taaaaccctg acgggaaaga ctatcaccct ggaggtagaa 120

ccgtccgaca ccatcgaaaa tgtcaaagct aaaatccaag acaaagaagg aattccacct 180

gaccagcaac gcctagcttt cgcaggacga caactagagg acgggctcac cctgtctgac 240

tacaacatcc aaaaagaatc caccctccac ctggcactcc tcctgcgggc cctcgaggcc 300

gaacaaaaac tcatctcaga agagaatctg tatttccagg gctag 345

<210> 33

<211> 51

<212> DNA

<213> Artificial Sequence

<220>

<223> Konstruktion einer Bibliothek von Ubiquitin-Varianten

<220>

<221> misc_feature

<222> (16)..(17)

<223> a, c, g or t

<220>

<221> misc_feature

<222> (19)..(20)

<223> a, c, g or t

<220>

<221> misc_feature

<222> (22)..(23)

<223> a, c, g or t

<220>

<221> misc_feature

<222> (25)..(26)

<223> a, c, g or t

<220>

<221> misc_feature

<222> (28)..(29)

<223> a, c, g or t

<400> 33
agactgatgt tgtagnnmnn mnnmnnmnnm gaggtggacc gtgaggagga c 51

<210> 34

<211> 48

<212> DNA

<213> Artificial Sequence

<220>

<223> Konstruktion einer Bibliothek von Ubiquitin-Varianten

<400> 34
gaggtggacc gtgaggagga cgcccgggag ctccggcttg tttttgag 48

## Beschreibung

Die vorliegende Erfindung betrifft modifizierte Proteine der Protein-Superfamilie "ubiquitin-like proteins", Proteine, die ein Ubiquitin-artiges Faltungsmotiv aufweisen sowie Fragmente oder Fusionsproteine hiervon, wobei das Protein aufgrund dieser Modifikation eine vorher nicht vorhandene Bindungsaffinität gegenüber einem vorbestimmten Bindungspartner aufweist, sowie Verfahren zur Herstellung dieser Proteine und deren Verwendung

Ubiquitin ist ein kleines, monomeres und zytosolisches Protein, das - in seiner Sequenz hochkonserviert - von den Protozoen bis zu den Vertebraten in allen bekannten eukaryotischen Zellen vorkommt. Es spielt im Organismus eine grundlegende Rolle bei der Regulation des kontrollierten Abbaus zelleigener Proteine. Hierbei werden die zum Abbau bestimmten Proteine beim Durchlaufen einer Enzymkaskade kovalent mit Ubiquitin oder Polyubiquitin-Ketten verknüpft und aufgrund dieser Markierung selektiv abgebaut. Nach neueren Erkenntnissen spielt Ubiquitin bzw. die Markierung von Proteinen mit Ubiquitin auch bei anderen zellulären Prozessen, wie beim Import mancher Proteine oder deren Genregulation eine wichtige Rolle (Marx, 2002).

Neben der Aufklärung seiner physiologischen Funktion ist Ubiquitin vor allem wegen seiner strukturellen und proteinchemischen Eigenschaften Objekt der Forschung. Die Polypeptidkette des Ubiquitins besteht aus 76 Aminosäuren, die in einer äußerst kompakten alpha/beta-Struktur gefaltet sind (Vijay-Kumar, 1987): Nahezu 87 % der Polypeptidkette sind durch Wasserstoffbrücken an der Ausbildung der Sekundärstrukturelemente beteiligt. Als prominente Sekundärstrukturen können drei-einhalb alpha-helikale Windungen sowie ein aus vier Strängen bestehendes, antiparalleles beta-Faltblatt gelten. Die charakteristische Anordnung dieser Elemente - ein zu einer Oberfläche des Proteins exponiertes antiparalleles beta-Faltblatt, welches auf seiner Rückseite von einer senkrecht darüber liegenden alpha-Helix bedeckt wird, - gilt generell als sogenanntes Ubiquitin-artiges Faltungsmotiv. Ubiquitin ist daher namensgebend für die entsprechende Protein-Superfamilie ("ubiquitin-like proteins") bzw. Protein-Familie ("ubiquitin-related Proteins") (Murzin *et al.,* 1995), welche Proteine wie z. B. SUMO-1 (Müller *et al.,* 2001), FAU (Michiels *et. al.,* 1993), NEDD-8 (Kumar *et al.,* 1993), UBL-1 (Jones und Candino, 1993) und GDX (Filippi *et al.,* 1990), die dieses Motiv und einen hohen Identitätsgrad zu Ubiquitin in ihrer Primärsequenz aufweisen, umfassen. Ein weiteres Merkmal der Struktur ist ein ausgeprägter hydrophober Bereich im Inneren des Proteins zwischen alpha-Helix und beta-Faltblatt.

Die künstliche Herstellung von Ubiquitin ist aufgrund der geringen Größe sowohl durch chemische Synthese, als auch mittels biotechnologischer Verfahren möglich. Wegen der günstigen Faltungseigenschaften kann Ubiquitin bei der gentechnischen Gewinnung mit Hilfe von Mikroorganismen wie z. B. *Escherichia coli* in verhältnismäßig großen Mengen wahlweise im Zytosol oder dem periplasmatischem Raum produziert werden. Letztere Strategie ist aufgrund des im Periplasma vorherrschenden oxidierenden Milieus gewöhnlich der Produktion sekretorischer Proteine vorbehalten. Die einfache und effiziente bakterielle Herstellung ermöglicht die Verwendung von Ubiquitin als Fusionspartner für andere herzustellende Fremdproteine, deren Produktion problematisch ist. Durch die Fusion mit Ubiquitin kann eine verbesserte Löslichkeit und damit eine verbesserte Ausbeute erzielt werden. Der in der vorliegenden Erfindung realisierte Ansatz, Ubiquitin als universelles künstliches Bindungsprotein bereitzustellen, ermöglicht eine völlig neuartige Ausnutzung seiner proteinchemischen Eigenschaften.

Unter denjenigen Proteinen, deren natürliche Funktion für künstliche Anwendungen - etwa in der Biotechnologie, der Bioanalytik oder der Medizin - genutzt wird, nehmen Antikörper (d. h. die Immunglobuline) eine herausragende Stellung ein. Aufgrund ihrer Fähigkeit zur spezifischen, nicht-kovalenten Bindung annähernd jeder beliebigen Substanz stellen sie für nahezu jede biowissenschaftliche Anwendung, die eine Erkennung, Bindung oder Abtrennung von Liganden, Rezeptoren oder sonstigen Zielmolekülen erfordert, das wichtigste Werkzeug dar. Die in den letzten Jahren entwickelten Methoden zur funktionellen Biosynthese von Antikörperfragmenten in *E. coli* haben die Anwendbarkeit der Immunglobuline zudem erweitert, gleichzeitig jedoch auch Schwierigkeiten und Grenzen sichtbar gemacht. Neben den im Prinzip auch durch konventionelle proteinchemische Methoden erhältlichen $F_{ab}$- und $F_v$-Fragmenten (Skerra und Plückthun, 1988) konnten mit Hilfe gentechnischer Methoden und aufgrund des modularen Aufbaus der Immunglobuline diverse künstliche Konstrukte entwickelt werden (Überblick in Dübel und Kontermann, 2001). Hervorzuheben sind hier *Single-Chain* $F_v$-Fragmente (scFv) (Bird *et al.,* 1988), disulfidverbrückte $F_v$-Fragmente (dsFv) (Brinkmann *et al.,* 1993) sowie bivalente (Carter *et al.,* 1992) bzw. bispezifische (z. B. *Diabodies,* Holliger *et al.,* 1993) Antikörperfragmente. Für die Diagnostik und den Einsatz in der Therapie können durch genetische Fusion der rekombinanten Ig-Fragmente mit Effektormodulen bifunktionelle Proteine erhalten werden. So stehen u. a. Fusionen mit der Alkalischen Phosphatase (Muller *et al.,* 1999) und dem grün fluoreszierenden Protein (GFP; Griep *et al.,* 1999) zur Verfügung. Fusionen von Antikörperfragmenten mit Radioisotopen oder zytotoxischen Substanzen sind für die Krebsbehandlung von potentiell großer Bedeutung (Immunotoxine; Reiter und Pastan, 1998). Dabei wird die selektive Bindung entsprechender Ig-Fragmente an spezifische Oberflächenproteine auf Tumorzellen für die ortsgerichtete Applikation von Therapeutika ausgenutzt (*Tumor Targeting*).

Die Methoden zur Herstellung von Antikörperfragmenten in *E. coli* ermöglichen jedoch nicht nur deren Bereitstellung für Diagnostik und Therapie in ausreichender Qualität und Quantität, sondern auch die einfache und schnelle Modifikation ihrer protein- und immunchemischen Eigenschaften. Die leichte Handhabbarkeit eines bakteriellen Wirts erlaubt die unkomplizierte Veränderung der vektorkodierten Gene des Fremdproteins mit molekularbiologischen Standardmethoden. Durch gezieltes *Antibody Engineering* (Kontermann und Dübel, 2001) können so Antikörperfragmente z. B. hinsichtlich ihrer Bindungsaffinität oder ihrer Wirtsverträglichkeit optimiert werden. Ebenso lassen sich spezifische Antikörper bzw. deren Fragmente gegen so unterschiedliche Zielsubstanzen wie niedermolekulare Strukturen oder z. B. Proteine künstlich d. h. außerhalb des Immunsystems herstellen. Bei solchen evolutiven Verfahren werden durch die Einführung von Zufallsmutationen synthetische Bibliotheken von Antikörperfragmenten hergestellt, die in ihrem Umfang dem menschlichen Repertoire nahe kommen können (Knappik *et al.,* 2000). Durch geeignete Selektionsstrategien wie dem *Phage Display* oder dem *Ribosome Display* (Winter, 1998, Hoogenboom *et al.,* 1998; Hanes *et al.,* 2000) werden im Erfolgsfall funktionelle Ig-Fragmente mit der gewünschten Bindungseigenschaft isoliert. Auf diese Weise ist es z. B. auch möglich, Bindeproteine für solche Antigene zu erhalten, die bei einer klassischen Immunisierung toxische Effekte oder nur eine schwache Immunantwort hervorrufen würden.

Trotz der genannten Erfolge und Möglichkeiten, die das *Antibody Engineering* bietet, können bestimmte Nachteile die praktische Verwendung von Antikörpern limitieren. So ist die Bereitstellung ausreichender Mengen problematisch: Die Produktion funktioneller Antikörper findet in eukaryotischen Zellkultursystemen - einem äußerst kostenintensiven Verfahren - statt. Weiterhin stehen die aufgrund ihrer Größe geringe Gewebepenetration der Antikörpermoleküle bzw. deren lange Verweildauer im Serum (langsame *Blut-clearence*) vielen therapeutischen Anwendungen entgegen. Kleinere Fragmente von Antikörpern wie $scF_v$ oder $F_{ab}$-Fragmente (s. o.) lassen sich zwar bakteriell und damit prinzipiell kostengünstiger herstellen, aufgrund ihrer ungünstigen Faltungseigenschaften und der notwendigen Ausbildung mehrerer Disulfidbrücken liegen die Ausbeuten der rekombinanten Produktion allerdings oft unter dem gewünschten Niveau. Weiterhin sind rekombinante Antikörperfragmente oftmals instabiler und weisen eine geringere Bindungsaktivität im Vergleich zum parentalen Antikörper auf.

Um solche Einschränkungen zu umgehen wird versucht, das Prinzip der Antikörperbindung - nämlich die Bindung mittels einer hypervariablen oberflächenexponierten Region, lokalisiert auf einem konservierten Proteingerüst - auf andere Proteine zu übertragen (Skerra, 2000). D.h. es werden vornehmlich variable Schleifen (Loops) variiert, um eine künstliche Bindungseigenschaft zu generieren. Dabei wird im allgemeinen von natürlichen Bindungsproteinen wie z. B. Lipocalinen (Beste *et al.,* 1999) oder der Fibronectin Typ III-Domäne (Koide *et al.,* 1998) ausgegangen, deren Bindungsstellen - analog zu Antikörpern - aus flexiblen "Loop"-Strukturen gebildet werden und deren Modifikation die Erkennung von anderen als den natürlichen Liganden ermöglicht.

Alternativ hierzu wird gemäß WO 01/04144 bei beta-Faltblatt-Strukturproteinen, die *per se* keine Bindungsstelle aufweisen, eine solche künstlich auf der Proteinoberfläche generiert. Durch eine solche *de novo* generierte, künstliche Bindungsstelle (s. u.) können z. B. Varianten des γ-Kristalins-einem Strukturprotein der Augenlinse- erhalten werden, die mit vorher definierten Substanzen mit quantifizierbarer Affinität und Spezifität wechselwirken, Im Unterschied zu der oben beispielhaft angeführten Modifikation vorhandener und aus flexiblen "Loop"-Strukturen gebildeter Bindungsstellen, werden diese gemäß WO 01/04144 *de novo* auf der Oberfläche von beta-Faltblättern erzeugt. Die WO 01/04144 beschreibt jedoch lediglich die Veränderung vergleichsweise großer Proteine zur Erzeugung von neuen Bindungseigenschaften. Aufgrund deren Größe sind die Proteine gemäß WO aus technischer Ebene nur durch vergleichsweise aufwendige Methoden modifizierbar. Bei den bisher offenbarten Proteinen wurde prozentual auch nur ein verhältnismäßig kleiner Anteil der gesamten Aminosäuren verändert, um die Gesamtstruktur des Proteins zu erhalten. Demnach steht auch ein nur relativ kleiner Bereich der Proteinoberfläche zur Verfügung, der zur Erzeugung vorher nicht vorhandener Bindungseigenschaften genutzt werden kann. Weiterhin offenbart die WO 01/04144 experimentell lediglich die Erzeugung einer Bindungseigenschaft an niedermolekulare, kleine Moleküle, jedoch nicht an größere Moleküle wie z. B. Proteine.

Es ist daher eine Aufgabe der vorliegenden Erfindung, Proteine mit neuen, vorher nicht vorhandenen Bindungsaffinitäten zu ausgewählten Bindungspartnern bereitzustellen, die obige Nachteile nicht aufweisen. Ein weiteres Ziel der vorliegenden Erfindung ist es, Ersatzmoleküle für Antikörper zu schaffen, welche jedoch die oben erwähnten Nachteile von Antikörpern nicht aufweisen.

Erfindungsgemäß wird diese Aufgabe durch die Bereitstellung von modifizierten Proteinen gemäß Anspruch 1 gelöst, die weitgehend auf der Proteinstruktur des Ausgangsproteins, z.B. des Ubiquitins basieren und die an ihrer Oberfläche eine künstlich generierte Bindungsstelle aufweisen.

Insbesondere wird erfindungsgemäß ein Protein bereitgestellt, ausgewählt aus der Gruppe, bestehend aus Proteinen der Protein-Superfamilie "ubiquitin-like proteins", Proteinen, die ein Ubiquitin-artiges Faltungsmotiv aufweisen sowie Fragmenten oder Fusionsproteinen hiervon, die je das Ubiquitin-artige Faltungsmotiv besitzen, wobei das Protein unter Beibehaltung des Ubiquitin-artigen Faltungsmotivs, aufgrund einer oder mehrerer Modifikationen von Aminosäuren in zumindest einem oberflächenexponierten Bereich des Proteins, der mindestens einen beta-Faltblattstrang des Beta-Faltblattbereichs und wahlweise Nicht-Beta-Faltblattbereiche beinhaltet, eine vorher nicht vorhandene Bindungsaffinität gegenüber einem vorbestimmten Bindungspartner aufweist.

Die Erfindung betrifft somit ein durch Substitution, Insertion, Deletion, chemische Modifikation oder Kombinationen hiervon modifiziertes Protein, das aus der Gruppe ausgewählt ist, bestehend aus Proteinen der Protein-Superfamilie "ubiquitin-like proteins", Proteinen, die ein Ubiquitin-artiges Faltungsmotiv aufweisen sowie Fragmenten oder Fusionsproteinen hiervon, die alle jeweils ein Ubiquitin-artiges Faltungsmotiv aufweisen, wobei das Protein aufgrund dieser Modifikation eine vorher nicht vorhandene Bindungsaffinität gegenüber einem vorbestimmten Bindungspartner besitzt, erhältlich durch folgendes Verfahren:

a) Auswählen eines zu modifizierenden Proteins;
b) Bestimmen eines Bindungspartners;
c) Auswählen von Aminosäuren in zumindest einem oberflächenexponierten Bereich des Proteins, der mindestens einen beta-Faltblattstrang des Beta-Faltblattbereichs und wahlweise Nicht-Beta-Faltblattbereiche beinhaltet;
d) Modifizieren der ausgewählten Aminosäuren durch Substitution, Insertion, Deletion und/oder chemische Modifikation, unter Beibehaltung des Ubiquitin-artigen Faltungsmotivs,
e) Inkontaktbringen des modifizierten Proteins mit dem in Schritt b) bestimmten Bindungspartner;
f) Ermitteln derjenigen Proteine, die eine Bindungsaffinität gegenüber dem in Schritt b) vorbestimmten Bindungspartner aufweisen.

Weiterhin hat die vorliegende Erfindung die Aufgabe, entsprechende Verfahren zur Gewinnung von oben genannten Ubiquitin-basierten modifizierten Proteinen und Verwendungen für diese modifizierten Proteine bereitzustellen.

Die Erfindung beschreibt somit auch ein Verfahren zur Herstellung eines Proteins, ausgewählt aus der Gruppe, bestehend aus Proteinen der Protein-Superfamilie "ubiquitin-like proteins", Proteinen, die ein Ubiquitin-artiges Faltungsmotiv aufweisen sowie Fragmenten oder Fusionsproteinen hiervon, die alle jeweils ein Ubiquitin-artiges Faltungsmotiv aufweisen, wobei das Protein aufgrund einer oder mehrerer Modifikationen eine vorher nicht vorhandene Bindungsaffinität gegenüber einem vorbestimmten Bindungspartner aufweist, erhältlich durch folgendes Verfahren:

a) Auswählen eines zu modifizierenden Proteins;
b) Bestimmen eines Bindungspartners;
c) Auswählen von Aminosäuren in einem oberflächenexponierten Bereich des Proteins, der mindestens einen beta-Faltblattstrang des Beta-Faltblattbereichs und wahlweise Nicht-Beta-Faltblattbereiche beinhaltet;
d) Modifizieren der ausgewählten Aminosäuren, bevorzugt durch Substitution, Insertion, Deletion und/oder chemische Modifikation, unter Beibehaltung des Ubiquitin-artigen Faltungsmotivs;
e) Inkontaktbringen des modifizierten Proteins mit dem in Schritt b) bestimmten Bindungspartner;
f) Ermitteln derjenigen Proteine, die eine Bindungsaffinität gegenüber dem in Schritt b) vorbestimmten Bindungspartner aufweisen.

Die Erfindung stellt somit Proteine bzw. Polypeptide bereit, die durch Modifikation von Proteinen bzw. Polypeptiden hergestellt wurden, welche ein Ubiquitin-artiges Faltungsmotiv, wie es in der vorliegenden Anmeldung definiert wird, besitzen. Hierzu gehören die Proteine der Protein-Superfamilie "ubiquitin-like-proteins", alle Proteine, die ein Ubiquitin-artiges Faltungsmotiv besitzen und Fragmente oder Fusionsproteine dieser Proteine, vorausgesetzt, sie besitzen ebenfalls ein Ubiquitin-artiges Faltungsmotiv. Ausgehend von diesen Proteinen bzw. Polypeptiden werden ein oder mehrere Aminosäuren im ursprünglichen Protein bzw. Polypeptid modifiziert. Die Modifikationen umfassen insbesondere den Austausch von Aminosäuren, aber auch Insertionen und Deletionen von ein oder mehreren Aminosäuren sowie chemische Modifikationen von Aminosäuren. Diese Modifikationen werden in mindestens einem oberflächenexponierten Bereich des zu modifizierenden Proteins vorgenommen. Die Modifikation zumindest einer Aminosäure umfaßt mindestens einen Beta-Faltblattstrang des Beta-Faltblattbereichs, wobei der Beta-Faltblattstrang an der Oberfläche des Proteins lokalisiert sein muß, so daß er für den Bindungspartner bzw. Liganden, der mit einer bestimmbaren Affinität an das modifizierte Protein binden kann, zugänglich ist. In einer weiteren Ausführungsform der Erfindung werden, zusätzlich zu den Änderungen im Beta-Faltblattstrang des Beta-Faltblattbereichs, auch nicht-beta-Faltblattbereiche modifiziert, welche bevorzugt oberflächenexponiert sind, um die Bindungsaffinität gegenüber dem vorbestimmten Bindungspartner zu beeinflussen, insbesondere zu erhöhen und damit die Spezifität zu steigern.

Dem Fachmann stehen verschiedenste, an sich bekannte Techniken zur Modifikation einer oder mehrerer Aminosäuren zur Verfügung. Diese werden nachfolgend detaillierter beschrieben. Ergänzend wird auch hingewiesen auf die Veröffentlichungen von Ausuebel et al., 1994, sowie Sambrook et al., 1989.

Modifikationen von Aminosäuren des nicht oberflächenexponierten Kernbereichs des Ubiquitins sind bereits bekannt (Finucane et al., Biochemistry, Vol. 38, No. 36, 1999 oder Lazar et al., Protein Science (1997), 6:1167-1178). Die dort vorgenommenen Änderungen betreffen Positionen, die aufgrund der Lokalisation im hydrophoben Kern nicht an Bindungen beteiligt sind, da sie dem Lösungsmittel oder möglichen Bindungspartnern nicht zugänglich sind.

Im folgenden soll erläutert werden, was unter dem Begriff "vorher nicht vorhandene Bindungseigenschaft" bzw. *de novo* generierte, künstliche Bindungsstelle in dieser Erfindung verstanden wird. Hierunter ist zu verstehen, dass das modifizierte Protein an dem modifizierten Bereich vorher keine Bindungseigenschaft zu einem vorbestimmten Bindungspartner oder einem natürlichen Bindungspartner von Ubiquitin aufweist. In einer weiteren Ausführungsform der Erfindung werden die zu modifizierenden Proteine so ausgewählt, daß sie zu dem vorbestimmten Bindungspartner keine Bindungsaffinität besitzen. Die Bindungspartner, die auch als Liganden definiert werden können, weisen eine meßbare Affinität zum erfmdungsgemäß modifizierten Protein auf. Als Mindestwert für das Vorliegen einer quantifizierbaren Bindungseigenschaft, d.h der Affinität, mit welcher der Partner gebunden wird, kann erfindungsgemäß eine Dissoziationskonstante für den gebildeten Komplex von $K_D = 10^{-5}$ M oder kleiner angesehen werden. Ab einem Wert von $10^{-5}$ M kann von einer quantifizierbaren Bindungsaffinität ausgegangen werden. Bevorzugt ist je nach Anwendung ein Wert von $10^{-6}$ M bis $10^{-12}$ M, weiterhin bevorzugt $10^{-7}$ bis $10^{-11}$ M für z. B. chromatographische Anwendungen oder $10^{-9}$ bis $10^{-12}$ M für z. B. diagnostische oder therapeutische Anwendungen. Weitere bevorzugte Bindungsaffinitäten liegen im Bereich von $10^{-7}$ bis $10^{-10}$M, bevorzugt bis $10^{-11}$M. Die Verfahren zur Bestimmung der Bindungsaffinitäten sind an sich bekannt und werden auf den nachfolgenden Seiten weiter beschrieben.

Unter Modifikation sind erfindungsgemäß Substitutionen von Aminosäuren, Insertionen, Deletionen oder chemische Modifikationen zu verstehen.

Als zu modifizierende Proteine kommen erfindungsgemäß Proteine der Superfamilie "ubiquitin-like proteins" in Frage. Diese Superfamilie umfasst erfindungsgemäß die in Murzin *et al.* (1995) aufgezählten Untergruppen. Hierzu gehören bspw. die Protein-Familien "ubiquitin-related proteins", "UBX domain", "GABARAP-like", RAS-binding domain" etc. Bevorzugt werden Proteine der Protein-Familie "ubiquitin-related proteins" eingesetzt. Erfmdungsgemäß sind auch solche Proteine umfasst, die ein Ubiquitin-artiges Faltungsmotiv aufweisen. Beispiele hierfür sind SUMO-1, FAU, NEDD-8, UBL-1 und GDX sowie Rub1, APG8, ISG15, URM1, HUB1, Elongin B, PLIC2. (N-terminale Domäne), human Parkin (N-terminale Domäne).

Die erfindungsgemäß einsetzbaren Proteine der Superfamilie ubiquitin-like proteins sind genauestens definiert. Nur beispielsweise wird auf folgende Internetseite hingewiesen: http://bip.weizmann.ac.il/scop/index.html. Danach ist die Familie der ubiquitin-like proteins als Superfamilie definiert, zu welcher die Familie der ubiquitin-related-proteins gehört. Alle Mitglieder dieser Superfamilie zeichnen sich hauptsächlich durch antiparallel angeordnete β-Faltblätter aus, die in α- und β-Abschnitte unterteilt sind. Die Faltung wird als beta-Grasp und damit ubiquitin-ähnlich definiert. Der Kernbereich ist wie folgt definiert: beta(2)-alpha-beta(2), wobei die Ziffern die Anzahl der Stränge bezeichnen, wobei die Summe der Stränge das β-Faltblatt bildet. Die Anordnung des mixed-beta-sheets ist 2143, worunter die Lage der Stränge bei Aufsicht auf das Faltblatt von links nach rechts (Aminoterminus unten, Carboxyterminus oben) bezeichnet wird. Charakteristisch für die Mitglieder der ubiquitin-like-proteins ist damit ein zu einer Oberfläche des Proteins exponiertes antiparalleles β-Faltblatt, welches auf seiner Rückseite von einer senkrecht darüberliegenden α-Helix bedeckt wird. Dieses ubquitin-artige Faltungsmotiv ist charakteristisch für die erfindungsgemäß einsetzbaren und modifizierbaren Proteine und grenzt die Mitglieder der Familie von anderen Proteinen eindeutig ab. Unter Berücksichtigung dieser Definition werden von der Erfindung auch umfaßt die ubiquitin-ähnliche N-terminale Domäne von PLIC-2 und die ubiquitin-ähnliche Domäne von Parkin.

Der Fachmann kann entweder anhand von Sequenzvergleichen, sogenannten Alignments oder Strukturüberlagerungen erste Schlüsse ziehen, ob es sich bei den Proteinen um ein Mitglied der Protein-Superfamilie der ubiquitin-like-proteins handelt. Letzte Sicherheit bietet natürlich immer eine Strukturanalyse, beispielsweise eine röntgenkristallographische Strukturanalyse oder multidimensionale Kernresonanz-Spektroskopie. Gute Voraussagen lassen sich mittlerweile auch durch Strukturanalysen mit Hilfe genetischer Algorithmen erzielen.

Weitere Informationen über die Ubiquitin-Superfamilie finden sich beispielsweise in der Veröffentlichung von Larsen et al., 2002. Ergänzend sei auch auf die Veröffentlichung von Buchberger et al., 2001, hingewiesen. Buchberger beschreibt die typische β-Grasp-Faltung als Charakteristikum für ubiquitin-like-proteins mit einer Sekundärstruktur der Organisation beta-beta-alpha-beta-beta-alpha-beta, d.h. die Anordnung von fünf beta-Strängen in Form einer "mixed-sheet" in der Anordnung 21534. Bemerkenswert ist hierbei, dass UBX keine signifikanten Homologie in der Primärsequenz zu z. B. Ubiquitin aufweist (Buchberger et al., 2001), trotzdem aber - aufgrund seiner dreidimensionalen Struktur, die identisch zu der von z. B. Ubiquitin ist - unter die ubiquitin-like proteins subsummiert wird. In diesem Zusammenhang sei darauf hingewiesen, dass im Ubiquitin die Aminosäuren an Positionen 48 und 49 z. T. ebenfalls als eigenständiger beta-Strang angesehen werden (Vijay-Kumar, 1987). Dieser fünfte Strang, welcher in der Struktur des Ubiquitins nach der Helix lokalisiert wäre und dem "mixed sheet" die Anordnung 21534 gäbe, weist allerdings nur zwei Aminosäuren auf, und es ist durchaus zweifelhaft, diesen aus zwei Aminosäuren bestehenden Strang als beta-Faltblatt-Strang zu bezeichnen. Nach Buchberger et al. (2001) könnten jedoch wie oben angeführt auch Proteine mit der Anordnung 21534 zwanglos unter die Superfamilie der ubiquitin-like-proteins subsummiert werden. Für die vorliegende Erfindung wurde für die Anordnung der beta-Stränge im Ubiquitin die oben näher beschriebene 2143-Definition gewählt.

Die Proteine der genannten Familie und Superfamilie sind in der Regel hochkonserviert.

Bspw. weist Ubiquitin nach bisherigen Erkenntnissen in allen Säugern die identische Aminosäuresequenz auf. Ubiquitin aus Hefe weist lediglich eine Abweichung in drei Aminosäuren davon auf. Humanes Ubiquitin bzw. Ubiquitin aus Säugern besteht aus 76 Aminosäuren und hat die eingangs beschriebene Struktur.

Erfindungsgemäß sollte das zu modifizierende Protein eine mindestens 30 %ige, bevorzugt mindestens 40%ige oder 50%ige, weiterhin bevorzugt mindestens 60%, mindestens 70%, mindestens 80%, mindestens 90% , oder mindestens 95 %ige Übereinstimmung in der Aminosäuresequenz zum Ausgangsprotein, welches modifiziert wird, z.B. zu humanem Ubiquitin, aufweisen, wobei das Protein jedenfalls ein Ubiquitin-artiges Faltungsmotiv, wie es oben eingehend definiert wurde, aufweist.

Erfindungsgemäß sind auch Fragmente der genannten Proteine umfasst, solange sie das eingangs beschriebene Ubiquitin-artige Faltungsmotiv beinhalten, sowie Fusionen der genannten Proteine mit anderen Proteinen. Im Fall solcher Fragmente und Fusionsproteine sind im Rahmen dieser Erfindung Aminosäurepositionsangaben so zu verstehen, dass diese sich immer auf die entsprechende Position in humanem Ubiquitin beziehen. Beispiele für Fusionspartner sind (Reporter-) Enzyme, Toxine oder andere Bindungsproteine etc. Weiterhin ist die chemische Kopplung bspw. mit niedermolekularen Substanzen wie Biotin, Digoxigenin, fluoreszierenden und/oder lumineszierenden Stoffen etc. möglich.

Im Fall von Fusionsproteinen kann erfindungsgemäß ein bereits fusioniertes Protein modifiziert werden. Erfindungsgemäß ist aber auch umfasst, dass ein Teil nach Modifikation oder Selektion anfusioniert wird. Dies kann jeweils nach dem Fachmann bekannten Verfahren geschehen.

Gemäß der vorliegenden Erfindung ist das Protein, das zur Herstellung des modifizierten Proteins ausgewählt wird, bevorzugt humanes Ubiquitin oder Ubiquitin anderen Ursprungs, bspw. ein anderes Säuger-Ubiquitin. Die Erfindung wird deshalb nachfolgend insbesondere am Beispiel des humanen Ubiquitins beschrieben. Anhand mehrerer Beispiele wird die Modifikation des humanen Ubiquitins beschrieben, um ein Protein zu erhalten, welches auch als Mutein bezeichnet werden kann, welches eine vorher nicht vorhandene Bindungsaffinität gegenüber einem vorbestimmten Bindungspartner zeigt. Als Säuger-Ubiquitine kommen insbesondere Ubiquitine von Nagetieren, von Haustieren und Nutztieren im Bereich der Säugetiere in Frage. Ist das Einsatzgebiet der erfindungsgemäß hergestellten Proteine bekannt, d.h. soll beispielsweise das modifizierte Protein als Arzneimittel zur Behandlung von Erkrankungen beim Menschen eingesetzt werden, kann bevorzugt ein humanes Protein als zu modifizierendes Ausgangsprotein eingesetzt werden; diese gilt analog für entsprechende Einsatzgebiete. Es wird darauf hingewiesen, daß die nachfolgenden Ausführungen nur beispielhaft auf humanes Ubiquitin gestützt sind. Auf der Grundlage dieser ausführlichen Beschreibung und der genannten Beispiele ist es für den Fachmann möglich, weitere Proteine mit Ubiquitin spezifischen Faltungsmotiven erfindungsgemäß zu modifizieren. Die Erfindung ist also nicht auf humanes Ubiquitin oder auf Ubiquitin allgemein beschränkt. Entsprechende Hinweise und Ausführungen sind als beispielhafte Ausgestaltungen der Erfindung, die allerdings besonders bevorzugt sind, zu verstehen.

Humanes bzw. Säuger-Ubiquitin weist, wie eingangs erwähnt, 76 Aminosäuren auf. Die Aminosäuren der vier Betastränge, die zu Bildung des antiparallelen beta-Faltblatts beitragen, sind erfindungsgemäß entsprechend der Struktur 1UBQ in der PDB-Datenbank (http://www.rcsb.org/pdb/index.html) die folgenden Aminosäurepositionen: Erster (aminoterminaler) Strang: 2 bis 7; zweiter beta-Faltblattstrang: 12 bis 16; dritter Strang: 41 bis 45; vierter (carboxyterminaler) Strang: 66 bis 71. Die Lage der Stränge bei Aufsicht auf das Faltblatt (Aminoterminus unten, Carboxyterminus oben) von links nach rechts ist: 2., 1., 4., 3., Strang, wobei die Polypeptidkette zwischen 1. und 4. Strang die alpha-Helix bildet.

Auswahl und Modifikation der zu modifizierenden Aminosäuren:

Ausgehend von entsprechenden Strukturdaten, wie sie z. B. in der Protein Data Bank™ (Berman *et al.,* 2000; http://www.rcsb.org/pdb) frei verfügbar sind, können mittels rechnergestützter Analyse die Positionen solcher Aminosäuren im Ausgangsprotein, z.B. im Ubiquitin-Proteingerüst, lokalisiert werden, deren Seitenketten oberflächenexponiert, d. h. dem Lösungsmittel oder einem potentiellen Bindungspartner zugewandt, sind. Weiterhin können durch rechnergestützte Analyse solche Aminosäuren im Ausgangsprotein, z.B. im Ubiquitin, identifiziert werden, deren zufällige Substitution vermutlich keinen oder nur einen geringen negativen Effekt auf die Stabilität des Proteingerüstes haben könnte. Diese Informationen können einen ersten Anhaltspunkt für die Eignung jeder einzelnen Aminosäure als Element einer Bindungsstelle darstellen, was dann einer Überprüfung in der Praxis bedarf. In einer bevorzugten Ausführungsform der vorliegenden Erfindung wurden bspw. aufgrund ihrer Oberflächenexposition und der Toleranz der Gesamtstruktur gegenüber ihrem zufälligen Austausch die Aminosäuren an den Positionen 2, 4, 6, 62, 63, 64, 65 und 66 im humanen Ubiquitin ausgewählt. Die genannten Positionen befinden sich in räumlicher Nähe zueinander am Beginn des ersten aminoterminalen beta-Faltblattstrangs (Pos. 2, 4, 6) sowie im Loop (Pos. 62, 63) bzw. am Beginn des carboxyterminalen beta-Faltblattstrangs (Pos. 64, 65, 66) und bilden mit ihren Aminosäureseitenketten einen zusammenhängenden Bereich auf der Oberfläche des Ubiquitins (Abb. 1). Durch zufällige Aminosäure-Substitutionen ("Randomisierung") in dem analysierten Bereich kann so - analog zu der Antigen-Bindungsstelle von Antikörpern - ein hypervariabler oberflächenexponierter Bereich auf der weiterhin intakten Proteinstruktur des Ubiquitins generiert werden.

Mit Hilfe der ProSAII-Software ("Protein Structure Analysis"; Proceryon Biosciences, Salzburg) konnte bspw. die Proteinstabilität von $10^4$ Varianten im Vergleich zum Ubiquitin (WT) und einer gleichgroßen Stichprobe von Varianten, bei denen die Reste eines "Kontrollepitopes" (randomisierte Positionen 24, 28, 31, 32, 35, 37, 38, 39) substituiert wurden, bestimmt werden. Dabei weisen ca. 19 % der *in silico* generierten Varianten, die im Bereich der Bindungsstelle zufällig substituiert worden waren, eine mindestens so hohe Stabilität wie Ubiquitin (WT) auf, während ca. 90 % stabiler als die Träger des "Kontrollepitopes" waren (Abb. 2). Dieses rechnergestützte Ergebnis kann dann als Hinweis für die Auswahl geeigneter Aminosäuren dienen.

Gemäß einer bevorzugten Ausführungsform wurden - ausgehend von den verfügbaren Strukturdaten des humanen Ubiquitins - zunächst acht Aminosäurepositionen im Bereich der zu generierenden Bindungsstelle ausgewählt. Durch zufällige Veränderungen der Primärsequenz in diesem Bereich (Random-Mutagenese) und anschließende spezifische Auswahl (Selektion) wurden solche Varianten gewonnen, welche die gewünschte Bindungsaktivität für ein vorgegebenes Hapten oder Antigen bzw. allgemein einen vorbestimmten Bindungspartner aufweisen. Obwohl den erhaltenen modifizierten Proteinen auf diese Weise eine *de novo* Bindungseigenschaft verliehen wird, bleiben sie hinsichtlich Struktur und proteinchemischen Eigenschaften weitestgehend mit dem Ausgangsprotein identisch. Damit weisen sie Vorteile wie z.B. geringe Größe, hohe Stabilität, kostengünstige Herstellung sowie einfache Modifizierbarkeit, gepaart mit hoher Affinität und Spezifität für einen vorher definierten Liganden auf. Die Eignung des Ubiquitins als Gerüststruktur für die Generierung künstlicher Bindungsproteine war dabei nicht vorhersehbar, da 1.) die Toleranz des Gerüsts gegenüber den umfangreichen Aminosäure-Austauschen aufgrund der geringen Größe des Ubiquitins nicht zu erwarten war und 2.) die Funktionalität der künstlichen Bindungsstelle unter Einbeziehung des als starr und unflexibel angesehenen beta-Faltblatts nicht von vornherein gegeben scheint.

Unter Antigen ist erfindungsgemäß eine Substanz zu verstehen, die von einem Antikörper gebunden wird. Der Begriff Antigen umfasst Haptene, Peptide, Proteine, Zucker, DNA etc. Aus dem Roche Lexikon Medizin (4. Auflage; http://www.gesundheit.de/roche) geht folgende Definition für Antigen und Hapten hervor, die für die vorliegende Erfindung übernommen wird:

> Antigen (AG): Bezeichnung für jede Substanz, die vom Immunsystem als fremd (»not self«) erkannt wird. Löst meist eine Immunreaktion aus, die zur Immunität führt (= »Immunogen«); im Fall der Allergie (= »Allergen«) bzw. Atopie (»Atopigen«) ist diese übersteigert. Das AG löst eine humorale (Antigen-Antikörper-Reaktion) u./oder zellvermittelte Abwehrreaktion (s.u. Immunität) aus. Wird das AG vom Immunsystem geduldet (Immuntoleranz), wird es auch als »Tolerogen« bezeichnet. Antigen wirksam sind v.a. komplexe u. größermolekulare Stoffe (Eiweißkörper, Polysaccharide, Nucleotide u. zahlreiche synthetische Verbindungen) mit chemisch charakterisierbaren Gruppierungen (Determinante), die für die Immunantwort verantwortlich sind. Unterschieden als 1) meist höhermolekulares Voll-AG, das allein in der Lage ist, eine Immunreaktion auszulösen, 2) als niedermolekulares Hapten (= Halbantigen), das erst nach Kopplung an ein größeres Trägermolekül als Immunogen wirkt. Bezeichnet z.B. als xeno-, allo- oder isogenes, autologes AG; Auto-, Hetero-, Transplantations-, Tumor-, Virus-AG.
>
> Hapten: einfache, niedermolekulare chemische Verbindung, die für die Spezifität eines Antigens (AG) verantwortlich bzw. durch ihre Struktur (Determinante) zur spezifischen Bindung des Antikörpers befähigt ist, im Gegensatz zum Voll-AG aber keine Allergie erzeugt. Wird nach Bindung an einen als Carrier bezeichneten Eiweißkörper zum Vollantigen (Antigen).

Es wird hervorgehoben, daß es mit Hilfe der vorliegenden Erfindung auch möglich ist, Varianten des Ubiquitin zu erzeugen, die eine Bindungseigenschaft gegenüber nicht immunogenen Substanzen als Bindungspartnern, wie z. B. Tumor-Markern, aufweisen.

In einer bevorzugten Ausführungsform der vorliegenden Erfindung erfolgt eine Modifikation, bevorzugt eine Substitution, zumindest teilweise an zwei oder mehreren in der Primärsequenz direkt benachbarten Aminosäuren, wobei sich die in der Tertiärstruktur benachbarten Aminosäuren weiterhin bevorzugt zumindest teilweise in einem beta-Faltblattstrang des Proteins befinden. Normalerweise geht jeder Austausch einer Aminosäure in einem Protein mit einer Beeinträchtigung der Stabilität des Proteins einher. Einzelaustausche können gegebenenfalls noch aufgrund der Einwirkung benachbarter Aminosäuren ohne weitgehende Destabilisierung verkraftet werden. Bei einer Veränderung eines ganzen Bereichs, also z.B. einer strukturellen Einheit, bestehend aus mehreren benachbarten Aminosäuren, kann von einem stabilisierenden Einfluss der unmittelbar benachbarten Aminosäuren nicht mehr ausgegangen werden. Dementsprechend wurden im Stand der Technik bisher auch nur nicht direkt benachbarte Aminosäuren des Ubiquitins modifiziert. Es war überraschend, dass bei einer solch tiefgreifenden Veränderung von Bereichen des Proteins durch Modifikation von direkt benachbarten Aminosäuren die Stabilität des Proteins nicht wesentlich nachließ. Überraschend und nicht zu erwarten war allein schon die Tatsache, dass zwei direkt benachbarte Aminosäuren in Ubiquitin oder Proteinen mit Ubiquitin-artigem Faltungsmotiv austauschbar sind, ohne die Stabilität und Struktur des Proteins zu beeinträchtigen.

Die Modifikation direkt benachbarter Aminosäuren hat weiterhin insbesondere im Fall des relativ kleinen Ubiquitins den Vorteil, dass eine derartige Modifikation gentechnisch leichter zu realisieren ist, als dies bei nicht direkt benachbarten Aminosäuren der Fall ist. Gemäß dieser Ausführungsform kann damit eine erleichterte Erzeugung einer großen Anzahl von modifizierten Proteinen sowohl auf Protein-Ebene als auch auf DNA-Ebene bereitgestellt werden.

Bevorzugt beträgt die Anzahl der Substitutionen direkt benachbarter Aminosäuren 2 bis 10, bevorzugter 2 bis 8 in der Primärsequenz direkt benachbarte Aminosäuren, weiterhin bevorzugt 3 bis 7 oder 4 bis 6 in der Primärsequenz direkt benachbarte Aminosäuren.

Wenn in der Primärsequenz direkt benachbarte Aminosäuren substituiert werden, kann ein Teil dieser Aminosäuren in den Bereich eines beta-Faltblattstrangs reichen. Dieser in den Bereich eines beta-Faltblattstrangs hineinreichende Teil kann eine Länge von zwei oder mehr Aminosäuren, bevorzugt zwei oder drei Aminosäuren, aufweisen. Der Bereich direkt benachbarter Aminosäuren liegt somit am Anfang oder Ende eines beta-Faltblattstrangbereichs, der bevorzugt etwa 2 bis 3 Aminosäuren lang ist.

In einer weiteren bevorzugten Ausführungsform werden 5 oder mehr direkt benachbarte Aminosäuren modifiziert, vorzugsweise substituiert, wobei zwei oder mehr, bevorzugt zwei oder drei, direkt benachbarte Aminosäuren den Anfang oder das Ende eines beta-Faltblattstrangbereichs bilden. Als Obergrenze für die Gesamtanzahl direkt benachbarter modifizierter Aminosäuren können in diesem Fall bevorzugt 8, 9 oder 10 Aminosäuren angesehen werden, besonders bevorzugt 8 Aminosäuren.

Im Fall der Modifikation direkt benachbarter Aminosäuren in einem beta-Faltblattstrang des Ubiquitins sind diese Aminosäuren in der Regel dann alle oberflächenexponiert, wenn sich diese Aminosäuren am Anfang oder am Ende eines beta-Faltblattstrangs befinden. In diesem Fall kann angenommen werden, dass dann alle Aminosäuren an der Generierung der neuen Bindungseigenschaft beteiligt sind.

In einer bevorzugten Ausführungsform der vorliegenden Erfindung werden zur Erzeugung eines Bereichs mit neuen Bindungseigenschaften solche Aminosäuren modifiziert, die einen zusammenhängenden Bereich auf der Oberfläche des Proteins bilden. Dadurch kann ein zusammenhängender Bereich mit einer zuvor nicht vorhandenen Bindungseigenschaft erzeugt werden. Unter "zusammenhängendem Bereich" ist erfindungsgemäß Folgendes zu verstehen: Aminosäuren wechselwirken aufgrund der Ladung, Raumstruktur und Hydrophobizität/ Hydrophilizität ihrer Seitenketten auf entsprechende Weise mit ihrer Umgebung. Die Umgebung kann das Lösungsmittel, i.A. Wasser, oder andere Moleküle sein, z.B. räumlich benachbarte Aminosäuren. Mittels der Strukturinformation des Proteins sowie entsprechender Software kann die Oberfläche des Proteins dargestellt werden. Z. B kann der Grenzbereich zwischen den Atomen des Proteins und dem Lösungsmittel so visualisiert werden, einschließlich der Information, wie dieser Grenzbereich strukturiert ist, welche Oberflächenabschnitte für das Lösungsmittel zugänglich sind oder wie die Ladungsverteilung auf der Oberfläche beschaffen ist. Ein zusammenhängender Bereich kann bspw. durch eine solche Visualiserung mittels einer geeigneten Software erkannt werden. Derartige Methoden sind dem Fachmann bekannt. Grundsätzlich steht erfindungsgemäß auch der gesamte oberflächenexponierte Bereich als zusammenhängender Bereich auf der Oberfläche für eine Modifikation zur Erzeugung neuer Bindungseigenschaften zur Verfügung. Bevorzugt kann eine Modifikation zu diesem Zweck auch den $\alpha$-Helixbereich umfassen.

Zwingend notwendig ist die Modifikation von Aminosäuren in zumindest einem oberflächenexponierten Bereich des Proteins, der mindestens einen $\beta$-Faltblattstrang des $\beta$-Faltblattbereichs umfaßt. Die Beta-Faltblatt-Struktur ist dadurch definiert, daß sie im wesentlichen plattenförmig und fast völlig gestreckt vorliegt. Im Gegensatz zu alpha-Helices, die aus einem kontinuierlichen Teil der Polypeptidkette gebildet werden, können Beta-Faltblätter aus verschiedenen Regionen der Polypeptidkette aufgebaut sein. Dadurch können in der Primärstruktur weiter auseinanderliegende Bereiche in unmittelbare Nähe gelangen. Ein Beta-Strang ist normalerweise 5-10 Aminosäuren lang und liegt fast völlig gestreckt vor. Die Beta-Stränge liegen so eng beieinander, daß sich Wasserstoffbrücken zwischen der C=O-Gruppe des einen und der NH-Gruppe des anderen Strangs bzw. umgekehrt ausbilden. Beta-Faltblätter können aus mehreren Strängen aufgebaut sein und haben eine plattenförmige Struktur. Dabei liegt das C-alpha-Atom immer abwechselnd über oder unter der plattenförmigen Ebene. Die Seitenketten der Aminosäuren folgen diesem Muster und sind somit alternativ einmal nach oben und einmal nach unten gerichtet. Je nach Orientierung der Beta-Stränge unterscheidet man parallele und antiparallele Faltblätter. Erfindungsgemäß können beide mutagenisiert und zur Herstellung der beanspruchten Proteine eingesetzt werden. Zur Mutagenese der Beta-Faltblatt-Struktur werden solche Beta-Faltblatt-Regionen im Protein ausgewählt, die oberflächennah sind. Oberflächenexponierte Aminosäuren können anhand der vorhandenen Röntgenkristallstruktur identifiziert werden. Liegt keine Kristallstruktur vor, so kann mittels Computeranalysen versucht werden, anhand der vorliegenden Primärstruktur oberflächenexponierte Beta-Faltblattbereiche und die Zugänglichkeit einzelner Aminosäurepositionen vorherzusagen (www.embl heideberg.de/predictprotein/predictprotein.html) oder die 3d-Proteinstruktur zu modellieren (www.expasy.ch/swissmo/SWISS-MODEL.html) und damit Aussagen über möglicherweise oberflächenexponierte Aminosäuren zu gewinnen.

Es sind aber auch Mutagenesen im Beta-Faltblatt möglich, bei denen eine zeitaufwendige Vorauswahl der zu mutagenisierenden Aminosäurepositionen entfällt. Diejenigen DNA-Bereiche, die für die Beta-Faltblatt-Strukturen kodieren, werden aus ihrer DNA-Umgebung isoliert, einer zufallsgesteuerten Mutagenisierung unterzogen und anschließend wieder in die für das Protein kodierende DNA, aus der sie zuvor entnommen wurden, integriert. Hieran schließt sich ein Selektionsverfahren auf Mutanten mit den gewünschten Bindungseigenschaften an.

In einer weiteren Ausführungsform der Erfindung werden, wie oben bereits ausgeführt, die oberflächennahen Beta-Faltblatt-Regionen ausgewählt und innerhalb dieser ausgewählten Regionen die zu mutagenisierenden Aminosäureposition identifiziert. Diese so ausgewählten Aminosäurepositionen können dann auf DNA-Ebene entweder zielgerichtet mutagenisiert werden, d.h. ein für eine bestimmte Aminosäure kodierendes Codon wird durch ein Codon ausgetauscht, das für eine andere, vorher ausgewählte spezifische Aminosäure kodiert, oder dieser Austausch erfolgt im Rahmen einer Random-Mutagenese, wobei die auszutauschende Aminosäureposition festgelegt ist, nicht dagegen das für die neue, noch unbestimmte Aminosäure kodierende Codon.

Oberflächenexponierte Aminosäuren sind dem umgebenden Lösungsmittel zugänglich. Bei einer Zugänglichkeit der Aminosäuren im Protein von mehr als 8% im Vergleich zur Zugänglichkeit der Aminosäure im Modelltripeptid Gly-X-Gly sprechen wir von oberflächenexponierten Aminosäuren. Diese Proteinbereiche bzw. einzelne Aminosäurepositionen sind auch bevorzugte Bindungsstellen für mögliche Bindungspartner, auf welche erfindungsgemäß selektiert werden soll.Ergänzend wird auch auf Connolly et al., 1983, und Shrake et al., 1973, hingewiesen, die zur vollständigen Offenbarung als Referenz in die Anmeldung mit aufgenommen werden.

Varianten des Ubiquitin-Proteingerüstes, die sich durch Aminosäure-Austausche im Bereich der *de novo* generierten, künstlichen Bindungsstelle vom parentalen Protein und voneinander unterscheiden, können durch gezielte Mutagenese der entsprechenden Sequenzabschnitte erzeugt werden. Dabei können Aminosäuren mit bestimmten Eigenschaften wie z. B. Polarität, Ladung, Löslichkeit, Hydrophobizität oder Hydrophilizität durch Aminosäuren mit entsprechenden anderen Eigenschaften ersetzt bzw. substituiert werden. Der Ausdruck "Mutagenese" umfasst neben Substitutionen auch Insertionen und Deletionen. Auf Protein-ebene können die Modifikationen auch durch chemische Veränderung der Aminosäureseitenketten nach dem Fachmann bekannten Verfahren vorgenommen werden.

Als Ausgangspunkt zur Mutagenese der entsprechenden Sequenzabschnitte kann bspw. die cDNA eines ubiquitin-like-proteins dienen, die mit dem Fachmann bekannten Methoden hergestellt, verändert und vervielfältigt werden kann. Zur ortsgerichteten Veränderung des Ubiquitins in relativ kleinen Bereichen der Primärsequenz (ca. 1-3 Aminosäuren) stehen kommerziell erhältliche Reagenzien und Verfahren zur Verfügung ("Quick Change", Stratagene; "Muta-Gene Phagemid *in vitro* Mutagenesis Kit", Biorad). Zur ortsspezifischen Mutagenese größerer Bereiche stehen dem Fachmann besondere Ausführungen z. B. der Polymerase-Kettenreaktion (PCR) zur Verfügung. Dabei kann beispielsweise eine Mischung synthetischer Oligodesoxynukleotide, die an den gewünschten Positionen degenerierte Basenpaar-Zusammensetzungen aufweisen, zur Einführung der Mutation verwendet werden. Dies kann auch durch die Verwendung von natürlicherweise nicht in genomischer DNA vorkommenden Basenpaaranaloga wie z. B. Inosin erreicht werden.

Ausgangspunkt zur Mutagenese eines oder mehrerer β-Faltblattstränge des β-Faltblattbereichs sowie wahlweise des nicht-β-Faltblattbereichs kann beispielsweise die cDNA eines ubiquitin-like-proteins sein oder auch die genomische DNA. Auch kann das für das Protein kodierende Gen synthetisch hergestellt werden.

Zur Mutagenese stehen die verschiedensten, an sich bekannten Methoden zur Verfügung, bei denen es sich um ortsspezifische Mutagenisierungsverfahren, zufallsgerichtete Mutagenisierungsverfahren, die Mutagenese mit Hilfe der PCR oder vergleichbare Verfahren handelt.

In einer bevorzugten Ausgestaltungsform der Erfindung werden die zu mutagenisierenden Aminosäurepositionen vorherbestimmt. Die Auswahl der zu modifizierenden Aminosäuren erfolgt entweder in Abhängigkeit vom zu modifizierenden Protein und/oder in Abhängigkeit vom ausgewählten Bindungspartner. Jedenfalls wird im allgemeinen eine Bibliothek verschiedenster Mutanten angelegt, welche mit Hilfe an sich bekannter Verfahren gescreent wird. Eine Vorauswahl der zu modifizierenden Aminosäuren ist natürlich dann besonders einfach zu treffen, wenn ausreichende Strukturinformationen über das zu modifizierende Protein vorliegen. Es ist aber auch möglich, ohne derartige Strukturinformationen mit Hilfe die Zufallsmutagenese einsetzender Verfahren und anschließender Selektion das Protein mit dem Ubiquitin-artigen Faltungsmotiv so abzuändern, daß es eine Bindungsaffinität zum vorherbestimmen Liganden bzw. Bindungspartner besitzt.

Gezielte Mutagenisierungsverfahren sowie die Mutagenisierung längerer Sequenzabschnitte, beispielsweise durch PCR, durch chemische Mutagenese oder durch Verwendung bakterieller Mutatorstämme gehört ebenfalls zum Stand der Technik und ist erfindungsgemäß einsetzbar.

In einer Ausführungsform der Erfindung erfolgt die Mutagenese durch Assemblierung von DNA-Oligonukleotiden mit dem Aminosäure-Codon NNK. Selbstverständlich sind auch andere Codons (Tripletts) einsetzbar.

Die Mutationen sind so, daß die Beta-Faltblatt-Struktur erhalten bleibt. Generell findet die Mutagenese an der Außenseite eines stabilen, an der Oberfläche des Proteines exponierten Beta-Faltblatt-Bereiches statt. Sie umfaßt sowohl site-spezifische als auch zufallsgesteuerte Mutagenisierungen. Ortsspezifische Mutagenesen, die einen relativ kleinen Bereich in der Primärstruktur umfassen (ca. 3-5 Aminosäuren), können mit den kommerziell angebotenen Kits von Stratagene (QuickChange) oder Bio-Rad (Muta-Gene phagemid in vitro mutagenesis kit) durchgeführt werden (vgl. US-A-5,789,166; US-A-4,873,192).

Bei größeren Bereichen einer ortsspezifischen Mutagenese muß eine DNA-Kassette hergestellt werden, wobei der zu mutagenisierende Bereich durch Assemblierung von Oligonukleotiden, die die mutierten und die nicht veränderten Positionen enthalten, erhalten wird (Nord et al., 1997; McConell und Hoess, 1995). Zufallsgesteuerte Mutagenesen können durch Vermehrung der DNA in Mutator-Stämmen oder durch eine PCR-Amplifikation (error-prone-PCR) eingeführt werden (z.B. Pannekoek et al., 1993). Dabei wird eine Polymerase mit erhöhter Fehlerrate verwendet. Um den Grad der eingeführten Mutagenese zu erhöhen, bzw. unterschiedliche Mutationen zu kombinieren, können die Mutationen in den PCR-Fragmenten mittels DNA shuffling (Stemmer, 1994) kombiniert werden. Eine Übersicht über diese Mutagenesestrategien bei Enzymen bietet das Review von Kuchner und Arnold (1997). Um diese zufallsgesteuerte Mutagenese in einem ausgewählten DNA-Bereich durchzufiihren, muß ebenfalls eine DNA-Kassette konstruiert werden, die für die Mutagenese genutzt wird.

Der zufällige Austausch von Aminosäuren gemäß einem Ausführungsbeispiel der vorliegenden Erfindung an den Positionen 2, 4, 6, 62, 63, 64, 65 und 66 des Ubiquitins kann besonders leicht durch PCR erfolgen, da die genannten Positionen sich nahe des Amino- bzw. Carboxyterminus des Proteins befinden. Dementsprechend liegen die zu manipulierenden Codons am 5'- bzw. 3'-Ende des entsprechenden cDNA-Stranges. So entspricht das erste für eine mutagene PCR-Reaktion eingesetzte Oligodesoxynukleotid in seiner Sequenz - abgesehen von den Codons der zu mutierenden Positionen 2, 4, und 6 - dem kodierenden Strang für den Aminoterminus des Ubiquitins. Das zweite Oligodesoxynukleotid entspricht demgemäß - bis auf die Codons der zu mutierenden Positionen 62, 63, 64, 65 und 66 - zumindest teilweise dem nicht-kodierenden Strang für die Polypeptidsequenz des Carboxyterminus. Mit beiden Oligodesoxynukleotiden kann eine Polymerase-Kettenreaktion unter Verwendung der für das Ubiquitin-Proteingerüst kodierenden DNA-Sequenz als Matrize durchgeführt werden.

Im weiteren kann das erhaltene Amplifizierungsprodukt unter Verwendung flankierender Oligodesoxynukleotide, die z. B. Erkennungssequenzen für Restriktions-Endonukleasen einführen, einer erneuten Polymerase-Kettenreaktion zugeführt werden. Die erhaltenen synthetischen DNA-Moleküle können nach der Hydrolyse mit den geeigneten Restriktions-Endonukleasen mit entsprechend vorbereiteten Nukleinsäuresequenzen von z.B. Klonierungs- oder Expressionsvektoren ligiert d. h. verknüpft werden. Derartige Vorgehensweisen und Systeme sind dem Fachmann bekannt Kommerziell erhältlich sind solche Systeme z. B. von Novagen (Madison, WI), IBA (Göttingen) oder New England

**Biolabs ( Beverly, MA))**: Erfindungsgemäß bevorzugt wird die erhaltene Genkassette in ein Vektorsystem verbracht, das für die Verwendung in nachfolgenden Selektionsverfahren für die Isolierung von Varianten des Ubiquitins mit Bindungseigenschaften für ein vorher bestimmtes Hapten oder Antigen geeignet ist.

Gemäß einer bevorzugten Ausführungsform der vorliegenden Erfindung werden nur Aminosäurepositionen zur Bildung einer neuen Bindungseigenschaft modifiziert, die nicht zu Bereichen gehören, die bei unmodifiziertem Ubiquitin an Bindungen an natürlichen Ubiquitin-Bindungspartnern beteiligt sind. Dadurch kann gewährleistet werden, dass nicht lediglich bereits vorhandene Bindungseigenschaften des Ubiquitins verändert werden.

Die Bereiche zur Modifikation können grundsätzlich danach ausgewählt werden, ob sie für einen möglichen Bindungspartner zugänglich sein können, und ob die Gesamtstruktur des Proteins vermutlich gegenüber einer Modifikation Toleranz zeigen wird.

Gemäß der vorliegenden Erfindung können bei dem Protein, bevorzugt Ubiquitin aus Säugern, mindestens 15% der in Betasträngen befindlichen Aminosäuren, vorzugsweise mindestens 20 %, weiterhin bevorzugt mindestens 25%, modifiziert, bevorzugt substituiert, werden, um eine vorher nicht vorhanden Bindungseigenschaft zu erzeugen. Maximal sind dabei bevorzugt etwa 40 % der in Betasträngen befindlichen Aminosäuren, weiterhin bevorzugt, maximal etwa 35% und noch bevorzugter etwa 30% modifiziert, bevorzugt substituiert.

Gemäß einem Ausführungsbeispiel der vorliegenden Erfindung wurden bspw. 6 der 24 in Betasträngen befindlichen Aminosäuren modifiziert, um eine Bindungseigenschaft zu einem vorbestimmten Bindungspartner zu erzeugen. Die Wahl einer größeren Anzahl von Aminosäuren für Modifikationen gibt die Möglichkeit, eine größere Bibliothek vom Proteinen mit Bindungsaffinität zu generieren, so dass die Wahrscheinlichkeit, dass eines dieser modifizierten Proteine eine quantifizierbare und/oder hohe Bindungsaffinität zu einem vorbestimmten Bindungspartner aufweist, zunimmt.

Weiterhin kann neben einer Modifikation in Betasträngen auch eine Modifikation in anderen oberflächenexponierten Bereichen des Proteins erfolgen, bevorzugt bspw. in Loop-Bereichen. Diese modifizierten Bereiche können neben den modifizierten Bereichen in den Betasträngen ebenfalls an der neu generierten Bindung beteiligt sein.

Gemäß einer weiteren bevorzugten Ausführungsform der vorliegenden Erfindung können mindestens 6, bevorzugt mindestens 8, oberflächenexponierte Aminosäuren eines Ubiquitins, bevorzugt Säuger- oder humanes Ubiquitin, modifiziert sein, wobei eine Substitution als Modifikation bevorzugt ist. Diese mindestens 6 oberflächenexponierten modifizierten Aminosäuren bilden dann den Bereich mit Bindungsaffinität zu dem vorbestimmten Bindungspartner. Dabei ist besonders bevorzugt, wenn sich mindestens 4, bevorzugt mindestens 6, weiterhin bevorzugt mindestens 8 der oberflächenexponierten Aminosäuren in einem beta-Faltblatt-Bereich, d.h. in einem beta-Faltblattstrang oder verteilt auf mehrere Betastränge befinden. Weiterhin ist bevorzugt, dass mindestens 5 der modifizierten, bevorzugt substituierten, Aminosäuren sich in direkter Nachbarschaft in der Primärsequenz zueinander befinden.

In einer weiteren bevorzugten Ausführungsform der vorliegenden Erfindung sind in dem Protein Aminosäuren in mindestens zwei, bevorzugt genau zwei, der vier Betastränge modifiziert, um eine neue Bindungseigenschaft zu generieren. Ebenfalls bevorzugt ist eine Modifikation in drei oder vier der vier Betastränge zur Erzeugung einer zuvor nicht vorhandenen Bindungseigenschaft gegenüber einem ausgewählten Bindungspartner.

Besonders bevorzugt ist, wenn Aminosäuren in dem aminoterminalen und dem carboxyterminalen Strang modifiziert werden, vorzugsweise substituiert, um neue Bindungseigenschaften zu generieren. Bevorzugt ist dabei weiterhin, wenn zusätzlich Aminosäuren in dem an den carboxyterminalen beta-Faltblattstrang angrenzenden Loop modifiziert werden, vorzugsweise substituiert werden.

Besonders bevorzugt ist eine Modifikation, vorzugsweise Substitution, an den folgenden Positionen eines Säuger-Ubiquitins, vorzugsweise humanem Ubiquitin: 2, 4, 6, 62, 63, 64, 65, 66. Diese Aminosäuren bilden einen zusammenhängenden, oberflächenexponierten Bereich auf der Oberfläche des Ubiquitins, der sich zur Generierung von modifizierten Proteinen mit einer vorher nicht vorhandenen Bindungsaffinität gegenüber einem Bindungspartner als besonders geeignet erwies.

Die Substitution von Aminosäuren zur Erzeugung neuer Bindungseigenschaften kann erfindungsgemäß mit beliebigen Aminosäuren erfolgen, d.h. bei der Modifikation zur Erzeugung neuer Bindungseigenschaften muss nicht darauf geachtet werden, dass die Aminosäuren eine ähnliche chemische Eigenschaft bzw. eine ähnliche Seitenkette aufweisen wie die ausgetauschten Aminosäuren, sondern es stehen hierfür beliebige Aminosäuren zur Verfügung.

Gemäß dieser Erfindung können als Proteine, die zur Erzeugung vorher nicht vorhandener Bindungsaffinitäten gegenüber ausgewählten Bindungspartnern modifiziert werden, schon vor dieser Modifikation andere Modifikationen wie Substitutionen, Insertionen, Deletionen und/oder chemische Modifikationen aufweisen, so dass biologische und/ oder proteinchemische Funktionen des Proteins ausgeschaltet oder neue hinzugekommen sind. Bspw. können so von vornherein die Bindungseigenschaften von Ubiquitin an seine natürlichen Bindungspartner ausgeschaltet werden.

So wurde in einer Ausführungsform der vorliegenden Erfindung eine Variante des humanen Ubiquitins verwendet, in welcher die Aminosäure Phenylalanin an Position 45 durch Tryptophan substituiert wurde. Dadurch konnte unter Beibehaltung der Struktur und Stabilität von Ubiquitin ein Protein bereitgestellt werden, das aufgrund des Austausches verbesserte spektroskopische Eigenschaften gegenüber Ubiquitin aufweist.

In einer anderen Ausführungsform der vorliegenden Erfindung wurden bspw. bei humanem Ubiquitin die Positionen 44, 48, 54, 70, 72 und 75 substituiert und die Aminosäure 76 deletiert. Dadurch konnten unter Beibehalt der Struktur und Stabilität von Ubiquitin ein Protein bereitgestellt werden, das keine natürlichen Funktionen des Ubiquitins mehr ausführen kann. Insgesamt erhält man, wenn man ein solches bereits vormodifiziertes Ubiquitin dazu verwendet, neue, vorher nicht vorhandene Bindungseigenschaften zu erzeugen, bevorzugt ein Ubiquitin, bei dem insgesamt mindestens 10, bevorzugt mindestens 15 Aminosäuren des Wildtyp-Ubiquitins oder generell eines Säuger-Ubiquitins ausgetauscht sind. Gemäß einem Ausführungsbeispiel konnte so ein modifiziertes Ubiquitin erhalten werden, dass unter Beibehaltung seiner ursprünglichen Struktur 14 Substitutionen und eine Deletion aufwies. Bezogen auf die Gesamtzahl der Aminosäuren des Ubiquitins entspricht dies einem Prozentsatz von etwa 20%. Dies war äußerst überraschend und nicht zu erwarten, da üblicherweise bereits ein sehr viel geringerer Prozentsatz genügt, um die Faltung des Proteins zu stören.

Der Schritt der Modifikation der ausgewählten Aminosäuren erfolgt erfindungsgemäß bevorzugt durch Mutagenese auf Genebene durch Random-Mutagenese, d.h. einem zufälligen Austausch der ausgewählten Aminosäuren. Bevorzugt erfolgt die Modifikation in Schritt d) mittels gentechnischer Verfahren zur Veränderung einer zu dem entsprechenden Protein gehörenden DNA. Bevorzugt erfolgt die Expression des Proteins dann in prokaryontischen oder eukaryontischen Organismen.

Ein modifiziertes Protein kann erfindungsgemäß ebenfalls bevorzugt durch chemische Synthese hergestellt werden. Bei dieser Ausführungsform sind dann die Schritte c) bis d) des Anspruch 1 in einem Schritt realisiert.

<u>Selektion bzw. Ermittlung der Aminosäuren mit Bindungsaffinität zu einem vorbestimmten Bindungspartner:</u>

Nach Erstellen einer Proteinbibliothek durch Modifikation ausgewählter Aminosäuren werden die modifizierten Proteine erfindungsgemäß mit einem vorbestimmten Bindungspartner in Kontakt gebracht, um ggf. eine Bindung der Partner aneinander zu ermöglichen, wenn eine Bindungsaffinität besteht.

Das Inkontaktbringen erfolgt erfindungsgemäß bevorzugt mittels eines geeigneten Präsentations- und Selektionsverfahrens wie dem Phage-Display-, Ribosomal Display-, mRNA-Display- oder Cell-Surface-Display-, Yeast Surface Display- oder Bacterial Surface Display-Verfahren, bevorzugt mittels des Phage-Display-Verfahrens. Zur vollständigen Offenbarung wird auch auf folgende Literaturstellen Bezug genommen: Hoess, Curr. Opin. Struct. Biol. 3 (1993), 572-579; Wells and Lowmann, Curr. Opin. Strut. Biol. 2 (1992), 597-604; Kay et al., Phage Display of Peptides and Proteins - A Laboratory Manual (1996), Academic Press. Die oben bezeichneten Verfahren sind dem Fachmann bekannt und können erfmdungsgemäß, einschließlich Abwandlungen hiervon, eingesetzt werden.

Das Ermitteln, ob das modifizierte Protein eine quantifizierbare Bindungsaffinität zu einem vorbestimmten Bindungspartner aufweist, kann erfindungsgemäß bevorzugt durch eines oder mehrere der folgenden Verfahren erfolgen: ELISA, Plasmon-Oberflächenresonanz-Spektroskopie, Fluoreszenz-Spektroskopie, FACS, Isothermale Titrationskalorimetrie und Analytische Ultrazentrifugation.

Als Beispiel für ein erfindungsgemäßes Selektionsverfahren für Varianten des Ubiquitins mit Bindungseigenschaften ist im Folgenden eine auf diese Anwendung adaptierte Form des *Phage Display*-Verfahrens beschrieben. Ebenso können z. B. Verfahren zur Präsentation auf Bakterien (Bacterial Surface Display; Daugherty et al., 1998) oder Hefezellen (Yeast Surface Display; Kieke et al., 1997) bzw. zellfreie Selektionssysteme wie das *Ribosome Display* (Hanes und Plückthun, 1997; He und Taussig, 1997) oder das *cis Display* (Odegrip *et al.,* 2003) oder das mRNA-*Display* Anwendung finden. Hierbei wird eine transiente physikalische Verknüpfung von Geno- und Phänotyp durch die Kopplung der Proteinvariante mit der dazugehörigen mRNA über das Ribosom erreicht.

Beim hier beschriebenen *Phage Display-Verfahren* werden rekombinante Varianten des Ubiquitins auf filamentösen Phagen präsentiert, während die kodierende DNA der präsentierten Variante gleichzeitig in einzelsträngiger Form im Phagenkapsid verpackt vorliegt. So können im Zuge einer Affinitätsanreicherung Varianten mit bestimmten Eigenschaften aus einer Bibliothek selektiert und deren genetische Information durch die Infektion geeigneter Bakterien amplifiziert bzw. einem weiteren Anreicherungszyklus zugeführt werden. Die Präsentation des mutierten Ubiquitins auf der Phagenoberfläche wird durch die genetische Fusion mit einer aminoterminalen Signalsequenz - bevorzugt der PelB-Signalsequenz - und einem Hüll- oder Oberflächenprotein des Phagen - bevorzugt die carboxyterminale Fusion mit dem Hüllprotein pIII oder einem Fragment hiervon - erreicht. Weiterhin kann das kodierte Fusionsprotein noch weitere funktionelle Elemente enthalten, wie z. B. ein Affinitätsanhängsel oder ein Antikörper-Epitop für den Nachweis und/oder die affinitätschromatographische Reinigung oder eine Protease-Erkennungs-sequenz für die spezifische Spaltung des Fusionsproteins im Verlauf der Affinitäts-anreicherung. Desweiteren kann sich zwischen dem Gen für die Ubiquitin-Variante und dem kodierenden Bereich für das Phagenhüll-Protein oder dessen Fragment z. B. ein Amber-Stopcodon befinden, welches in einem geeigneten Supressorstamm während der Translation zum Teil durch den Einbau einer Aminosäure überlesen wird.

Der bakterielle Vektor, der für das Selektionsverfahren im Zuge der Isolierung von Varianten des Ubiquitins mit Bindungseigenschaften für ein vorher bestimmtes Hapten oder Antigen geeignet ist und in den die Genkassette für das beschriebene Fusionsprotein inseriert wird, wird als Phasmid bezeichnet. Dieser weist u. a. die intergenische Region eines filamentösen Phagen (z. B. M13 oder f1) oder eines Teils hiervon auf, was bei Superinfektion der phagemid-tragenden Bakterienzelle mit Helferphagen wie z. B. M13K07 zum Verpacken eines geschlossenen Strangs der Phasmid-DNA in eine Phagenhülle führt. Die so entstandenen Phagemide werden vom Bakterium sekretiert und präsentieren die jeweilige kodierte Ubiquitin-Variante - aufgrund deren Fusion mit dem Hüllprotein pIII oder dessen Fragment - auf dessen Oberfläche. Durch das Vorhandensein von nativen pIII-Hüllproteinen im Phagemid, bleibt dessen Fähigkeit geeignete Bakterienstämme erneut zu infizieren und damit die Möglichkeit zur Vermehrung der entsprechenden DNA, erhalten. Damit wird die physikalische Verknüpfung zwischen dem Phänotyp der Ubiquitin-Variante - d. h. deren potentielle Bindungseigenschaft - und deren Genotyp gewährleistet. Im vorliegenden Beispiel wird das dafür konstruierte Phasmid pMUBI-1 (Abb. 3) für die Insertion der kodierenden Sequenzen von Ubiquitin-Varianten und zur Gewinnung von Phagemiden verwendet. Gewonnene Phasmide können hinsichtlich der Bindung der auf ihnen präsentierten Ubiquitin-Variante an vorbestimmte Haptene oder Antigene mittels dem Fachmann bekannter Methoden selektiert werden. Hierzu können die präsentierten Ubiquitin-Varianten transient an z. B. auf Mikrotiterplatten gebundene Zielsubstanz immobilisiert werden und nach Abtrennung nicht-bindender Varianten spezifisch eluiert werden. Die Elution erfolgt bevorzugt mit basischen Lösungen wie z. B. 100 mM Triethylamin. Alternativ kann die Elution unter sauren Bedingungen, Proteolyse oder die direkte Zugabe infizierbarer Bakterien erfolgen. Die so gewonnenen Phagemide können reamplifiziert werden und durch aufeinanderfolgende Zyklen von Selektion und Amplifikation Ubiquitin-Varianten mit Bindungseigenschaften für ein vorher bestimmtes Hapten oder Antigen angereichert werden.

Die weitere Charakterisierung der auf diese Weise gewonnenen Ubiquitin-Varianten kann als Phagemid d. h. in Fusion mit dem Phagen oder nach Umklonierung der entsprechenden Genkassette in einen geeigneten Expressionsvektor als lösliches Protein erfolgen. Die entsprechenden Methoden sind dem Fachmann bekannt oder in der Literatur beschrieben. Die Charakterisierung kann z.B. die Ermittlung der DNA- und damit der Primärsequenz der isolierten Varianten umfassen. Weiterhin kann die Affinität und die Spezifität der isolierten Varianten z. B. mittels immunologischen Standardmethoden wie ELISA oder Plasmon-Oberflächenresonanz-Spektroskopie, Fluoreszenz-Spektroskopie, FACS, Isothermale Titrationskalorimetrie oder Analytische Ultrazentrifugation ermittelt werden. Hinsichtlich der Stabilitätsanalyse sind dem Fachmann z. B. spektroskopische Methoden in Verbindung mit chemischer oder physikalischer Entfaltung bekannt.

Bei dem ebenfalls verwendeten *Ribosomal Display* Verfahren werden Varianten des Ubiquitins mittels eines zellfreien Transkriptions-/Translationssystems hergestellt und als Komplex mit der entsprechenden mRNA sowie dem Ribosom präsentiert. Dabei wird von einer wie oben beschriebenen DNA-Bibliothek, in der die Gene der Varianten mit entsprechenden regulatorischen Sequenzen für die Expression und die Proteinbiosynthese fusioniert vorliegen, ausgegangen. Durch die Deletion des Stop-Kodons am 3'-Ende der Gen-Bibliothek sowie geeigneten experimentellen Bedingungen (niedrige Temperatur, hohe $Mg^{2+}$-Konzentration) bleibt der ternäre Komplex aus naszierendem Protein, mRNA und Ribosom während der *in vitro* Transkription / Translation erhalten.

Diese Komplexe können hinsichtlich der Bindung der auf ihnen präsentierten Ubiquitin-Variante an vorbestimmte Haptene oder Antigene mittels dem Fachmann bekannten Methoden selektiert werden. Hierzu können die auf dem Ribosomen-Komplexen präsentierten Ubiquitin-Varianten transient an z. B. auf Mikrotiterplatten gebundene Zielsubstanz immobilisiert bzw. an nach Bindung in Lösung an magnetische Partikel gebunden werden. Nach Abtrennung nicht-bindender Varianten kann die genetische Information bindungsaktiver Varianten durch Zerstörung des Ribosomen-Komplexes in Form der mRNA spezifisch eluiert werden. Die Elution erfolgt bevorzugt mit 50 mM EDTA. Die so gewonnene mRNA kann isoliert und mit geeigneten Methoden in DNA rückübersetzt (Reverse Transkriptase Reaktion) und die so gewonnene DNA reamplifiziert werden.

Durch aufeinanderfolgende Zyklen von *in vitro* Transkription / Translation, Selektion und Amplifikation können Ubiquitin-Varianten mit Bindungseigenschaften für ein vorher bestimmtes Hapten oder Antigen angereichert werden.

Die weitere Charakterisierung der auf diese Weise gewonnenen Ubiquitin-Varianten kann nach Umklonierung der entsprechenden Genkassette in einen geeigneten Expressionsvektor als lösliches Protein wie oben ausgeführt erfolgen. Die entsprechenden Methoden sind dem Fachmann bekannt oder in der Literatur beschrieben.

Bevorzugt schließt sich an(d) den Schritt der Ermittlung der Proteine mit einer Bindungsaffinität gegenüber einem vorbestimmten Bindungspartner noch ein Schritt der Isolierung und/oder Anreicherung des ermittelten Proteins an.

Nach der Expression der erfindungsgemäß modifizierten Proteine mit dem Ubiquitin-artigen Faltungsmotiv können diese durch an sich bekannte Methoden weiter aufgereinigt und angereichert werden. Die ausgewählten Verfahren hängen von einigen, dem Fachmann an sich bekannten Faktoren ab, beispielsweise dem verwendeten Expressionsvektor, dem Wirtsorganismus, dem späteren Einsatzgebiet, der Größe des Proteins und anderen Faktoren. Zur erleichterten Aufreinigung können die erfindungemäß modifizierten Proteine mit weiteren Peptidsequenzen fusioniert werden, welche eine erhöhte Affinität zu Trennmaterialien zeigen. Solche Fusionen werden bevorzugt so ausgewählt, daß sie die Funktionalität des Ubiquitin-Proteins nicht nachteilig verändern, oder durch Einfiigung spezifischer Proteaseschnittstellen können sie nach der Aufreinigung wieder abtrennbar sein. Derartige Methoden sind dem Fachmann ebenfalls an sich bekannt.

Erfindungsgemäß und insbesondere nach den gerade beschriebenen Verfahren können generell Varianten des Ubiquitins mit Bindungsaffinität für einen vorher bestimmten Bindungspartner wie z. B. ein Hapten oder Antigen isoliert, werden.

Als Bindungspartner für die erfindungsgemäß bereitgestellten modifizierten Proteine können alle biologisch und medizinisch aktiven und relevanten Moleküle eingesetzt werden. Nachfolgend werden beispielhaft mögliche Bindungspartner genannt. Es ist jedoch darauf hinzuweisen, daß diese Liste noch durch eine Vielzahl anderer möglicher Liganden ergänzt werden kann. Ähnlich wie beim Verhältnis Antikörper zu Antigen ist der Liste der potentiellen Bindungspartner durch weitere potentielle Liganden ergänzbar.

Bevorzugt handelt es sich bei dem Bindungspartner um einen biologischen Rezeptor, bevorzugt G-Protein gekoppelter Rezeptor (GPCR; z. B. humaner GLP-1 Rezeptor, humaner PTH Rezeptor), oder EGF-Rezeptor, HER2, HER3, VEGF/R1-4, Ep-CAM, oder einen Liganden oder eine Domäne hiervon, einen Tumormarker (Prostate-Specific Membrane Antigen (PSMA)), Zytokine (Tumor Necrose Factor Alpha (TNF-$\alpha$), Tumor Necrose Factor Beta(TNF$\beta$)), Interleukine (z. B. IL-2, IL-6, IL-11, IL-12), Wachstumsfaktoren (z. B. NGF (Nerve Growth Factor) und dessen Proform ProNGF, BMPs, EGF, MIA, MIA-2, FGFs, Vascular Endothelial Growth Factor (VEGF), PDGF, P1GF, IGFs), Kinasen, Integrine (z. B. Glycoprotein RezeptorIIb/IIIa (GPIIb/IIIa)), HSA (Human Serum Albumin), F4-Fimbrien, T- und B-Zell Antigen, bevorzugt CD4, CD11, CD14, CD16, CD20, CD22, CD25, CD34, CD47, CD56, CD83, CD154, CTLA-4, ein Immunglobulin oder einen Teil hiervon, bspw. einen gesamten Antikörper, (z. B. Immunglobulin G, E, M), ein $F_C$-Teil von z. B. humanem Immunglobulin M oder einen Bereich eines Antikörpers im Bereich der Antigenbindungsstelle, oder ein Zucker (Lewis Y, Lewis X), oder ein Toxin bspw. Mycotoxin oder ein Hormon, bspw. Hydrocortison.

Es ist ein besonderer Vorteil der vorliegenden Erfindung, dass die modifizierten Proteine oder Ubiquitine sowohl intra- wie auch extrazellulär aktiv sind, d. h. ihre entsprechenden Bindungspartner innerhalb und außerhalb einer Zelle binden.

Es ist ein besonderer Vorteil, dass die modifizierten Proteine oder Ubiquitine der vorliegenden Erfindung sowohl Haptene, also kleine Moleküle, als auch Antigene, also große Moleküle, wie Proteine quantifizierbar binden können. Durch diese Variabilität der erfindungsgemäßen modifizierten Proteine werden somit universell für eine große Breite von Bindungspartnern mögliche neu erzeugte Bindungspartner bereitgestellt.

Die Proteine der vorliegenden Erfindung können weiterhin zum Nachweis und zur quantitativen Bestimmung sowie zur Trennung und Isolierung des entsprechenden Bindungspartners verwendet werden.

Eine weitere Anwendung liegt in der Diagnose und Behandlung von Krankheiten, an denen der entsprechende Bindungspartner beteiligt ist.

Wie bereits erwähnt, betrifft die vorliegende Erfindung auch die gezielte Veränderung einzelner Aminosäurepositionen, die außerhalb der *de novo* generierten, künstlichen Bindungsstelle liegen. So können z. B. Positionen, welche im natürlichen Ubiquitin mit Aminosäuren besetzt sind, die für dessen biologische Funktion verantwortlich sind, mit anderen Aminosäuren besetzt werden. Auf diese Weise wird ein - hinsichtlich seiner biologischen Funktionen, die z. B. die Wechselwirkung mit Enzymen der Ubiquitinilierungs-Kaskade betreffen, - inaktives Ubiquitin-Proteingerüst erhalten, welches jedoch hinsichtlich seiner Struktur und der proteinchemischen Eigenschaften weitestgehend mit dem Ausgangsprotein identisch ist. Dies kann z. B. durch die Bestimmung der Expressionsrate in *E. coli,* die Analyse der Stabilität mit spektroskopischen Methoden wie Fluoreszenz- oder Zirkulärem Dichroismus-Messung in Verbindung mit chemischer oder physikalischer Entfaltung oder die Detektion in immunologischen Standard-Tests wie ELISA erfolgen.

Beispielsweise kann durch die Substitutionen Arg54 und Arg72 jeweils zu Leu die Interaktion zum Ubiquitin-aktivierendem Enzym E1 unterbunden werden (Burch und Haas, 1994). Weiterhin sind die Aminosäuren Lys48 sowie Va170 bis Gly76 u. a. an der Wechselwirkung mit dem Ubiquitin-konjugierendem Enzym E2 beteiligt, was durch entsprechende Substitutionen unterbunden wird (Miura *et al.,* 1999). Ferner erfolgt die Verknüpfung von Ubiquitin mit für den proteolytischen Abbau bestimmte Proteine bzw. die kovalente Verknüpfung zu Polyubiquitin-Ketten selektiv über die Reste Gly75 und Gly76 und kann durch entsprechende Substitutionen verhindert werden. Schließlich verhindern die Austausche Ile44Ala und Val70Ala weitestgehend den Kontakt von Ubiquitin zur 26S-Protease und damit die Degradation von Ubiquitin-markierten Proteinen (Beal *et al.,* 1996). In einer bevorzugten Ausführung der Erfindung dient dementsprechend ein modifiziertes Ubiquitin-Proteingerüst mit den Substitutionen Ile44Ala, Lys48Arg, Arg54Leu, Val70Ala, Arg72Leu, Gly75Ala sowie mit der Deletion von Gly76 als Ausgangspunkt für die Gewinnung von modifizierten Ubiquitinen mit neuen Bindungseigenschaften. Dieses modifizierte Ubiquitin-Proteingerüst ist hinsichtlich seiner Struktur und seiner proteinchemischen Eigenschaften, d.h. Stabilität, Faltung, Produzierbarkeit in *E. coli,* Wechselwirkung mit anti-Ubiquitin Antiseren etc. weitestgehend mit Ubiquitin identisch, was mit den oben genannten Methoden determiniert wurde.

Es war überraschend, dass die in den verschiedenen Zitaten getrennt genannten Modifikationen in einem einzigen Ubiquitin zusammengefasst werden konnten, ohne dessen Struktur oder Stabilität wesentlich zu verändern.

Überraschenderweise ist es mit dem beschriebenen Ansatz, der dieser Erfindung zugrunde liegt, möglich, modifizierte Proteine auf der Basis von Proteinen mit einem Ubiquitin-artigen Faltungsmotiv zu gewinnen, die zum einen neu generierte Bindungseigenschaften aufweisen und andererseits weitestgehend die proteinchemischen Eigenschaften des Ubiquitins besitzen. Dabei weisen die Ubiquitin-basierten modifizierten Proteine Dissoziationskonstanten von vorzugsweise $10^{-6}$ M oder kleiner, bspw. $10^{-6}$ - $10^{-12}$ M auf, die mit denen von Antikörpern bzw. deren Fragmenten vergleichbar sind. Weiterhin gelingt es erstaunlicherweise, modifizierte Proteine mit spezifischen Bindungseigenschaften wahlweise gegen große Moleküle wie Proteine oder auch kleiner Moleküle wie Haptene oder Hormone zu generieren. Dabei können die vorher definierten Zielsubstanzen oder Zielsubstanzklassen mit hoher Selektivität gebunden werden. Die beobachtete Funktionalität der *de novo* generierten, künstlichen Bindungsstelle hinsichtlich Affinität und Spezifität war nicht von vornherein zu erwarten, da sich weite Bereiche der Bindungsstelle in dem im allgemeinen als starr und unflexibel angesehenen beta-Faltblatt befinden. Im Gegensatz hierzu werden natürliche universelle Bindungsstellen - wie z. B. die von Antikörpern - durch flexible Bogen- ("Loop-") strukturen ausgebildet.

Überraschend ist weiterhin, dass das im vorliegenden Fall verwendete Ubiquitin-Proteingerüst die realisierten tiefgreifenden Veränderungen der Primärsequenz offensichtlich toleriert, ohne dass die Faltung der Polypeptidkette beeinträchtigt wird. Dies ist nicht nur im Hinblick auf die Anzahl der Aminosäure-Austausche - ca. 20 % der Sequenz des Wildtyp-Ubiquitins konnten verändert werden - unerwartet, sondern auch aufgrund der Lage der veränderten Positionen im Proteingerüst. So war z. B. eine Toleranz gegenüber Austauschen innerhalb des im allgemeinen als starr und unflexibel angesehenen beta-Faltblattes nicht *a priori* zu erwarten, insbesondere von direkt benachbarten Aminosäuren nicht.

Ausgehend von den erhaltenen mutierten DNA-Sequenzen der Ubiquitin-basierten modifizierten Proteine, lassen sich diese mittels bekannten gentechnischen Methoden herstellen. Bevorzugt ist dabei - aufgrund der geringen Kosten und der hohen Ausbeute - die Produktion in einem prokaryontischen Wirt, was die mögliche Verwendung von eukaryontischen oder zellfreien Systemen jedoch nicht ausschließt. Im allgemeinen wird nach Insertion der DNA-Sequenz in einen geeigneten Expressionsvektor und der Transformation, Transfektion oder Infektion entsprechender Organismen das zellfremde modifizierte Protein durch das bakterielle Transkriptions-/Translationssystem synthetisiert. Hierbei kann das Herstellungsverfahren auf einzelne modifizierte Proteine mit den neuen Bindungseigenschaften angepasst werden. So kann z. B. bei der Verwendung von *E. coli* als Wirt das Ubiquitin-basierte modifizierte Protein mittels einer geeigneten Signalsequenz in den periplasmatischen Raum sekretiert werden oder im Cytosol hergestellt werden. Wenn das modifizierte Protein dieser Erfindung in der Zelle nicht gefaltet wird und aggregiert, ist ebenso die funktionelle Rückfaltung aus solchen Einschlußkörpern möglich. Zellfreie Systeme können z. B. bei Varianten mit niedriger Expressionsrate oder toxischem Effekt auf den Wirtsorganismus vorteilhaft sein. Geeignete gentechnische Verfahren zur Herstellung bzw. Reinigung rekombinanter Proteine sind dem Fachmann bekannt und in der Literatur beschrieben (z. B. Sambrook *et al., 2001).*

Dementsprechend ist es möglich, Ubiquitin-basierte modifizierte Proteine mit neuer Bindungsaffinität durch die Generierung einer künstlichen Bindungsstelle bereitzustellen, deren hochvariable Oberfläche die molekulare Erkennung vorher festgelegter Liganden wie z. B. Haptene, Peptide, Proteine und anderer Makromoleküle oder kleinere Moleküle wie bspw. Hormone erlaubt.

Darüber hinaus können aufgrund der vielfältigen Oberflächeneigenschaften des randomisierten Bereiches mittels geeigneter Selektionsmethoden auch Ubiquitin-basierte modifizierte Proteine mit anderen Eigenschaften als Bindungseigenschaften erhalten werden. Dies kann z. B. eine neue, vorher nicht vorhandene katalytische Aktivität für eine vorher definierte chemische Reaktion sein. Diese Eigenschaft ergibt sich bspw., wenn der Bindungspartner ein Molekül oder Übergangszustand ist, das/der von dem modifizierten Protein derart gebunden wird, dass dadurch die jeweilige Reaktion katalysiert wird.

Die vorliegende Erfindung beinhaltet somit die Bereitstellung von Molekülen wie Ubiquitin als Gerüstmolekül zur Einführung neuer, vorher nicht vorhandener Bindungseigenschaften.

Weiterhin kann gemäß dieser Erfindung eine Genbibliothek in Schritt d) des Verfahrens erstellt werden, z.B. mittels Random-Mutagenese. Die vorliegende Erfindung umfasst gemäß einer Ausführungsform auch derartig hergestellte Genbibliotheken. Insbesondere sind solche Genbibliotheken umfasst, die aus humanem Ubiquitin erstellt wurden, das an den Aminosäurepositionen 2, 4, 6, 62, 63, 64, 65 und 66 substituiert wurde.

Weiterhin kann das Proteingerüst außerhalb der künstlichen Bindungsstelle gerichtet modifiziert werden, um dem modifizierten Protein zusätzliche Funktionen zu verleihen. Dies kann z. B. die Einführung zusätzlicher oder der Austausch einzelner Aminosäuren oder Peptide - bevorzugt am Amino- und Carboxyterminus - beinhalten, um Proteinkonjugate durch chemische Kopplung mit geeigneten Reagenzien zu erhalten. Solche Fusionen können auch mittels gentechnischer Methoden direkt durch die Verknüpfung des Gens des modifizierten Proteins mit dem des Fusionspartners hergestellt werden. Dies wird - im Vergleich zu den Antikörpern - dadurch vereinfacht, dass lediglich ein Fremdgen und daher nur eine Polypeptidkette durch den bakteriellen Wirt exprimiert bzw. funktionell gefaltet werden muß. Solche Fusionspartner können Enzyme, Cytotoxine, Bindungs- und Multimerisierungsdomänen oder auch modifizierte Proteine mit derselben bzw. unterschiedlicher Bindungsspezifität sein.

Das erfindungemäß bereitgestellte modifizierte Protein mit dem Ubiquitin-typischen Faltungsmotiv kann mit einem Protein gleicher Spezifität oder auch unterschiedlicher Spezifität ortsgerichtet und kovalent verknüpft sein, um auf diese Weise bivalente bzw. bispezifische Bindungseigenschaften gegenüber einem oder mehreren Bindungspartnern zu erhalten.

So können bspw. zwei gleiche Varianten des Ubiquitins, die durch die oben beschriebene Vorgehensweise erhalten wurden und welche dasselbe Antigen binden, ortsgerichtet über einen singulären, zusätzlich eingeführten Cysteinrest mittels dem Fachmann bekannter Methoden miteinander kovalent verknüpft werden. Solche bivalenten Bindungsproteine zeichnen sich erfahrungsgemäß gegenüber monovalenten durch eine apparent stärkere Bindung aus (Aviditätseffekt).

Ebenso können zwei Varianten des Ubiquitins, welche unterschiedliche Antigene binden, mit entsprechenden Methoden in einer Weise miteinander verknüpft werden, dass ausschließlich bispezifische Bindungsmoleküle erhalten werden. Für die selektive Bildung solcher Heterodimere können Polypeptide, die aus entweder positiv oder negativ geladenen Aminosäuren bestehen und jeweils mit den carboxyterminalen Enden der Fusionspartner fusioniert sind, verwendet werden. Diese sogenannten Polyionischen Anhängsel werden - bei Verwendung gegensätzlich geladener Aminosäuren - selektiv elektrostatisch miteinander wechselwirken und die Bindungsproteine unterschiedlicher Spezifität in gewünschtem 1: Verhältnis miteinander verknüpfen. In der Krebstherapie können solche bispezifischen Agenzien durch das Adressieren entsprechender Oberflächenstrukturen bspw. Tumorzellen mit Effektorzellen des Immunsystems in Kontakt gebracht werden, um erstere gezielt zu zerstören.

Weiterhin können Ubiquitin Varianten, die mit einer der oben beschriebenen Selektionsmethoden isoliert wurden und daher bereits Bindungseigenschaften für ein bestimmtes Antigen aufweisen, dahingehend modifiziert werden, dass deren Affinität und/oder Spezifität weiter verbessert wird. Hierzu können erneute gerichtete und/oder zufällige Substititionen von Aminosäuren innerhalb und/oder außerhalb der Bindungsstelle durchgeführt werden. Dem Fachmann sind entsprechende Methoden für solche Maturierungsverfahren bekannt. Die Maturierung vorhandener Ubiquitin Varianten kann Affinität und Spezifität umfassen, muß aber nicht hierauf beschränkt sein. Weitere Proteineigenschaften, die verbessert werden können sind bspw. Stabilität, Löslichkeit und Produktionsniveau in Prokaryoten. Im Rahmen der vorliegenden Erfindung wurde so bspw. die Affinität einer Ubiquitin Variante, welche den $F_c$ Teil von Immunglobulin M bindet, durch den gerichteten Austausch zweier Aminosäuren innerhalb der Bindungsstelle um den Faktor 10 erhöht.

Den durch die vorliegende Erfindung bereitgestellten Ubiquitin-basierten modifizierten Proteinen eröffnet sich - analog zu den Antikörpern und deren Fragmenten - ein breites Anwendungsspektrum. Dies umfasst diagnostische und therapeutische Anwendungen sowie chromatographische Verfahren. So können Zielsubstanzen in beliebigen bioanalytischen Tests wie ELISA, Western-Blot o. ä. direkt nachgewiesen werden. Weiterhin eignen sich die erfindungsgemäß modifizierten Proteine, die z. B. Immunglobuline binden und mit einem Enzym oder Fluorophor konjugiert sind, als universelle sekundäre Reportermoleküle in geeigneten Testsystemen. Bevorzugte Anwendung finden die Ubiquitinbasierten modifizierten Proteine aufgrund ihrer vorteilhaften Eigenschaften im therapeutischen Einsatz wie z. B. der Behandlung von Tumor- oder Infektionserkrankungen. Hierbei können Effekte, die auf der Rezeptor- oder Ligandenblockade durch spezifische Bindung des modifizierten Proteins beruhen, ausgenutzt werden. Ebenso können modifizierte Proteine mit den neuen Bindungseigenschaften, die an Oberflächenproteine auf Tumorzellen binden, durch Konjugation mit geeigneten Effektoren solche Zellen gerichtet zerstören bzw. entsprechende Prä-Toxine selektiv im Tumorgewebe aktivieren.

Für die erfindungsgemäß modifizierten und selektierten Proteine steht damit eine breite Palette von Einsatzmöglichkeiten zur Verfügung. Die Einsatzmöglichkeiten liegen nicht nur im medizinisch-pharmazeutischen Bereich, sondern auch im Bereich der Analytik, der Nahrungsmittel- und Futtermittelindustrie, der Nahrungsergänzungsmittel, der Kosmetik, der medizinischen und nicht-medizinischen Diagnostik und Analyse usw. Das Einsatzgebiet hängt selbstverständlich von der Art des ausgewählten Bindungspartners ab.
Im Bereich der humanen und veterinärmedizinischen Therapie und Prophylaxe können durch an sich bekannte Verfahren pharmazeutisch wirksame Arzneimittel hergestellt werden. Diese Zusammensetzungen können, je nach galenischer Herrichtung, intravenös, intraperitonial, intramuskulär, subkutan, transdermal oder durch andere Applikationsmethoden verabreicht werden. Die Art und Weise der pharmazeutischen Herrichtung hängt von der Art der zu behandelnden Erkrankung, von der Schwere der Erkrankung, vom zu behandelnden Patienten und weiteren, dem medizinischen Fachpersonal bekannten Faktoren ab. Die Verabreichung kann entweder parenteral durch Injektion oder Infusion, systemisch, rektal oder weitere übliche Verfahren efolgen.
Die Zusammensetzungen werden so ausgelegt, daß sie eine therapeutisch wirksame Dosis enthalten. Die Menge der zu verabreichenden Dosis hängt vom zu behandelnden Organismus, von der Art der Erkrankung, vom Alter und Gewicht des Patienten und weiteren Faktoren, die an sich bekannt sind, ab.
Die Zusammensetzungen können an sich bekannte Hilfsstoffe enthalten. Hierzu gehören beispielsweise Stabilisierungsmittel, Tenside, Salze, Puffer, Farbstoffe usw.
Die pharmazeutische Zusammensetzung kann in Form einer Flüssigzubereitung, einer Creme, einer Lotion zur topischen Anwendung, eines Aerosols, in Form von Pulvern, Granulaten, Tabletten, Suppositorien oder Kapseln, in Form einer Emulsion oder einer liposomalen Zubereitung vorliegen. Die Zusammensetzungen sind bevorzugt steril, nicht-pyrogen und isotonisch und enthalten die an sich bekannten pharmazeutisch üblichen und verträglichen Additive. Ergänzend wird auf die Vorschriften des U.S. Pharmacopeia Bezug genommen.
Die nachfolgenden Beispiele dienen zur weiteren Veranschaulichung der Erfindung. Die Erfindung wird insbesondere am Beispiel der Modifikation von Ubiquitin dargestellt. Die Erfindung ist jedoch nicht hierauf beschränkt, sondern die nachfolgenden Beispiele zeigen die Ausführbarkeit der Erfindung auf der Grundlage der vorstehenden Beschreibung. Zur vollständigen Offenbarung der Erfindung wird auch auf die in der Anmeldung und im Anhang genannten Literaturstellen Bezug genommen, die vollinhaltlich als Referenz in die Anmeldung mit einbezogen werden.
Die vorliegende Erfindung wird im Folgenden anhand von Beispielen und beigefügten Abbildungen detaillierter erläutert, wobei

**Abb. 1** einen Bereich der *de novo* generierten, künstlichen Bindungsstelle auf der Oberfläche des Wildtyp-Ubiquitins (PDB-Code: 1ubi) zeigt;

**Abb. 2** die Ergebnisse der rechnergestützte Analyse der Proteinstabilität von jeweils $10^4$ Varianten, die an Positionen 2, 4, 6, 62, 63, 64, 65, 66 zufällig substituiert wurden, im Vergleich zum Ubiquitin (WT) und einem Kontrollepitop darstellt;

**Abb. 3** den Phasmidvektor pMUBI-1 für die Verwendung bei der Selektion von Ubiquitin-basierten modifizierten Proteinen mit neuen Bindungseigenschaften erklärt;

**Abb. 4** die Vorgehensweise bei der Konstruktion einer Bibliothek von Ubiquitin-Varianten erläutert;

**Abb. 5** das Ergebnis eines ELISA-Experimentes zum Nachweis der Bindung eines mittels *Phage Display* selektierten Ubiquitin-basierten modifizierten Proteins an das entsprechende Protein zeigt;

**Abb. 6** das Ergebnis eines ELISA-Experimentes zum Nachweis der Bindung eines mittels *Ribosomal Display* selektierten Ubiquitin-basierten modifizierten Proteins an das entsprechende Protein zeigt;

**Abb. 7** das Ergebnis eines BIACORE-Experimentes zum Nachweis der Bindung eines mittels *Ribosomal Display* selektierten Ubiquitin-basierten modifizierten Proteins an das entsprechende Protein zeigt;

**Abb. 8** das Ergebnis eines ELISA-Experimentes zum Nachweis der Bindungsspezifität eines mittels *Phage Display* selektierten Ubiquitin-basierten modifizierten Proteins an das entsprechende Hapten darstellt;

**Abb. 9** das Ergebnis eines ELISA-Experimentes zum Nachweis der verbesserten Bindung einer mittels Sekundärmutagenese erhaltenen Variante von SPU-3-A7, einem durch *Phage Display* selektierten Ubiquitin-basierten Bindungsproteins für IgM-$F_c$, an sein Antigen zeigt; **Abb. 10** das Ergebnis eines Experiments zur ortsgerichteten, kovalenten Kopplung zweier Ubiquitin-basierten Proteine über singuläre, carboxyterminale Cysteinreste mittels Bis-Maleimido-Hexan darstellt.

**Abbildung 1:** Kristallstruktur des Wildtyp-Ubiquitins (PDB-Code: 1ubi; Vijay-Kumar et *al.,* 1987) mit der künstlich generierten Bindungsstelle auf der Oberfläche. Die dreidimensionale Darstellung der Sekundärstruktur-Elemente wurde mit dem Programm PyMOL (De-Lano Scientific, San Francisco) realisiert. Die Reste, die in der beispielhaft hergestelleten Bibliothek zufällig substituiert werden sollen, umfassen die Positionen 2, 4, 6, 62, 63, 64, 65, 66 und sind mit ihren Seitenketten dargestellt.

**Abbildung 2:** Generierung einer Bindungsstelle auf der Oberfläche des Wildtyp-Ubiquitins (PDB-Code: 1ubi). Die Reste, die in der herzustellenden Bibliothek zufällig substituiert werden sollen (in hellgrau dargestellte Positionen 2, 4, 6, 62, 63, 64, 65, 66), wurden durch rechnergestützte Analyse hinsichtlich ihrer Exposition zum Lösungsmittel und des stabilisierenden bzw. destabilisierenden Effekts, den ein Aminosäure-Austausch an den entsprechenden Positionen haben könnte, ausgewählt. Mit Hilfe der Software ProSAII wurde dann die Proteinstabilität von jeweils $10^4$ Varianten im Vergleich zum Ubiquitin (WT) und einer gleichgroßen Stichprobe von Varianten, bei denen die Reste eines "Kontrollepitopes" (in dunkelgrau dargestellte Positionen 24, 28, 31, 32, 35, 37, 38, 39) substituiert worden waren, bestimmt. Als Maß für die Stabilität ist dabei der Wert der kombinierten $C^\alpha/C^\beta$-Potentiale ("zp-comb") angegeben. Dabei wiesen ca. 19 % der *in silico* generierten Varianten, die im Bereich der Bindungsstelle zufällig substituiert worden waren, eine mindestens so hohe Stabilität wie Ubiquitin (WT) auf, während ca. 90 % stabiler als die Träger des "Kontrollepitopes" waren. Die Generierung einer Bindungsstelle durch zufällige Aminosäure-Substitutionen in dem analysierten Bereich sollte demnach von der Proteinstruktur des Ubiquitin toleriert werden, ohne deren Stabilität nachhaltig zu beeinflussen. Diese rechnerische Methode bietet eine Hilfe bei der Wahl geeigneter oberflächexponierter Aminosäuren.

**Abbildung 3:** Der Phasmidvektor pMUBI-1 für die Verwendung bei der Selektion von MUBI-Varianten durch *phage-display*. pMUBI-1 wird zur Herstellung von Phagenbibliotheken verwendet und kodiert unter der Transkriptionskontrolle des Tetrazyklin-Promotor/Operators ($tet^{p/o}$) für ein Fusionsprotein aus der Pe1B-Signalsequenz, dem Gen für die Ubiquitin-Variante, MyCUT-Tag und einem C-terminalen Fragment des Phagenhüllproteins (AS 253 - 406, delta gpIII). Amber bezeichnet die Position des Amber-Stopcodons im Fusionsgen. Die Insertion der mutierten Genkassette des MUBI erfolgt über die beiden *Sfi*I-Schnittstellen. f1Ig, $bla^p$, tetR, Ori, ColE1 und Cat- bezeichnen die intergenische Region des Phagen f1, das Tetrazyklin-Repressorgen unter Kontrolle des β-Lactamase-Promotors, den Replikationsursprung und das Chloramphenicol-Acetytransferase-Gen.

**Abbildung 4:** Vorgehensweise bei der Konstruktion einer *Phage Display*-Bibliothek von Ubiquitin-Varianten.

**Abbildung 5:** Bindung der Ubiqitin-Variante SPU-1-D10 aus der Selektion mittels *Phage Display* gegen recGLP1-R an die rekombinant hergestellte aminoterminale Domäne des humanen GLP-1 Rezeptors im ELISA. Eine entsprechende Anzahl von Vertiefungen einer Mikrotiter-Platte wurde über Nacht bei 4 °C mit recGLP1-R bzw. mit BSA oder der rekombinant hergestellte aminoterminale Domäne des humanen PTH Rezeptors (recPTH-R) belegt. Verbleibende Bindungsplätze wurden durch Inkubation mit 3 % BSA in PBS mit 0,5 % Tween (2 Std., Raumtemperatur (RT)) abgesättigt. SPU-1-D10 wurde in Konzentrationsreihen in PBST 0,1 für 90 min bei 30 °C appliziert. Gebundenes modifiziertes Protein wurde mit anti-Ubiquitin Antiserum (1 : 10 in PBST 0,1, 60 min bei 30 °C) und anti-Kaninchen Antikörper (1 : 2000 in PBST 0.1, 60 min bei 30 °C) konjugiert mit Peroxidase detektiert. Der Nachweis erfolgte mit Hilfe des ImmunoPure-Kits von Pierce. Zwischen den Schritten wurde dreimal mit PBST 0.1 - vor dem chromogenen Nachweis zusätzlich 3 x mit PBS - gewaschen. Die Signalintensität wurde nach Abstoppen der Farbreaktion mit $H_2SO_4$ bei 405 nm detektiert und gegen die jeweilige Konzentration des modifizierten Proteins aufgetragen. Die Ermittlung des $K_D$-Wertes unter Gleichgewichtsbedingungen erfolgte durch nicht-lineare Regression.

**Abbildung 6:** Bindung der Ubiqitin-Variante SPW-11-A1 aus der Selektion mittels *Ribosomal Display* an VEGF im ELISA. Die Durchfiihrung des Experiments entsprach der aus Abb. 5. Die Ermittlung des $K_D$-Wertes unter Gleichgewichtsbedingungen erfolgte durch nicht-lineare Regression.

**Abbildung 7:** Bindung der Ubiqitin-Variante SPU-11-58 aus der Selektion mittels *Ribosomal Display* an VEGF im BIACORE. Auf der Oberfläche eines Kanals eines *CMS-Chips* wurde zunächst VEGF immobilisiert, während ein Referenzkanal unbeladen blieb. Bei der Applikation verschiedener Konzentrationen von SPU-11-58 wurde jeweils das Nettosignal des Bindungsereignis dargestellt. Die Injektionsdauer für alle Lösungen betrug 2 min bei einer Flußgeschwindigkeit von 35 μl/min. Die Bindungssignale verschiedener Konzentrationen von SPU-11-58 (1,3 μM, 0,65 μM, 0,325 μpM, 0,163 μM, 0,081 μM) sind relativ zum Injektionsstart übereinandergelegt dargestellt. Nach jeder Messung wurde die Oberfläche durch einen Puls von 15 s mit 10 mM HCl regeneriert. Die Ermittlung des

$K_D$-Wertes erfolgte mit Hilfe der BIA*evaluation Software* 3.1 (Biacore).

**Abbildung 8:** Bindung der Ubiqitin-Variante SPU-3-H13 aus der Selektion gegen Hydrocortison an Steroide im ELISA. Jeweils drei Vertiefungen einer Mikrotiter-Platte wurden Hydrocortison-, Testosteron- und Östradiol-BSA Konjugat bzw. mit BSA belegt und mit SPU-3-H13 (4 μM) bzw. mit dem im Bereich der Bindungsstelle nicht veränderten Ubiquitin-Proteingerüst (50 μM) jeweils in PBST 0,1 inkubiert (90 min bei 30 °C). Der Bindungsnachweis erfolgte mit Ni-NTA/Peroxidase Konjugat (1: 500 in PBST 0,1, 60 min bei 30 °C) und mit Hilfe des ImmunoPure-Kits von Pierce. Blocking und Waschen: s. Abb. 5. Die Signalintensität wurde nach Abstoppen der Farbreaktion mit $H_2SO_4$ bei 405 nm detektiert und die Mittelwerte von drei Messungen aufgetragen.

**Abbildung 9:** Vergleich der Bindung der Ubiqitin-Varianten SPU-2-A7 und SPU-2-A7(62/63) an $F_c$-IgM nach Affinitätsmaturierung mittels ortsgerichteter Zufallsmutagenese im ELISA. Die Durchfiihrung des Experiments entsprach der aus Abb. 5. Die Ermittlung des $K_D$-Wertes unter Gleichgewichtsbedingungen erfolgte durch nicht-lineare Regression **Abbildung 10:** Analyse der ortsgerichteten, kovalenten Kopplung zweier Ubiquitin-basierter Proteine über singuläre, carboxyterminale Cysteinreste durch SDS-PAGE. Aufgetragen wurden jeweils 20 μl des ungekoppelten Proteins (Spur 1) sowie des Ansatzes nach der Kopplungsreaktion (Spur 2). Im Anschluß an die Elektrophorese wurde das Gel mit Coomassie gefärbt. Die Molmassen des Größenstandards (Spur M) sind in kDa angegeben.

Beispiel 1: Bereitstellung eines synthetischen Ubiquitin-Gens für die Selektion von modifizierten Proteinen mit neu erzeugter Bindungsaffinität

Die gentechnischen Arbeiten wurden sofern nicht anders angegeben nach dem Fachmann geläufigen Standardprotokollen wie z. B. von Sambrook *et al*. (2001) durchgeführt.

Für die Herstellung der DNA-Sequenz (Seq-ID No. 1) für ein modifiziertes Ubiquitin-Proteingerüst mit den Substitutionen Ile44Ala, Lys48Arg, Arg54Leu, Val70Ala, Arg72Leu, Gly75Ala sowie mit der Deletion von Gly76 als Ausgangspunkt für die Gewinnung von künstlichen Bindungsproteinen wurde folgendermaßen vorgegangen: Für die Gensynthese wurde eine PCR-Reaktion in einem Volumen von 50 μl durchgeführt, in dem jeweils 2,5 μl der sechs Oligodesoxynukleotide (Seq-ID No. 2, Seq-ID No. 3, Seq-ID No. 4, Seq-ID No. 5, Seq-ID No. 6, Seq-ID No. 7; jew. 0,1 μM), die in ihrer Basenpaarabfolge insgesamt das zu synthetisierende Gen repräsentierten, als Templates vorlagen. Die Sequenzen der eingesetzten Oligodesoxynukleotide entsprachen dabei jeweils 40 bis 50 Basenpaar langen Abschnitten des kodierenden bzw. nicht-kodierenden DNA-Stranges des künstlichen Gens und überlappten alternierend an ihren 3'- und 5'-Enden mit ca. 15 Basen. Zusätzlich enthielt der Ansatz jeweils 2,5 μl flankierende Primer (Seq-ID No. 8, Seq-ID No. 9; 10 μM) sowie 5 μl, 10x Taq-Puffer (100 mM Tris/HCl pH 9,0, 500 mM KC1, 1 % (v/v) Triton X-100), 3 μl 25 mM $MgCl_2$ und 4 μl dNTP-Mix (je 2,5 mM dATP, dCTP, dGTP, dTTP). Nach Auffüllen mit $H_2O$ wurde der Reaktionsansatz im Thermozykler zwecks Denaturierung für 2 min auf 94 °C erhitzt. Dann wurden 2,5 U der *Taq*-Polymerase (Promega) in der Hitze zugegeben (Hot Start) und das PCR-Programm gestartet. In 25 Zyklen wurde für je 1 min bei 94 °C, 1 min bei 55 °C und für 1,5 min bei 72 °C inkubiert. Eine abschließende Inkubation erfolgte für 5 min bei 72 °C.

Das gewünschte PCR-Produkt wurde mittels analytischer Agarose-Gelelektrophorese identifiziert und aus dem Ansatz mit Hilfe des MinElute Reaction Cleanup-Kit (Qiagen) gereinigt. 1,0 ng der isolierten DNA wurden als Template für eine zweite Amplifizierung verwendet, die diesmal unter Verwendung der *Pfu*-Polymerase (Promega) ebenfalls in einem Volumen von 50 μl realisiert wurde. Dazu wurden 5 μl des mitgelieferten 10x *Pfu*-Puffers (200 mM Tris/HCl, pH 8,8, 20 mM $MgCl_2$, 100 mM KCl, 100 mM $(NH_4)_2SO_4$, 1 % (v/v) Triton X-100, 1 mg/ml BSA) sowie 4 μl dNTP-Mix verwendet und mit $H_2O$ aufgefüllt. Zusätzlich enthielt der Ansatz flankierende Primer (Seq-ID No. 8, Seq-ID No. 9; 10 μM) zur Einführung geeigneter Schnittstellen. Das gewünschte PCR-Produkt wurde mittels präparativer Agarose-Gelelektrophorese isoliert und mit Hilfe des Zero Blunt® TOPO® PCR Cloning Kits (Invitrogen) nach Angaben des Herstellers in den Klonierungsvektor pCR®4Blunt-TOPO® inseriert. Mit dem entsprechenden Ligations-Reaktionsansatz wurden mitgelieferte chemisch kompetente Zellen transformiert und auf einer Agar-Platte mit LB/Amp/Kan-Medium ausplattiert. Die Platte wurde für 16 Std. bei 37 °C bebrütet und gewachsenen Kolonien hinsichtlich des erwünschten Ligationsproduktes analysiert. Dazu wurde Plasmid-DNA im Mini-Maßstab mit Hilfe des Plasmid-Isolierungskits der Firma Qiagen nach Angaben des Herstellers präpariert und einem Restriktionsverdau mit den DNA-Endonukleasen *Nde*I und *Xho*I (New England Biolabs), deren Erkennungssequenzen durch die flankierenden Primer in das PCR-Produkt eingeführt worden waren, unterworfen. Mit Plasmiden, die das erwartete Schnittmuster aufwiesen, wurde im Bereich der inserierten Gen-Kassette mit Hilfe der *Taq* DNA-Polymerase eine DNA-Sequenzanalyse durchgeführt. Dabei wurde der CycleReader™ AutoDNA Sequencing Kit (Fermentas) nach Angaben des Herstellers sowie 0,5 μg Plasmid-DNA und 1,0 pmol des entsprechenden fluoreszenzmarkierten Primers verwendet. Der dabei neusynthetisierte DNA-Strang wurde während der Polymerasereaktion markiert und durch den Einbau von Di-desoxynukleotiden statistisch, aber basenspezifisch terminiert. Die entstandenen fluoreszierenden DNA-Fragmente wurden anschließend in einem Liquor-Sequenzierautomaten durch Polyacrylamid-Harnstoff Gelelektrophorese aufgetrennt und als Bandenmuster für A, C, G, T in benachbarten Spuren sichtbar gemacht.

Genkassetten mit korrekter DNA-Sequenz wurden durch präparativen *Nde*I/*Xho*I-Restriktionsverdau aus dem Klonierungsvektor pCR®4Blunt-TOPO® herausgeschnitten und durch präparative Agarose-Gelelektorphorese isoliert. Die Insertion des Gens für das modifizierte Ubiquitin-Proteingerüst erfolgte in den Expressionsvektor pET20B(-) (Novagen) für die Produktion des entsprechenden Proteins bzw. in den Phasmid-Vektor pMUBI-1 zur Konstruktion einer Bibliothek von Ubiquitin-Varianten.

Beispiel 2: Herstellung einer Bibliothek von Ubiquitin-Varianten

Zur zufälligen ortsgerichteten Mutagenese von 8 Kodons am Amino- bzw. Carboxyterminus des synthetischen Ubiquitin-Gens wurden zwei aufeinanderfolgende PCR-Reaktionen durchgeführt. Der erste Amplifizierungsschritt fand unter Verwendung der *Pfu*-Polymerase (Promega) in einem Volumen von 10 x 50 μl statt. Dazu wurden pro Ansatz 5 μl des mitgelieferten 10x *Pfu*-Puffers sowie 4 μl dNTP-Mix verwendet und mit $H_2O$ aufgefüllt. Weiterhin enthielt der Ansatz jeweils 2,5 μl flankierende Primer (Seq-ID No. 10, Seq-ID No. 11; 10 μM) zur Einführung der gewünschten Basenpaar-Austausche. Als Template wurden 1,0 ng pMUBI-1 verwendet, der das nicht-mutierte synthetische Ubiquitin-Gen trug. Nach Zugabe von 2,5 U der *Pfu*-Polymerase (s. o.) wurde in 25 Zyklen für je 1 min bei 94 °C, 1 min bei 60 °C und für 1,5 min bei 72 °C inkubiert. Eine abschließende Inkubation erfolgte für 5 min bei 72 °C. Für den selektiven Abbau der eingesetzten Matrizen-DNA, wurden pro Reaktionsansatz 10 U *Dpn*I zugegeben und für 1 Std. bei 37 °C inkubiert. Das gewünschte PCR-Produkt wurde mittels präparativer Agarose-Gelelektrophorese und dem QIAquick Gel Extraction Kit (Qiagen) isoliert.

Der zweite Amplifizierungsschritt wurde in einem 1.000 μl-Ansatz durchgeführt, wobei ca. 1,0 ng des in der ersten PCR-Reaktion gewonnenen Produktes eingesetzt und die *Taq*-Polymerase verwendet wurden. Der Reaktionsansatz wurde - adaptiert auf das 20fache Volumen - wie oben ausgeführt aus 10x *Taq*-Puffer, 25 mM $MgCl_2$, dNTP-Mix sowie den flankierenden Primern (Seq-ID No. 12, Seq-ID No. 13; 10 μM), die an ihren 5'-Enden biotinyliert waren und jeweils nicht miteinander kompatible Erkennungssequenzen für die Endonuklease *Sfi*I trugen, pipettiert. Nach Auffüllen mit $H_2O$ wurden 2,5 U der *Taq*-Polymerase in der Hitze zugegeben (s. o.) und das PCR-Programm gestartet. In 25 Zyklen wurde für je 1 min bei 94 °C, 1 min bei 60 °C und für 1,5 min bei 72 °C inkubiert. Eine abschließende Inkubation erfolgte für 5 min bei 72 °C.

Die darauf folgende Spaltung des erhaltenen Amplifizierungsproduktes erfolgte direkt im PCR-Reaktionsansatz. Dazu wurden in einem Gesamtvolumen von 4.000 μl die komplette PCR-Reaktionslösung mit entsprechenden Volumen des mitgelieferten 10x Puffer II (100 mM Tris/HCl, pH 7,9, 100 $MgCl_2$, 500 mM NaCl, 10 mM Dithiothreitol), 10x BSA-Lösung und $H_2O$ gemischt. Weiterhin wurden 4.000 U des Restriktionsenzyms *Sfi*I (New England Biolabs) zugegeben und für 16 Std. bei 50 °C inkubiert. Die DNA wurde aus dem Ansatz mit Hilfe des MinElute Reaction Cleanup Kit (Qiagen) isoliert und in 400 μl sterilem $H_2O$ aufgenommen. Zur Abtrennung von nicht *Sfi*I-geschnittenem PCR-Produkt wurde die isolierte DNA mit dem gleichen Volumen "Binding-Solution" (Dynal), das 1,0 mg/ml magnetische Kügelchen mit oberflächengekoppeltem Streptavidin ("Dynabeads Kilobase Binder") enthielt, gemischt und für 4,5 Std. auf einem Rollenmischer bei Raumtemperatur (RT) inkubiert. Die Kügelchen mit eventuell noch vorhandener biotinylierter DNA wurden präzipitiert, während komplett mit *Sfi*I gespaltene DNA, die keine biotinylierten Enden mehr aufweisen sollte, im Überstand verblieb und über Nacht gefällt wurde. Das so erhaltene mit *Sfi*I gespaltene und an den gewünschten Positionen mutagenisierte Ubiquitin-Gen wurde in sterilem $H_2O$ gelöst, nochmals mit Hilfe des QIAquick PCR Purification Kit (Qiagen) entsalzt und wies schließlich eine Konzentration von 200 fmol/μl in $H_2O$ auf.

Zur Vorbereitung des Empfängervektors wurde das Phasmid pMUBI-1 nach Herstellerangaben mit SfiI geschnitten und das größere (Vektor-)Fragment mittels präparativer Agarose-Gelelektrophorese und dem QIAquick Gel Extraction Kit (Qiagen) isoliert. Um intramolekulare Ligation zu verhindern wurden seine 5'-Enden dephosphoryliert. Hierfür wurden 0,5 U der Alkalische Phosphatase aus Shrimp (*Pandalus borealis*) sowie der mitgelieferte Puffer in einem Gesamtvolumen von 200 μl verwendet. Die Mischung wurde für 90 min bei 37 °C inkubiert, die DNA aus dem Ansatz mit Hilfe des QIAquick PCR Purification Kit (Qiagen) isoliert und nochmals entsalzt (QIAquick PCR Purification Kit). Die DNA des Vektorfragments wies schließlich eine Konzentration von 50 fmol/μl in $H_2O$ auf. Zur Ligation wurden 1,6 pmol des PCR-Fragments und 8,0 pmol des Vektorfragments von pMUBI-1 in Gegenwart von 2 U T4 DNA-Ligase (GibcoBRL) in einem Gesamtvolumen 1.600 μl (50 mM Tris/HCl, pH 7,6, 10 mM $MgCl_2$, 1 mM ATP, 1 mM DTT, 5 % (w/v) PEG-8.000) für drei Tage bei 16 °C inkubiert. Nach Erhitzen des Ansatzes auf 65 °C für 15 min wurde die DNA gefällt. Dazu wurden jeweils 100 μl der Reaktionslösung mit 100 μl Ethanol sowie 10 μl 5 M NaAc, pH 3,0 gemischt und für 16 Std. bei -20 °C gelagert. Anschließend wurde zentrifugiert (60 min, 12.500 g), mit Ethanol (70 % v/v, -20 °C) gewaschen, erneut zentrifugiert und die präzipitierte DNA schließlich in 60 μl sterilem $H_2O$ gelöst.

Zur Elektroporation wurde das Gene Pulser® II-System (Biorad) sowie Küvetten mit Elektrodenabstand von 1,0 mm (Biozym) bei 4 °C im Kühlraum verwendet. Mit jeweils 3,5 μl der oben erhaltenen Lösung wurden elektrokompetente *E. coli* XL1Blue (Stratagene) nach Angaben des Herstellers transformiert. Die erhaltene Zellsuspension wurde auf fünf Agarplatten (20 x 20 cm) mit LB/Chloramphenicol-Medium ausplattiert. Die Platten wurden für 16 Std. bei 37 °C bebrütet und die gewachsenen Kolonien ausgezählt. Die konstruierte Bibliothek enthielt demnach 2,8 x $10^7$ unabhängige Klone von denen jeder 10.000fach in der Bibliothek vorliegen sollte. Die Kolonien wurden dann mit insgesamt 100 ml SOC-Medium mit 10 % (v/v) Glycerin abgeschwemmt und in Aliquots ä 1,0 ml bei -80 °C gelagert. Von den erhaltenen Klonen wurde mit Hilfe des DNA-Miniprep Kits der Firma Qiagen von 12 willkürlich ausgewählten der Phasmid-Vektor isoliert und die DNA-Sequenz im Bereich des mutagenisierten Ubiquitin-Gens analysiert. Dabei wiesen alle Klone funktionelle Sequenzen - d. h. keine Verschiebung des Leserasters durch Insertionen oder Deletionen - sowie qualitativ völlig unterschiedliche Substitutionen an den mutagenisierten Positionen auf. Zufällige Austausche außerhalb der mutagenisierten Bereiche waren nicht vorhanden.

Beispiel 3: Darstellung von Ubiquitin-Varianten auf der Phagenoberfläche und Selektion von Ubiquitin-Varianten gegen Proteine und Haptene

Für die Produktion von Phagemiden mit oberflächen-präsentierten Varianten des mutierten Ubiquitins wurden 100 ml 2xYT/Chloramphenicol-Medium mit 1,0 ml der in Beispiel 2 erhaltenen Glycerin-Kultur inokuliert und für 16 Std. bei 37 °C und 220 rpm inkubiert. Mit 10 ml dieser stationären Kultur wurde 112xYT/Chloramphenicol-Medium angeimpft und bei 37 °C und 220 rpm bis zu einer Zelldichte von $OD_{600}$ = 0,4 geschüttelt. Dann erfolgte die Infektion mit $10^{13}$ cfu M13KO7-Helferphagen und eine Inkubation bei 37 °C ohne Schütteln für 30 min. Nach Zugabe von 50 mg/l Kanamycin wurde für 30 min bei 37 °C und 220 rpm geschüttelt und dann die Genexpression auf pMUBI-1 durch Einstellen der Kultur auf 0,2 mg/l Anhydro-Tetrazyklin (Stammlösung: 2,0 mg/ml in DMF) induziert. Die Inkubatortemperatur wurde dann auf 26 °C erniedrigt und die Kultur für 16 Std. bei 220 rpm geschüttelt. Die Zellen wurden schließlich durch Zentrifugation (30 min, 12.000 g, 4 °C) sedimentiert und verworfen während der Überstand filtriert (0,45 μm) wurde. Die enthaltenen Phagemide wurden durch Zugabe von ¼ Volumen 20 % (w/v) PEG 6.000, 2,5 M NaCl und Inkubation für 1 Std. auf Eis gefällt und durch Zentrifugation (30 min, 12.000 g, 4 °C) präzipitiert. Die Phagemide wurden dann in 4 ml sterilem eiskaltem PBS (137 mM NaCl, 2,7 mM KCl, 8 mM $Na_2HPO_4$, 2 mM $KH_2PO_4$) gelöst, für 30 min auf Eis gelagert und zentrifugiert (30 min, 12.000 g, 4 °C). Der Überstand wurde mit ¼ Volumen 20 % (w/v) PEG 6.000, 2,5 M NaCl versetzt, die Phagemide erneut durch Inkubation für 1 Std. auf Eis gefällt und durch Zentrifugation (30 min, 12.000 g, 4 °C) präzipitiert. Die Phagemide wurden dann wiederum in 4 ml sterilem eiskaltem PBS gelöst, für 30 min auf Eis gelagert und zentrifugiert (30 min, 12.000 g, 4 °C). Der Überstand wurde 1 : 1 mit 4 % (w/v) Rinderserum-Albumin (BSA) in PBS gemischt, für 30 min auf einem Rollenmischer bei RT inkubiert und dann direkt für die Affinitätsanreicherung verwendet.

Als Affinitätsmatrix für die Isolierung von Phagemiden mit oberflächenpräsentierten Ubiquitin-Varianten, die vorher definierte Zielsubstanzen binden sollten, wurden jeweils 24 Vertiefungen einer Mikrotiter-Platte mit der entsprechenden Substanz beschichtet. Als Zielsubstanzen diente die rekombinant hergestellte aminoterminale Domäne des humanen GLP-1 Rezeptors (recGLP1-R; Bazarsuren *et al.,* 2002), der $F_c$-Teil von humanem Immunglobulin M (Fc-IgM) sowie an BSA gekoppeltes Hydrocortison (HC), die über Nacht bei 4 °C an der Mikrotiter-Platte immobilisiert wurden.

Unbelegte Bindungsplätze an der Oberfläche der Mikrotiter-Platte wurden durch Inkubation der Vertiefungen mit jeweils 400 μl 4% BSA in PBS für 90 min bei RT geblockt. Dann wurden pro Vertiefung 100 μl der vorbereiteten Phagemid-Lösung pipettiert und für 90 min bei RT (Raumtemperatur) inkubiert. Ungebundene Phagemide wurden dann durch zweimaliges Waschen mit PBS mit 0,05 % (v/v) Tween 20 (PBST 0,05), zweimalige Inkubation mit 400 μl 4 % BSA in PBS für 5 min und zweimaliges Waschen mit PBS sowie kräftiges Ausklopfen entfernt. An die Affinitätsmatrix gebundene Phagemide wurden schließlich mit Hilfe von 100 μl 100 mM Triethylamin pro Vertiefung und Inkubation für 10 min bei RT eluiert. Die Lösungen mit den eluierten Phagemiden wurden - getrennt nach den jeweiligen Zielsubstanzen - vereinigt und durch Zugabe von ½ Volumen 1 M Tris/HCl, pH 7,4 sofort neutralisiert. Die jeweils erhaltene Lösung wurde zur Infektion von *E. coli* XL1Blue zu 20 ml einer Kultur mit einer Zelldichte von $OD_{600}$= 0,4 überführt und für 30 min bei 37 °C und 220 rpm geschüttelt. Die Vertiefungen der Mikrotiter-Platte wurden erneut gewaschen (5 x PBST 0,05, 1 x PBS) und jeweils 100 μl einer Kultur von *E. coli* XL1Blue mit einer Zelldichte von $OD_{600}$= 0,4 zugegeben. Nach einer Inkubation bei 37 °C für 30 min wurden diese Zellen mit den vorher infizierten vereinigt. Die jeweilige Zellsuspension wurden auf einer Agarplatte (20 x 20 cm) mit LB/Chloramphenicol-Medium ausplattiert und enthielten gewöhnlich zwischen $10^5$ und $10^7$ koloniebildende Klone. Die Platte wurden für 16 Std. bei 37 °C bebrütet, die gewachsenen Kolonien mit 10 ml SOC-Medium mit 10 % (v/v) Glycerin abgeschwemmt und in Aliquots ä 1,0 ml bei -80 °C gelagert.

Zur wiederholten Phagenproduktion und erneuten Zyklen der Affinitätsanreicherung wurde das beschriebe Verfahren wiederholt, wobei für die Anzucht der Bakterienzellen jeweils zehnfach geringere Kulturvolumina verwendet und stringentere Waschbedingungen (2. Runde: 3 x Waschen mit PBST 0,1, dreimalige Inkubation mit 4 % BSA in PBS für 5 min und 3 x Waschen mit PBS; 3. Runde: 6 x Waschen mit PBST 0,1, dreimalige Inkubation mit 4 % BSA in PBS für 5 min und 3 x Waschen mit PBS) gewählt wurden.

Beispiel 4: Isolierung und Charakterisierung von monoklonalen Phagemiden mit spezifischer Bindung an die Zielsubstrate (Einzelphagen-ELISA)

Von den nach der 3. Runde der Affinitätsanreicherung an recGLP1-R, $F_c$-IgM sowie HC erhaltenen Klonen wurden jeweils 96 zufällig ausgewählt und im Einzelphagen-ELISA hinsichtlich ihrer Bindung an das entsprechende Antigen bzw. Hapten analysiert. Dazu wurden je 300 µl 2xYT/Chloramphenicol-Medium mit einer Einzelkolonie inokuliert und für 18 Std. bei 37 °C und 220 rpm geschüttelt. Mit jeweils 80 µl dieser stationären Kultur wurden 4 ml 2xYT/ Chloramphenicol-Medium angeimpft und für 4 Std. bei 37 °C und 180 rpm geschüttelt. Um paralleles Arbeiten zu ermöglichen wurden hierzu 4 "Deep-Well"-Platten (Qiagen; jeweils 24 Vertiefungen) verwendet. Nach Infektion der XL1Blue-Zellen mit jeweils ca. $10^{11}$ cfu M13KO7-Helferphagen wurde für 30 min bei 37 °C inkubiert. Nach weiteren 30 min bei 37 °C und 180 rpm erfolgte die Zugabe von 50 mg/l Kanamycin. Dann wurde für 30 min bei 37 °C und 220 rpm geschüttelt und die Genexpression auf pMUBI-1 durch Einstellen der Kultur auf 0,2 mg/l Anhydro-Tetrazyklin (Stammlösung: 1,0 mg/ml in DMF) induziert. Die Inkubatortemperatur wurde dann auf 22 °C erniedrigt und die Kultur für 16 Std. bei 180 rpm geschüttelt. Die Zellen wurden schließlich durch Zentrifugation (30 min, 5.000 g , 4 °C) sedimentiert und die Überstände in frische "Deep-Well"-Platten überführt. Die enthaltenen Phagemide wurden durch Zugabe von 1 Volumen 20 % (w/v) PEG 6.000, 2,5 M NaCl und Inkubation für 1 Std. auf Eis gefällt und durch Zentrifugation (30 min, 5.000 g , 4 °C) präzipitiert. Die Phagemide wurden dann in 1,0 ml sterilem eiskaltem PBS gelöst und 1 : 1 mit 6 % BSA in PBST 0,1 gemischt und für 1 Std. bei RT inkubiert.

Zur Durchfiihrung des ELISA wurde pro zu analysierendem monoklonalem Phagemid je eine Mikrotiterplatten-Vertiefung über Nacht bei 4 °C mit Antigen- und eine mit BSA-Lösung belegt. Zur Absättigung verbliebener Bindungsstellen an der Plastikoberfläche wurde dann jede Vertiefung für 2 Std. mit 3 % BSA (w/v) PBST 0,5 geblockt. Die Vertiefungen wurden danach dreimal mit PBST 0,1 gespült und ausgeklopft. Anschließend wurden je 100 µl der oben hergestellten Phagemid-Lösung in die entsprechenden Vertiefungen der Platte pipettiert. Nach einer Inkubationsdauer von zwei Stunden wurde dreimal mit PBST 0,1 gewaschen. Für den Nachweis von gebundenen Phagemiden wurde ein anti-M13 Antikörper-Peroxidase Konjugat (Amersham Pharmacia Biotech) im Verhältnis 1 : 5.000 in PBST 0,1 verdünnt und je 100 µl davon jeder Vertiefung zugesetzt. Nach einer Stunde Inkubation bei RT wurden die Vertiefungen dreimal mit PBST 0,1, dann dreimal mit PBS gespült. Schließlich wurden jeweils 100 µl des ImmunoPure-Kits (Pierce) hinzu pipettiert und die Farbreaktion dann nach 15 min durch Zugabe von 100 µl 2 M $H_2SO_4$ gestoppt. Die Messung der Extinktion erfolgte mit Hilfe eines Sunrise Remote Readers (Tecan) bei 450 nm.

Die DNA von Ubiquitin-Varianten aus Phagemiden, die im ELISA ein relativ starkes Bindungssignal an das jeweilige Antigen - nicht aber an BSA - zeigten (jeweils ca. 20), wurde mit Hilfe des Primer Seq-ID No. 14 und dem oben beschriebenen Verfahrens sequenziert. Ein Teil der analysierten DNA-Sequenzen wiesen Verschiebungen des Leserasters oder Amber-Stopcodons auf und wurden daher nicht weiter verwendet. Beispielhaft sind Aminosäure-Substitutionen von Ubiquitin-Varianten, die auf diese Weise erhalten und weiter analysiert wurden, sind in Tabelle 1 aufgelistet.

**Tabelle 1:** Aminosäure-Substitutionen im Bereich der *de novo* generierten Bindungsstelle von Ubiquitin-basierten modifizierten Proteinen nach Selektion gegen unterschiedliche Zielsubstanzen mittels *Phage Display.*

| | Selektion | Pos. 2 : | Pos. 4 : | Pos. 6 : | Pos. 62 : | Pos. 63: | Pos. 64 : | Pos. 65 : | Pos. 66 : |
|---|---|---|---|---|---|---|---|---|---|
| SPU[1] | - | Gln | Phe | Lys | Gln | Lys | Glu | Ser | Thr |
| SPU-1-D10 | RecGLP1-R | Ser | Phe | Pro | Tyr | Ser | Lys | Pro | Ser |
| SPU-2-A7 | $F_c$-IgM | Ser | Leu | Pro | Pro | Pro | Gly | Arg | Asn |
| SPU-3-H13 | HC | Gly | Gly | Lys | Phe | Phe | Val | Thr | Asn |
| SPU-15-G7 | TNFα | Tyr | Cys | Asn | Asn | Leu | Ser | Trp | Gln |
| SPU-15-E1 | TNFα | Gln | Ala | Ile | Met | Phe | Gln | Thr | Ser |

[1]SPU: Ubiquitin-Proteingerüst ohne Substitutionen in der Bindungstasche

Beispiel 5: Darstellung von Ubiquitin-Varianten in einem *in vitro* Transkriptions- / Translationssystem und Selektion von Ubiquitin-Varianten gegen Proteine mittels *Ribosomal Display*

Die Bereitstellung eines Expressionskonstruktes für die *in vitro* Transkription / Translation sowie die Selektion von Bindungsproteinen mittels Ribosomal Display erfolgte in Anlehnung an Schaffitzel *et al.* (2001). Im Gegensatz hierzu wurde im vorliegenden Fall jedoch keine Bibliothek von Antikörperfragmenten verwendet, sondern zu der in Beispiel 2 beschriebenen analogen Bibliotheken von Varianten des Ubiquitins. Eine dieser Bibliotheken wurde auf der Grundlage von der DNA-Sequenz (Seq-ID No. 1) für ein modifiziertes Ubiquitin-Proteingerüst mit den Substitutionen Ile44Ala, Lys48Arg, Arg54Leu, Val70Ala, Arg72Leu, Gly75Ala sowie mit der Deletion von Gly76 hergestellt. Eine zweite Bibliothek wurde auf der Grundlage der DNA-Sequenz (Seq-ID No. 15) für ein modifiziertes Ubiquitin-Proteingerüst mit der Substitution Phe45Trp hergestellt.

In einem ersten Schritt wurden die entsprechend Beispiel 2 an 8 Kodons mutagenisierten synthetischen Gene für die Ubiquitin-Varianten, welche die Bibliothek darstellen, auf der Grundlage von Seq-ID No. 1 bzw. Seq-ID No. 15 mittels PCR hergestellt. Dies wurde unter Verwendung der *Pfu*-Polymerase (Promega) in einem Volumen von 50 µl realisiert. Dazu wurden 5 µl des mitgelieferten 10x *Pfu*-Puffers sowie 4 µl dNTP-Mix verwendet und mit $H_2O$ aufgefüllt. Weiterhin enthielt der Ansatz jeweils 2,5 µl flankierende Primer (Seq-ID No. 16 und Seq-ID No. 17 für die Bibliothek auf der Grundlage von Seq-ID No. 1 sowie Seq-ID No. 18 und Seq-ID No. 19 für die Bibliothek auf der Grundlage von Seq-ID No. 15; 10 µM) zur Einführung der gewünschten Basenpaar-Austausche. Als Template wurde jeweils 1,0 ng Plasmid-DNA verwendet, welche die nicht-mutierten synthetischen Ubiquitin-Gene Seq-ID No. 1 oder Seq-ID No. 15 trug. Nach Zugabe von 2,5 U der *Pfu*-Polymerase wurde in 25 Zyklen für je 1 min bei 94 °C, 1 min bei 65 °C und für 1,5 min bei 72 °C inkubiert. Eine abschließende Inkubation erfolgte für 5 min bei 72 °C. Das gewünschte PCR-Produkt wurde mittels präparativer Agarose-Gelelektrophorese und dem QIAquick Gel Extraction Kit (Qiagen) isoliert.

Der sogenannte *Spacer* - ein Abstandhalter, welcher ein Teil des Hüllproteins III des Bakteriophagen M13 umfasst und im Zuge der *in vitro* Translation das nascierende Protein aus dem Ribosomen-Kanal herausschiebt, um so eine optimale Präsentation der Ubiquitin-Varianten zu gewährleisten, - wurde in zwei aufeinanderfolgenden PCR-Reaktionen synthetisiert. Dies geschah zunächst in einem Gesamtvolumen von 50 µl mit 5 µl des mitgelieferten 10x *Pfu*-Puffers, 4 µl dNTP-Mix und einer entsprechenden Menge $H_2O$. Weiterhin enthielt der Ansatz jeweils 2,5 µl flankierende Primer (Seq-ID No. 20 und Seq-ID No. 21; 10 µM) sowie 1,0 ng DNA des Bacteriophagen M13. Nach Zugabe von 2,5 U der *Pfu*-Polymerase wurde in 25 Zyklen für je 1 min bei 94 °C, 1 min bei 65 °C und für 1,5 min bei 72 °C inkubiert. Eine abschließende Inkubation erfolgte für 5 min bei 72 °C. Das gewünschte PCR-Produkt wurde mittels präparativer Agarose-Gelelektrophorese, dem QIAquick Gel Extraction Kit (Qiagen) isoliert und diente als Template für die zweite PCR-Reaktion. Diese wurde unter Verwendung der *Pfu*-Polymerase (Promega) in einem Volumen von 50 µl realisiert. Dazu wurden 5 µl des mitgelieferten 10x *Pfu*-Puffers sowie 4 µl dNTP-Mix verwendet und mit $H_2O$ aufgefüllt. Weiterhin enthielt der Ansatz jeweils 2,5 µl flankierende Primer (Seq-ID No. 22 und Seq-ID No. 21; 10 µM). Als Template wurde jeweils 1,0 ng DNA aus der vorherigen PCR-Reaktion verwendet. Nach Zugabe von 2,5 U der *Pfu*-Polymerase wurde in 25 Zyklen für je 1 min bei 94 °C, 1 min bei 55 °C und für 1,5 min bei 72 °C inkubiert. Eine abschließende Inkubation erfolgte für 5 min bei 72 °C. Das gewünschte PCR-Produkt wurde mittels präparativer Agarose-Gelelektrophorese und dem QIAquick Gel Extraction Kit (Qiagen) isoliert.

Im anschließenden Schritt wurden die an 8 Kodons mutagenisierten synthetischen Gene für die Ubiquitin-Varianten, welche die Bibliothek darstellen, mittels einer verknüpfenden PCR-Reaktion mit der bereitgestellten *Spacer*-DNA fusioniert. Dies wurde unter Verwendung der *Pfu*-Polymerase (Promega) in einem Volumen von 50 µl realisiert. Dazu wurden 5 µl des mitgelieferten 10x *Pfu*-Puffers sowie 4 µl dNTP-Mix verwendet und mit $H_2O$ aufgefüllt. Weiterhin enthielt der Ansatz jeweils 2,5 µl flankierende Primer (Seq-ID No. 23 und Seq-ID No. 21; 10 µM). Als Template wurden jeweils 500 ng Bibliotheks-DNA sowie 500 ng *Spacer*-DNA aus den vorherigen PCR-Reaktionen verwendet. Nach Zugabe von 2,5 U der *Pfu*-Polymerase wurde in 25 Zyklen für je 1 min bei 94 °C, 1 min bei 50 °C und für 1,5 min bei 72 °C inkubiert. Eine abschließende Inkubation erfolgte für 5 min bei 72 °C. Das gewünschte PCR-Produkt wurde mittels präparativer Agarose-Gelelektrophorese und dem QIAquick Gel Extraction Kit (Qiagen) isoliert.

Im abschließenden Schritt wurden mit Hilfe einer weiteren PCR-Reaktion entsprechende regulative Sequenzen (T7-Promoter, Ribosomen-Bindungsstelle) für die *in vitro* Transkriptions- / Translationreaktion eingeführt und das lineare Expressionskonstrukt fertiggestellt. Die Reaktion wurde in einem 500 µl-Ansatz durchgeführt, wobei ca. 500 ng des in der vorherigen PCR-Reaktion gewonnenen Produktes eingesetzt und die *Taq*-Polymerase verwendet wurde. Der Reaktionsansatz wurde - adaptiert auf das 10fache Volumen - wie in Beispiel 2 ausgeführt aus 10x *Taq*-Puffer, 25 mM $MgCl_2$, dNTP-Mix sowie den flankierenden Primern (Seq-ID No. 24, Seq-ID No. 21; 10 µM), pipettiert. Nach Auffüllen mit $H_2O$ wurden 2,5 U der *Taq*-Polymerase zugegeben und das PCR-Programm gestartet. In 25 Zyklen wurde für je 1 min bei 94 °C, 1 min bei 65 °C und für 1,5 min bei 72 °C inkubiert. Eine abschließende Inkubation erfolgte für 5 min bei 72 °C. Das gewünschte PCR-Produkt wurde mittels präparativer Agarose-Gelelektrophorese, dem QIAquick Gel Extraction Kit (Qiagen) isoliert und konnte direkt für die *in vitro* Transkriptions- / Translationreaktion verwendet werden.

Für die *in vitro* Transkriptions- / Translationreaktion wurde der RTS100 *E. coli* Kit (Roche; Kat. Nr. 3186 148) nach den Angaben des Herstellers verwendet. Dabei wurden 24.0 µl *E.* coli-Lysat, 20.0 µl Reaktions Mix, 24.0 µl Aminosäure Mix (ohne Met), 2.0 µl Methionin, 10.0 µl Reaktionspuffer (10 x), 2.0 µl MgAc (500 mM), 2.0 µl Anti_ssrA DNA (200 µM) sowie 20.0 µl der jeweiligen DNA-Bibliothek (0.5 - 1.0 µg) mit der Pipette vorsichtig gemischt und für 60 min bei 30 °C oder 37 °C inkubiert. Die folgenden Schritte wurden im Kühlraum bzw. auf Eis durchgeführt. Der Ansatz wurde für 5 min auf Eis abgekühlt und die Reaktion durch Zugabe von 400 μl WBT (50 mM Tris/HCl (pH 7.5), 150 mM NaCl, 50 mM MgAc, 0.1 % Tween 20) mit 3 % BSA gestoppt. Dieser Puffer kann ggf. 2.5 mg/ml Heparin (Sigma) enthalten. Die unlöslichen Anteile des Ansatzes wurden durch Zentrifugieren für 5 min bei 13 000 rpm und 4 °C präzipitiert, wobei die ternären Komplexe aus Ubiquitin-Variante, entsprechender mRNA und Ribosom im Überstand verblieben.

Als Affinitätsmatrix für die Isolierung von ternären Ribosom-Komplexen mit oberflächenpräsentierten Ubiquitin-Varianten, die vorher definierte Zielsubstanzen binden sollten, wurden 2 Vertiefungen einer Mikrotiter-Platte mit der entsprechenden Substanz beschichtet. Als Zielsubstanzen diente der rekombinant hergestellte Wachstumsfaktor VEGF, der über Nacht bei 4 °C an der Mikrotiter-Platte immobilisiert wurde. Unbelegte Bindungsplätze an der Oberfläche der Mikrotiter-Platte wurden durch Inkubation der Vertiefungen mit jeweils 400 μl 3 % BSA in WBT für 90 min bei RT geblockt. Nach entfernen der Block-Lösung wurden pro Vertiefung 250 μl der Lösung mit den ternären Ribosom-Komplexen aus der *in vitro* Transkriptions- / Translationreaktion zugegeben und für 60 min bei 4 °C und 50 rpm inkubiert. Ungebundene Komplexe wurden dann durch fünfmaliges Waschen mit z. B. WBT (300 μl pro Vertiefung, 3 min, 4 °C, 50 rpm) und kräftiges Ausklopfen entfernt. An die Affinitätsmatrix gebundene Komplexe wurden schließlich mit Hilfe von 100 μl EB (50 mM Tris/HCl (pH 7.5), 1.5 M NaCl, 20 mM EDTA) - dieser Puffer kann ggf. 50 μg/ml RNA aus Hefe (Sigma) enthalten - pro Vertiefung und Inkubation für 5 min bei 4 °C und 50 rpm in mRNA, Protein und die ribosomalen Untereinheiten zerlegt.

Die RNA Isolierung aus der erhaltenen Suspension erfolgte möglichst zügig bei Raumtemperatur und unter Verwendung des RNAeasy Kits von Qiagen (Kat. Nr. 74104) nach Angaben des Herstellers. Dabei erfolgte die Elution der RNA im letzten Schritt mit 30 μl RNase-freiem $H_2O$. Zum Abbau eventuell verschleppter DNA linearer Expressionskonstrukte wurde die erhaltenen RNA-Lösung mit DNase I (Invitrogen) behandelt. Dazu erfolgte die Zugabe von 3 μl DNase-Puffer (10x) und 3 μl DNase I (1 u/μl) und die Inkubation für 15 min bei Raumtemperatur. Die DNase wurde dann durch Zugabe von 3 μl 25 mM EDTA und die Inkubation für 10 min bei 65 °C inaktiviert. Die so erhaltenen RNA wurde anschließend mit Hilfe der Reverse Transkriptase Reaktion komplementiert. Hierzu wurden zunächst 8.5 μl der RNA mit 0.5 μl des Oligodesoxynukleotids T7te (Seq-ID No. 21; 100 μM) und 4.0 μl dNTP (2.5 mM *each*) gemischt, für 5 min bei 65 °C inkubiert und dann für 1 min auf Eis gestellt. Dann wurden als weitere Komponenten 4.0 μl RT-Puffer (5x), 1.0 μl DTT (0.1 M),1.0 μl RNAsin (Promega) sowie 1.0 μl SS-Reverse Transkriptase III (200 u/μl; Invitrogen) hinzugegeben und für 60 min bei 55 °C inkubiert.

Der Reaktionsansatz aus der Reverse Transkriptase Reaktion wurde mittels PCR direkt für die erneute Re-Amplifizierung der genetischen Information angereicherter Ubiquitin-Varianten sowie für die Wiedereinführung der regulativen Sequenzen für einen erneuten Selektionszyklus verwendet. Hierzu wurden zwei aufeinanderfolgende PCR-Reaktionen unter Verwendung des *Expand High Fidelity* PCR Systems (Roche) in einem Gesamtvolumen von 50 μl durchgeführt. Dazu wurden 5 μl des mitgelieferten 10x Puffers (inkl. 15 mM $MgCl_2$) sowie 4 μl dNTP-Mix verwendet und mit $H_2O$ aufgefüllt. Weiterhin enthielt der Ansatz jeweils 2,5 μl flankierende Primer (Seq-ID No. 23 und Seq-ID No. 21; 10 μM) sowie 2.5 μl Di-Methyl-Sulfoxyd (DMSO). Als Template wurden entweder 2.5 oder 5.0 μl der vorherigen RT-Reaktion verwendet. Nach Zugabe von 0.75 μl Polymerase-Mix (3.5 u/μl) wurde in 25 Zyklen für je 15 s bei 94 °C, 30 s bei 50 °C und für 1,0 min bei 72 °C inkubiert. Eine abschließende Inkubation erfolgte für 7 min bei 72 °C. Das gewünschte PCR-Produkt wurde mittels präparativer Agarose-Gelelektrophorese sowie dem QIAquick Gel Extraction Kit (Qiagen) isoliert und konnte direkt für die zweite PCR-Reaktion eingesetzt werden. Für diese wurden 5 μl des mitgelieferten 10x Puffers (inkl. 15 mM $MgCl_2$) sowie 4 μl dNTP-Mix verwendet und mit $H_2O$ auf 50 μl aufgefüllt. Weiterhin enthielt der Ansatz jeweils 2,5 μl flankierende Primer (Seq-ID No. 24 und Seq-ID No. 21; 10 μM). Als Template wurde die gesamte DNA aus der vorherigen PCR-Reaktion verwendet. Nach Zugabe von 0.75 μl Polymerase-Mix (3.5 u/μl) wurde in 25 Zyklen für je 15 s bei 94 °C, 30 s bei 50 °C und für 1,0 min bei 72 °C inkubiert. Eine abschließende Inkubation erfolgte für 7 min bei 72 °C. Das gewünschte PCR-Produkt wurde mittels präparativer Agarose-Gelelektrophorese und dem QIAquick Gel Extraction Kit (Qiagen) isoliert und konnte direkt für die *in vitro* Transkription / Translation eines erneuten Selektionszyklus eingesetzt werden.

Alternativ erfolgte die Klonierung des entsprechenden Gens in den Expressionsvektor pET20B(-) (s. o.). Dies ermöglicht die Analyse der erhaltenen Ubiquitin-Varianten hinsichtlich ihrer DNA-Sequenz, deren rekombinante Herstellung in *E. coli* sowie deren Einschritt-Reinigung durch Immobilisierte Metalchelat Affinitäts-Chromatographie (IMAC) mit Hilfe eines carboxyteminal fusioniertem Hexahistidin-Peptids (s. u.). Beispielhaft sind Aminosäure-Substitutionen von Ubiquitin-Varianten, die auf diese Weise erhalten und weiter analysiert wurden, sind in Tabelle 2 aufgelistet.

**Tabelle 2:** Aminosäure-Substitutionen im Bereich der *de novo* generierten Bindungsstelle von Ubiquitin-basierten modifizierten Proteinen nach Selektion gegen unterschiedliche Zielsubstanzen mittels *Ribosomal Display.*

| | Selektion | Pos. 2 : | Pos. 4 : | Pos. 6 : | Pos. 62 : | Pos. 63 : | Pos. 64 : | Pos. 65 : | Pos. 66 : |
|---|---|---|---|---|---|---|---|---|---|
| SPU[1] | - | Gln | Phe | Lys | Gln | Lys | Glu | Ser | Thr |

(fortgesetzt)

| | Selektion | Pos. 2 : | Pos. 4 : | Pos. 6 : | Pos. 62 : | Pos. 63 : | Pos. 64 : | Pos. 65 : | Pos. 66 : |
|---|---|---|---|---|---|---|---|---|---|
| SPU-11-RD3/1 | VEGF | Arg | Arg | Ala | Arg | His | Gly | Thr | Ser |
| SPU-11-A8 | VEGF | Leu | Leu | Trp | Ser | Leu | Ser | Gly | Ile |
| SPU-11-58 | VEGF | Leu | Leu | Trp | Arg | Ser | Asp | Leu | Asn |
| SPW-11-A1 | VEGF | Phe | Trp | Val | Gly | His | Gln | Arg | Gly |

[1]SPU: Ubiquitin-Proteingerüst ohne Substitutionen in der Bindungstasche

Beispiel 6: Herstellung und Reinigung der Ubiquitin-basierten modifizierten Proteine

Die Charakterisierung der proteinchemischen Eigenschaften von selektierten Ubiquitin-Varianten aus Phagemiden mit relativ starkem Bindungssignal im ELISA und mit funktioneller DNA-Sequenz erfolgte nach der Klonierung des entsprechenden Gens in den Expressionsvektor pET20B(-) (s. o.). Dies ermöglichte die rekombinante Herstellung der jeweiligen Variante mittels *E. coli* BL21/pUBS sowie deren Einschritt-Reinigung durch Immobilisierte Metalchelat Affmitäts-Chromatographie (IMAC) mit Hilfe eines carboxyteminal fusioniertem Hexahistidin-Peptids.

Für die rekombinante Herstellung der Ubiquitin-basierten modifizierten Proteine mit neuen Bindungseigenschaften wurden 50 ml 2xYT/Amp/Kan-Medium mit einer entsprechenden Einzelkolonie inokuliert und für 16 Std. bei 37 °C und 220 rpm geschüttelt. Mit dieser Vorkultur wurden 1,5 12xYT/Amp/Kan-Medium im Verhältnis 1 : 50 angeimpft und bis zum Erreichen einer Zelldichte von $OD_{600}$ = 0,5 bei 37 °C und 220 rpm inkubiert. Nach Induktion der Fremdgen-Expression durch Zugabe von 1 mM/l α-D-Isopropyl Thiogalaktosid (IPTG) wurde für weitere 3 Std. bei 37 °C und 220 rpm geschüttelt. Die Zellen wurden dann durch Zentrifugation (30 min, 5.000 g, 4 °C) sedimentiert und in 40 ml NPI-20 (50 mM $NaH_2PO_4$ pH 8,0, 300 mM NaCl, 20 mM Imidazol) resuspendiert. Der Aufschluß der Zellen erfolgte durch halbstündige Inkubation mit 200 µg/ml Lysozym und 500 U Benzonase bei RT sowie fünfmaligem Pulsen mit Ultraschall für jeweils 15 s. Die Zelltrümmer wurden durch Zentrifugation (30 min, 15.000 g, 4 °C) sedimentiert und der Überstand, der das lösliche Gesamtzellprotein enthielt, konnte direkt für eine darauffolgende IMAC eingesetzt werden.

Die Chromatographie erfolgte an einer ÄKTA™-Explorer FPLC-Anlage (Amersham Pharmacia Biotech) unter Verwendung einer 5 ml HiTrap Chelating HP Säule (Amersham Pharmacia Biotech) bei RT und einer Flußgeschwindigkeit von 5 ml/min. Die Säule wurde zunächst mit 5 Säulenvolumen NPI-20 äquilibriert, woraufhin das Gesamtzellprotein über die Säule geleitet wurde. Nicht-gebundenes Protein wurde durch Spülen mit 30 Säulenvolumen NPI-20 ausgewaschen. Die Elution erfolgte mit einem linearen Gradienten von 20 mM bis 250 mM Imidazol in insgesamt 20 Säulenvolumen wobei das Eluat in Fraktionen ä 2,5 ml aufgefangen wurde. Die Fraktionen, welche die gereinigte Ubiquitin-Variante enthielten, wurden mittels SDS-Polyacrylamid-Gelelektrophorese analysiert und vereinigt. Die erzielten Ausbeuten der gereinigten rekombinanten Ubiquitin-Varianten lagen dabei zwischen 10 und 30 mg/l Kulturvolumen.

Beispiel 7: Charakterisierung der Bindungseigenschaften von Ubiquitin-basierten modifizierten Proteinen mit neuen Bindungseigenschaften

Die Bindung der jeweils selektierten Ubiquitin-Varianten, die wie in Beispiel 6 beschrieben gereinigt worden waren, an recGLP1-R, $F_c$-IgM sowie HC wurde im ELISA entweder mit einem Ni/NTA-Peroxidase Konjugat (Qiagen) oder mit einem anti-Ubiquitin Antiserum (Sigma) aus Kaninchen nachgewiesen. Dazu wurden Mikrotiterplatten-Vertiefung über Nacht bei 4 °C mit dem entsprechenden Antigen und z. B. BSA für den Nachweis unspezifischer Bindung belegt und zur Absättigung verbliebener Bindungsstellen 2 Std. mit 3 % BSA (w/v) PBST 0,5 geblockt. Die Platte wurden dann dreimal mit PBST 0,1 gespült und ausgeklopft. Anschließend wurde je 100 µl der Lösung des jeweiligen gereinigten modifizierten Proteins in PBST 0,1 in Konzentrationsabstufungen (von unverdünnt bis zur Verdünnung 1 : 16) in die Vertiefungen der Platte pipettiert. Nach einer Inkubationsdauer von zwei Stunden wurde dreimal mit PBST 0,1 gespült. Für den Nachweis des gebundenen modifizierten Proteins wurde das Ni/NTA-Peroxidase Konjugat im Verhältnis 1 : 500 bzw. das anti-Ubiquitin Antiserum im Verhältnis 1 : 10 in PBST 0,1 verdünnt und je 100 µl davon jeder Vertiefung zugesetzt. Nach einer Stunde Inkubation bei RT erfolgte entweder direkt die Detektion des Ni/NTA-Peroxidase Konjugats (s. u.) bzw. die Inkubation mit einem anti-Kaninchen Antikörper konjugiert mit Peroxidase (1 : 2.500 in PBST 0,1) für eine Stunde bei RT. Zur Detektion wurden die Vertiefungen dreimal mit PBST 0,1, dann dreimal mit PBS gespült.

Schließlich wurden jeweils 100 μl ImmunoPure-Kits (Pierce) hinzu pipettiert und die Farbreaktion dann nach 15 min durch Zugabe von 100 μl 2 M $H_2SO_4$ gestoppt. Die Messung der Extinktion erfolgte mit Hilfe eines Sunrise Remote Readers (Tecan) bei 450 nm. Die aus erhaltenen Werte der Absorptionsintensität wurden mit Hilfe des Computerprogramms "Sigma Plot" ausgewertet. Dazu wurde die jeweils gemessene Extinktion gegen die entsprechende eingesetzte Proteinkonzentration aufgetragen und die erhaltene Kurve mit Hilfe der Formel (1) durch nicht-lineare Regression angepaßt.

$$y = \frac{a * x}{(b + x)}$$

Unter der Annahme eines Assoziations/Dissoziations-Gleichgewichtes zwischen dem immobilisierten Antigen und dem modifizierten Protein ist dabei:

x = Konzentration des eingesetzten modifizierten Proteins
y = Konzentration des Antigen/ modifiziertes Protein -Komplexes (hier indirekt über die enzymatische Aktivität des Reporterenzyms gemessen)
a = Gesamtkonzentration des immobilisierten Antigens
b = Dissoziationskonstanten ($K_D$)

Beispielhaft sind in Abb. 5 die aus einem solchen ELISA-Experiment erhaltenen Bindungskurven der Ubiquitin-Variante SPU-1-D10, welche aus der Affinitätsanreicherung an recGLP1-R mittels *Phage Display* gemäß Beispiel 3 und 4 erhalten worden war, dargestellt. Weiterhin ist in Abb. 6 die Bindungskurven der Ubiquitin-Variante SPW-11-A1, welche aus der Affinitätsanreicherung an VEGF mittels *Ribosomal Display* gemäß Beispiel 5 erhalten worden war, dargestellt. Die für die einzelnen selektierten Ubiquitin-basierten modifizierten Proteine mit neuen Bindungseigenschaften erhaltenen Bindungsdaten sind in Tabelle 3 zusammengefasst.

Für die quantitative Analyse der Bindung von Varianten des Ubiquitins an ein vorher definiertes Antigen wurden ebenfalls ein BIACORE 3000 System (Biacore) verwendet. Für solche Oberflächen-Plasmonresonanz (*Surface Plasmon Resonance,* SPR) Messungen wurde ein zur Analytimmobilisierung befähigter Träger (*Sensor-Chip*) in dem Gerät positioniert und mit dem darin integrierten Mikrofluß-System (*Integrated μ-Fluidic Cartridge,* IFC) gekoppelt. Dies erlaubte einen kontinuierlichen Flüßigkeitsstrom aus dem Pufferreservoir über die *Chip*-Oberfläche und eine Detektion der Bindung am Analytträger quasi in Lösung.

Für eine Messung wurde zunächst das Targetmolekül, z. B. VEGF, mittels NHS/EDC-Kopplung über primäre Amingruppen an die Carboxy-Methyldextran-Oberfläche des Sensor-*Chips* CM5 (Biacore) nach Angaben des Herstellers gekoppelt. Die Messungen wurden bei 25 °C und einem kontinuierlichen Pufferfluß von 35 μl/min durchgeführt. Als Laufpuffer und Lösungsmittel der verwendeten Proteine wurde PBS (sterilfiltriert und entgast) unter Zusatz von 0.005 % Detergenz P-20 (Biacore) benutzt. Die Wechselwirkung unterschiedlicher Konzentrationen der Ubiquitin Varianten, die über die Probenschleife injiziert wurden, mit dem immobilisierten VEGF konnte aufgrund des generierten SPR-Signals detektiert werden. Das Signal wurde als sogenannte Resonanzeinheiten (*Resonance Units,* RU) in Abhängigkeit der Zeit quantifiziert und entsprechende Bindungskurven generiert. Die erhaltenen Bindungskurven wurden mit Hilfe der BIA*evaluation Software* (Version 3.1; Biacore) ausgewertet und der Wert für die Dissoziationskonstante ($K_D$) ermittelt. Beispielhaft sind in Abb. 7 die aus einem solchen BIACORE-Experiment erhaltenen Bindungskurven der Ubiquitin-Variante SPU-11-58, welche aus der Affinitätsanreicherung an VEGF mittels *Ribosomal Display* gemäß Beispiel 5 erhalten worden war, dargestellt. Die für die einzelnen selektierten Ubiquitin-basierten modifizierten Proteine mit neuen Bindungseigenschaften erhaltenen Bindungsdaten sind in Tabelle 3 zusammengefasst.

**Tabelle 3:** Dissoziationskonstanten von Komplexen aus selektierten Ubiquitin-basierten modifizierten Proteinen und unterschiedlichen Zielsubstanzen.

| Modifiziertes Protein | Zielsubstanz | Apparenter $K_D$-Wert |
|---|---|---|
| SPU-1-D10[1] | recGLP1-R | 166 ± 0,06 nM |
| SPU-2-A7[1] | $F_c$-IgM | 9,4 ± 0,9 μM |
| SPU-3-H13[1] | HC | 10,7 ± 1,0 nM |
| SPU-11-RD3/1[2] | VEGF | 25 μM |
| SPU-11-A8[2] | VEGF | 70 nM |

(fortgesetzt)

| Modifiziertes Protein | Zielsubstanz | Apparenter $K_D$-Wert |
|---|---|---|
| SPU-11-58[2] | VEGF | 50 nM |
| SPW-11-A1[1] | VEGF | 1,2 ± 0,1 μM |
| SPU-15-G7[1] | TNFα | < 1,0 μM |
| SPU-15-E1[1] | TNFα | < 1,0 μM |

[1]Analyse mittels ELISA; [2]Analyse mittels BIACORE

Weiterhin wurden einzelne modifizierte Proteine hinsichtlich der Spezifität ihrer Bindung überprüft. Dazu wurden ELISA-Experimente mit einer einzelnen geeigneten Konzentration der modifizierten Proteine wie oben beschrieben durchgeführt. Dabei wurden entsprechende Vertiefungen der Mikrotiterplatte mit dem Zielsubstrat sowie mit strukturell ähnlichen Substanzen belegt. Beispielhaft sind in Abb. 8 die aus einem solchen ELISA-Experiment erhaltenen Bindungskurven der Ubiquitin-Variante SPU-3-H13, welche aus der Affinitätsanreicherung an HC gemäß Beispiel 3 und 4 erhalten worden war, dargestellt.

Beispiel 8: Verbesserung der Bindungseigenschaften von Ubiquitin-basierten Bindungsproteinen durch ortsgerichtete Sekundärmutagenese

Auf Grundlage der Ubiquitin-Variante SPU-2-A7, die eine *de novo* Bindungseigenschaft gegenüber $F_c$-IgM zeigt, wurde eine generell anwendbare Strategie zur Affinitätsmaturierung etabliert. Diese umfaßte zunächst die erneute zufällige Substitution ausgewählter Positionen in der *de novo* generierten Bindungsstelle auf DNA-Ebene sowie die anschließende parallele Expression, Reinigung und Bindungsanalyse von jeweils 96 der entsprechenden Varianten. Die Verwendung dieses "96er Formates" erlaubt den Tranfer dieser Strategie auf Laborroboter und damit die Analyse von Varianten im Hochdurchsatz.

Hierfür wurden in zwei parallelen Ansätzen die Kodons von jeweils zwei Positionen (62 und 63 sowie 64 und 65) mittels PCR zufällig mutiert. Die entsprechenden Reaktionen wurden in einem Volumen von jeweils 50 μl durchgeführt, wobei ca. 1.0 ng des Gens für SPU-2-A7 inseriert in pET20B(-) eingesetzt und die *Taq*-Polymerase verwendet wurde. Der Reaktionsansatz wurde wie in Beispiel 2 ausgeführt aus 10x *Taq*-Puffer, 25 mM $MgCl_2$, dNTP-Mix sowie den flankierenden Primern (Seq-ID No. 12, Seq-ID No. 25 für die Mutation der Positionen 62 und 63 bzw. Seq-ID No. 12, Seq-ID No. 26 für die Mutation der Positionen 64 und 65; 10 μM), pipettiert. Nach Auffüllen mit $H_2O$ wurden 2,5 U der *Taq*-Polymerase zugegeben und das PCR-Programm gestartet. In 25 Zyklen wurde für je 1 min bei 94 °C, 1 min bei 55 °C und für 1,5 min bei 72 °C inkubiert. Eine abschließende Inkubation erfolgte für 5 min bei 72 °C. Die gewünschten PCR-Produkte wurde mittels präparativer Agarose-Gelelektrophorese und dem QIAquick Gel Extraction Kit (Qiagen) isoliert. Die erhaltenen DNA-Fragmente, die jeweils die Bibliotheken von an Positionen 62/63 bzw. 64/65 mutagenisiertem SPU-2-A7 präsentierten, wurden mittels präparativem *Nde*I/*Xho*I-Restriktionsverdau geschnitten und wiederum durch präparative Agarose-Gelelektorphorese isoliert. Die Insertion der Gen-Bibliotheken für das modifizierte SPU-2-A7 erfolgte in den Expressionsvektor pET20B(-) (Novagen; vgl. Beispiel 1) für die Produktion des entsprechenden Proteins.

Nach Transformation elektrokompetenter *E. coli,* z. B. Nova-Blue oder BL21-Zellen wurden Einzelkolonien erhalten, welche in Form des Expressionsplasmids die genetische Information jeweils eines Klons der erhaltenen Bibliothek enthielten. Mit 96 dieser Einzelkolonien wurden jeweils 300 μl 2xYT/Amp/Kan inokuliert und über Nacht bei 37 °C und 200 rpm geschüttelt. Mit jeweils 100 μl dieser Kulturen wurden dann 96 x 4 ml 2xYT/Amp/Kan angeimpft und bis zum Erreichen einer Zelldichte von $OD_{600}$ = 0,5 bei 37 °C und 220 rpm inkubiert. Hierfür wurden jeweils vier 24er Kultur-Platten (Qiagen) verwendet. Nach Induktion der Fremdgen-Expression durch Zugabe von 1 mM/L α-D-Isopropyl Thiogalaktosid (IPTG) wurde für weitere 3 Std. bei 37 °C und 180 rpm oder über Nacht bei 30 °C und 180 rpm geschüttelt. Die Zellen wurden dann durch Zentrifugation (30 min, 4.000 g, 4 °C) sedimentiert und der Überstand abgenommen. Der Zellaufschluß wurde durch physikalische (Schockgefrieren und Auftauen) oder chemische (Detergenzien) Lyse sowie die Zugabe von Lysozym (200 μg/ml) und Benzonase 10 u/ml im Endvolumen) bewerkstelligt. Die Reinigung der Varianten von SPU-2-A7 aus dem erhaltenen Gesamtzellprotein erfolgte mit Hilfe des BioRobot 9600 Kits von Qiagen und einer manuell betriebenen Vakuum-Station (QIAvac 96, Qiagen). Die erhaltenen Proteinlösungen wurden in einem ELISA-Experiment, welches im Aufbau dem aus Beispiel 7 entsprach, qualitativ hinsichtlich ihrer Bindung an $F_c$-IgM analysiert.

Varianten von SPU-2-A7 mit relativ starkem Bindungssignal im ELISA wurden zur Überprüfung der Funktionalität ihres Gens bzw. der Analyse der erfolgten Substitutionen einer DNA-Sequenzierung unterworfen. Vielversprechende Kandidaten wurden im 1.5 L-Maßstab rekombinant hergestellt und mittels Immobilisierte Metalchelat Affinitäts-Chromatographie (IMAC) mit Hilfe des carboxyteminal fusionierten Hexahistidin-Peptids gereinigt. Zur Quantifizierung der Affinität wurde ein wie in Beispiel 7 beschriebener konzentrationsabhängiger ELISA im Vergleich mit SPU-2-A7 - der Ubiquitin-Variante, von welcher ausgehend die Maturierung realisiert wurde, - durchgeführt. Beispielhaft sind in Abb. 9 die aus einem solchen ELISA-Experiment erhaltenen Bindungskurven der Ubiquitin-Variante SPU-2-A7 sowie SPU-2-A7(62/63), dargestellt. Diese affinitätsmaturierte Variante zeigt mit $K_D$ = 1.0 μM im Vergleich zu SPU-2-A7 eine um den Faktor 10 verbesserte Bindungsstärke.

Beispiel 9: Ortsgerichteten Kopplung zweier Ubiquitin-basierter Proteine über singuläre, carboxyterminale Cysteinreste mittels Bis-Maleimid-Reagenzien

Die Verknüpfung zweier identischer Ubiquitin-Varianten erfolgte kovalent über speziell hierfür eingeführte Cysteinreste mit Hilfe von Bis-Maleimido-Hexan (BMH, Pierce). Hierfür wurde zunächst ein entsprechendes Kodon für Cystein gefolgt von einem für Prolin am 3'-Ende des Leserahmens - d. h. im Protein carbobyterminal hinter dem Hexahistidin-Peptid - eingeführt. Der abschließende Prolinrest sollte dabei das resultierende Protein vor proteolytischem Abbau während Produktion und Reinigung schützen. Die Insertion der entsprechenden Basenpaare erfolgte mit Hilfe des Quick Change™ Site-Directed Mutagenesis Kits (Stratagene) nach Angaben des Herstellers und unter Verwendung der Oligodesoxynukleotide Seq-ID No. 27 und Seq-ID No. 28 sowie dem Gen einer Ubiquitin-Variante (Seq-ID No. 15) in pET20B(-) als Template. Erhaltene Klone wurden durch DNA-Sequenzierung analysiert und Kolonien, welche das Expressionsplasmid mit der erwünschten Insertion besaßen, für die rekombinante Produktion und Reinigung gemäß Beispiel 6 verwendet.

Das gereinigte Protein wurde gegen PBS mit 1 mM EDTA und 5 mM β-Mercaptoethanol dialysiert und mit einer Konzentration von ca. 1 mg/ml für die nachfolgende Kopplungsreaktion eingesetzt in der PBS, 1 mM EDTA als Reaktionspuffer diente. Dazu wurde der freie Cysteinrest zunächst durch Einstellen der Proteinlösung auf 100mM Di-Thio-Threitol (DTT) und Inkubation für eine Stunde bei Raumtemperatur reduziert. Danach wurden reduzierenden Reagenzien vom Protein mit Hilfe einer PD10 Sephadex G-25 M Säule (Amersham Biosciences) abgetrennt, dieses mit einem zweifachen molaren Überschuss an Kopplungsreagenz (Stammlösung: 100 mM BMH in DMSO) versetzt und für eine Stunde bei Raumtemperatur inkubiert. Überschüssiges BMH wurde erneut mit Hilfe eine PD10 Säule abgetrennt. Um eventuell vorhandenes nur einfach reagiertes Kopplungsreagenz abzusättigen, wurde zum so erhaltenen Kopplungsansatz nochmals eine äquimolare Menge an frisch reduziertem Protein zugegeben. Dieser zweite Kopplungsschritt fand für 2 Std. bei Raumtemperatur statt.

Der mit dieser Methode erhaltene Kopplungsansatz wurde mittels SDS-PAGE analysiert (Abb. 10) und der Kopplungsgrad abgeschätzt. Dieser betrug für die beschriebene Vorgehensweise ca. 40 %.

Literatur:

Ausuebel, F.M., Brent, R., Kinston, R.E., Moore, D.D., Seidmann, J.G., Smith, J.A. and Struhl, K. (1994): Current protocols in molecular biology. John Wiley & Sons, Inc.

Bazarsuren, A., Grauschopf, U., Wozny, M., Reusch, D., Hoffmann, E., Schaefer, W., Panzner, S. und Rudolph, R. (2002) In vitro folding, functional characterization, and disulfide pattern of the extracellular domain of human GLP-1 receptor. Biophys. Chem. 96, 305-318.

Beal, R., Deveraux, Q., Xia, G., Rechsteiner, M. und Pickart, C. (1996) Surface hydrophobic residues of multiubiquitin chains essential for proteolytic targeting. Proc. Natl. Acad. Sci. USA 93, 861-866.

Berman, H. M., Westbrook, J., Feng, Z., Gilliland, G., Bhat, T. N., Weissig, H., Shindyalov, I. N. und Bourne, P. E. (2000) The Protein Data Bank. Nucleic Acid Res., 28, 235-242.

Beste, G. Schmidt, F. S., Stibora, T. und Skerra, A. (1999) Small antibody-like proteins with predescribed ligand specificities derived from the lipocalin fold. Proc. Natl. Acad. Sci. USA 96, 1898-1903.

Bird, R. E., Hardman, K. D., Jacobson, J. W., Johnson, S., Kaufman, R., Lee, S. M., Pope, H. S., Riordan, G. S. und Whitlow, M. (1988) Single-chain antigen-binding proteins. Science 242, 423-426.

Burch, T. J. und Haas, A. L. (1994) Site-directed mutagenesis ofUbiquitin. Differential roles for Arginine in the interaction with Ubiquitin-activating enzyme. Biochemistry 33, 7300-7308.

Brinkmann, U., Reiter, Y., Jung, S. H., Lee, B. und Pastan, I. (1993) A recombinant immunotoxin containing a disulfide-stabilized Fv-Fragment. Proc. Natl. Acad. Sci. USA 90, 7538-7542.

Buchberger A, Howard MJ, Proctor M, Bycroft M, National Library of Medicine, J Mol Bil. 2001 Mr 16; 307(1); 17-24.

Carter, P., Kelley, R. F., Rodrigues, M. L., Snedecor, B., Covarrubias, M., Velligan, M. D., Wong, W. L. T., Rowland, Kotts, C. E., Carver, M. E., Yang, M., Bourell, J. H., Shepard, H. Connolly, M.L. (1983) "Solvent-Accessible Surfaces of Proteins and Nucleic Acids" Science, 221, 709 -713.

M. und Henner, D. (1992) High level Escherichia coli expression and production of a bivalent humanized antibody fragment. Biotechnology 10, 163-167.

Daugherty, P. S., Chen, G., Olsen, M. J., Iverson, B. L. und Georgiou, G. (1998) Antibody affinity maturation using bacterial surface display. Protein Eng. 11, 825-832.

Dübel, S. und Kontermann, R. E. (2001) Recombinant Antibodies. In: Kontermann, R. und Dübel, S. (Hrsg.) "Antibody Engineering." Springer Verlag, Heidelberg.

Filippi, M., Tribioli, C. und Toniolo, D. (1990) Linkage and sequence conservation of the X-linked genes DX253 (P3) and DXS254E (GdX) in mouse and man. Genomics 7, 453-457.

Griep, R. A., van Twisk, C., van der Wolf, J. M. und Schots, A. (1999) Fluobodies: green fluorescent single-chain Fv fusion proteins. J. Immunol. Methods 230, 121-130.

Hanes, J., Jermutus, L., Weber-Bornhauser, S., Bosshard, H. R. und Plückthun, A. (1998) Ribosome display efficiently selects and evolves high-affinity antibodies in vitro from immune libraries. Proc. Natl. Acad. Sci. USA 95, 14130-14135.

Hanes, J., Schaffitzel, C., Knappik, A. und Plückthun, A. (2000) Picomolar affinity antibodies from a fully synthetic naive library selected and evolved by ribosome display. Nature Biotechnology 18, 1287-1292.

He, M. und Taussig, M. J. (1997) Antibody-ribosome-mRNA (ARM) complexes as efficient selection particles for in vitro display and evolution of antibody combining sites. Nucleic Acids Res. 25, 5132-5134.

Holliger, P., Prospero, T. und Winter, G. (1993) "Diabodies": small bivalent and bispecific antibodies. Proc. Natl. Sci. USA 90, 6444-6448.

Hoogenboom, H. R., de Bruine, A. P., Hufton, S. E., Hoet, R. M., Arends, J. W. und Roovers, R. C. (1998) Antibody phage display technology and its applications. Immunotechnology 4, 1-20.

Jones, D. und Candido, E. P. (1993) Novel ubiquitin-like ribosomal protein fusion genes from the nematodes Caenorhabditis elegans and Caenorhabditis briggsae. J. Biol. Chem. 268, 19545-195451.

Kieke, M. C., Cho, B. K., Boder, E. T., Kranz, D. M. und Wittrup, K. D. (1997) Isolation of anti-T cell receptor scFv mutants by yeast surface display. Protein Eng. 10, 1303-1310.

Knappik, A., Ge, S., Honegger, A., Pack, P., Fischer, M., Wellnhofer, G., Hoess, A., Wölle, J., Plückthun, A. und Virnekäs, B. (2000) Fully synthetic human combinatorial antibody libraries (HuCAL) based on modular consensus frameworks and CDRs randomized with trinucleotides. J. Mol. Biol., 296, 57-86.

Koide, A., Bailey, C. W., Huang, X. und Koide, S. (1998) The fibronectin type III domain as ascaffold for novel binding proteins. J. Mol. Biol. 284, 1141-1151.

Kuchner, O. and Arnold, F.H. (1997): Directed evolution of enzyme catalysts. TIBTECH 15, 523-530.

Kumar, S., Yoshida, Y. und Noda, M. (1993) Cloning of a cDNA which encodes a novel ubiquitin-like protein. Biochem. Biophys. Res. Commun. 195, 393-399.

Larsen CN, Wang H., National Library of Medicine ; J Proteome Res. 2002 Sep-Oct; 1(5): 411-9.

Marx, J. (2002) Ubiquitin lives up to its name. Science 297, 1792-1794.

McConell S. and Hoess R.H. (1995): Tendamistat as a scaffold for conformationally constrained phage peptide libraries. J. Mol. Biol. 250, 460-470.

Michiels, L., Van der Rauwelaert, E., Van Hasselt, F., Kas, K. und Merregaert, J. (1993) Fau cDNA encodes a ubiquitin-like-S30 fusion protein and is expressed as an antisense sequence in the Finkel-Biskis-Reilly murine sarcoma virus. Oncogene 8, 2537-2546.

Miura, T., Klaus, W., Gsell, B., Miyamoto, C. und Senn, H. (1999) Characterization of the binding interface between Ubiquitin and class I human Ubiquitin-conjugating enzyme 2b by multidimensional heteronuclear NMR spectroscopy in solution. J. Mol. Biol. 290, 213-228.

Muller, B.H., Chevrier, D., Boulain, J.-C und Guesdon, J.-L. (1999) Recombinant single-chain Fv antibody fragment-alkaline phosphatase conjugate for one-step immunodetection in molecular hybridization. J. Immunol. Methods 227, 177-185.

Muller, S., Hoege, C., Pyrowolakis, G. und Jentsch, S. (2001) SUMO, ubiquitins mysterious cousin. Nat. Rev. Mol. Cell Biol. 2, 202-210.

Murzin A. G., Brenner S. E., Hubbard T. und Chothia C. (1995). SCOP: a structural classification of proteins database for the investigation of sequences and structures. J. Mol. Biol. 247, 536-540.

Nord K., Gunneriusson, E., Ringdahl, J., Stahl, S., Uhlen, M. and Nygren, P.A. (1997); Binding proteins selected from combinatorial libraries of an beta-helical bacterial receptor domain. Nat. Biotechnol. 8, 772-777.

Odegrip, R., Coomber, D., Eldridge, B., Herderer, R., Kuhlman, P. A., Ullman, C., Fitz-Gerald, K. und McGregor, D. (2003) CIS display: In vitro selection ofpeptides from libraries ofprotein- DNA complexes. PNAS 101, 2806-2810.

Pannekoek, H., van Meijer M., Schleef, R.R., Loskutoff, d.J. and Barbas, C.F. (1993): Functional display of human plasminogen-activator inhibitor 1 (PAI-1) on phages: Novel perspectives for structure-function analysis by error-prone DNA synthesis. Gene 128, 135-140.

Reiter, Y. und Pastan, I. (1998) Recombinant Fv immunotoxins and Fv fragments as novel agents for cancer therapy and diagnosis. Trends Biotechnol. 16, 513-520.

Sambrook, J., Maniatis, T. and Fritsch, E.F. (1989): Molecular Cloning: A laboratory manual. Cold spring Harbor. Cold Spring Harbour Laboratory Press, New York.

Sambrook, J., Fritsch, E. F. und Maniatis, T. (2001) "Molecular Cloning: A Laboratory Manual" 3rd ed. Cold Spring Harbor Laboratory Press, Cold Spring Harbor, NY.

Shrake, A. and Rupley, J.A. (1973) Environment and Exposure to Solvent of Protein Atoms. Lysozyme and Insuline. J. Mol. Biol. 79, 351-371.

Skerra, A. und Plückthun, A. (1988) Assembly of a functional immunoglobulin Fv fragment in Escherichia coli. Science 240, 1038-1041.

Schaffitzel, C., Zahnd, C., Amstutz, P., Luginbühl, B. und Plückthun, A.(2001) In vitro selection and evolution of protein-ligand interactions by ribosome display. In: Protein-Protein Interactions, A Molecular Cloning Manual, E. Golemis, Ed. (Cold Spring Harbor Laboratory Press, New York, 2001, pp. 535-567.

Skerra, A. (2000) Engineered protein scaffolds for molecular recognition. J. Mol. Recognit. 13, 167-187.

Stemmer, W.P.C. (1994): Rapid evolution of a protein in vitro by DNA shuffling. Nature 370, 389-391.

Vijay-Kumar, S., Bugg, C. E. und Cook, W. J. (1987) Structure ofUbiquitin refined at 1.8 A resolution. J. Mol. Biol. 194, 531-544.

Winter, G. (1998) Synthetic human antibodies and a strategy for protein engineering. FEBS Lett. 430, 92-94.

Wintrode, P. L., Makhatadze, G. I. und Privalov, P. L. (1994) Thermodynamics ofUbiquitin unfolding. Proteins Struct. Funct. Genet. 18, 246-253.

Die Anmeldung beinhaltet die folgenden bevorzugten Ausgestaltungen der Erfindung:

1. Protein, ausgewählt aus der Gruppe, bestehend aus Proteinen der Protein-Superfamilie "ubiquitin-like proteins", Proteinen, die ein Ubiquitin-artiges Faltungsmotiv aufweisen sowie Fragmenten oder Fusionsproteinen hiervon, die je das Ubiquitin-artige Faltungsmotiv besitzen, wobei das Protein unter Beibehaltung des Ubiquitin-artigen Faltungsmotivs aufgrund einer oder mehrerer Modifikationen von Aminosäuren in zumindest einem oberflächenexponierten Bereich des Proteins, der mindestens einen beta-Faltblattstrang des Beta-Faltblattbereichs und wahlweise Nicht-Beta-Faltblattbereiche beinhaltet, eine vorher nicht vorhandene Bindungsaffinität gegenüber einem vorbestimmten Bindungspartner aufweist.

2. Protein nach Ausgestaltung 1,
wobei das Protein, das zur Modifikation ausgewählt wird, eine mindestens 30%ige Aminosäuresequenzidentität zu humanem Ubiquitin und ein Ubiquitin-artiges Faltungsmotiv aufweist und/oder der Protein-Familie "ubiquitin-related protein" angehört.

3. Protein nach Ausgestaltung 1 oder 2,
wobei das Protein, das zur Modifikation ausgewählt wird, ein Ubiquitin-artiges Faltungsmotiv aufweist, und bevorzugt aus der Gruppe ausgewählt wird, bestehend aus SUMO-1, FAU, NEDD-8, UBL-1, Rub1, APG8, ISG15, URM1, HUB1, GDX, Elongin B, PLIC2 (N-terminale Domäne), human Parkin (N-terminale Domäne).

4. Protein nach einer oder mehreren der vorhergehenden Ausgestaltungen,
wobei das Protein humanes Ubiquitin oder ein anderes Säuger-Ubiquitin ist.

5. Protein nach einer oder mehreren der vorhergehenden Ausgestaltungen,
wobei die Modifikationen einen zusammenhängenden Bereich von 5 bis 10 Aminosäuren, bevorzugt 6 bis 8 Aminosäuren auf der Oberfläche des Proteins umfassen, wobei hiervon bevorzugt 2 bis 4 Aminosäuren in einem oberflächenexponierten beta-Faltblatt liegen.

6. Protein nach einer oder mehreren der vorhergehenden Ausgestaltungen,
wobei der erste, und vierte.beta-Faltblattstrang an seinen oberflächenexponierten Aminosäuren modifiziert ist, und wahlweise zusätzlich beim Säuger-Ubiquitin die Aminosäurebereiche 62 bis 65 der nicht beta-Faltblattbereiche modifiziert sind.

7. Protein nach einer oder mehreren der vorhergehenden Ausgestaltungen,
wobei die Modifikation eine Substitution, Insertion, Deletion, chemische Modifikation oder Kombinationen hiervon ist, und sie ein oder mehrere Aminosäuren, bevorzugt eine Substitution, zumindest teilweise an in der Primärsequenz direkt benachbarten oder nicht direkt benachbarten Aminosäuren umfaßt,wobei bevorzugt die in der Primärsequenz nicht benachbart liegenden, modifizierten Aminosäuren in der Sekundärstruktur einen bevorzugt zusammenhängenden Bindungsbereich für den Bindungspartner bilden.

8. Protein nach einer oder mehreren der vorhergehenden Ausgestaltungen ,
wobei die Anzahl der Modifikationen, bevorzugt Substitutionen, von direkt in der Primärsequenz benachbarten Aminosäuren 2 bis 8 direkt benachbarte Aminosäuren, weiterhin bevorzugt 3 bis 7 oder 4 bis 6 oder 2 bis 4 direkt benachbarte Aminosäuren, beträgt.

9. Protein nach einer oder mehreren der vorhergehenden Ausgestaltungen,
wobei ein Teil der in der Primärsequenz direkt benachbarten modifizierten Aminosäuren in einem Anfangs- oder Endbereich eines beta-Faltblattstrangs liegt, wobei dieser Teil eine Länge von zwei oder mehr Aminosäuren, bevorzugt zwei oder drei Aminosäuren, aufweist.

10. Protein nach einer oder mehreren der vorhergehenden Ausgestaltungen ,
wobei 5 oder mehr direkt in der Primärsequenz benachbarte Aminosäuren modifiziert, bevorzugt substituiert, sind, wovonein, zwei oder mehr, bevorzugt zwei oder drei, direkt benachbarte Aminosäuren den Anfang oder das Ende eines beta-Faltblattstrangbereichs bilden.

11. Protein nach einer oder mehreren der vorhergehenden Ausgestaltungen , wobei die Modifikation solcher Aminosäuren vorgenommen wird, die einen zusammenhängenden Bereich auf der Oberfläche des Proteins bilden.

12. Protein nach einer oder mehreren der vorhergehenden Ausgestaltungen, wobei Aminosäuren an solchen Positionen modifiziert sind, die nicht zu Bereichen gehören, die bei einem Wildtyp-Protein nach Ausgestaltung 1 an Bindungen zu natürlichen Ubiquitin-Bindungspartnern beteiligt sind.

13. Protein nach einer oder mehreren der vorhergehenden Ausgestaltungen, wobei mindestens 25 % der in Betasträngen befindlichen Aminosäuren des Proteins, bevorzugt des Ubiquitins, modifiziert sind.

14. Protein nach einer oder mehreren der vorhergehenden Ausgestaltungen, wobei eine Modifikation von Aminosäuren auch in Loop-Bereichen des Proteins erfolgt.

15. Protein nach einer oder mehreren der vorhergehenden Ausgestaltungen, wobei es sich um humanes Ubiquitin oder ein hierzu homologes Protein handelt, und mindestens 8 oberflächenexponierte Aminosäuren des Ubiquitins modifiziert, bevorzugt substituiert, sind, so dass diese modifizierten Aminosäuren den Bereich mit Bindungsaffinität zu dem Bindungspartner umfassen.

16. Protein nach Ausgestaltung 15, wobei sich mindestens sechs der acht oberflächenexponierten Aminosäuren in einem Bereich des Proteins befinden, der dem beta-Faltblatt-Bereich zuzuordnen ist.

17. Protein nach Ausgestaltung 15 oder 16, wobei sich mindestens 5 der modifizierten, bevorzugt substituierten, Aminosäuren in direkter Nachbarschaft in der Primärsequenz zueinander befinden.

18. Protein nach einer oder mehreren der vorhergehenden Ausgestaltungen, wobei Aminosäuren modifiziert sind, die sich in dem aminoterminalen Beta-Faltblattstrang und/oder in dem carboxyterminalen Beta-Faltblattstrang des Proteins befinden.

19. Protein nach einer oder mehreren der vorhergehenden Ausgestaltungen, wobei weiterhin Aminosäuren modifiziert sind, die sich in dem an den carboxyterminalen Beta-Faltblattstrang anschließenden Loop befinden.

20. Protein nach einer oder mehreren der vorhergehenden Ausgestaltungen, wobei das modifizierte Protein humanes Ubiquitin ist, welches an den Positionen 2, 4, 6, 62, 63, 64, 65 und 66 substituiert, deletiert, insertiert und/oder chemisch modifiziert, bevorzugt substituiert, wurde.

21. Protein nach einer oder mehreren der vorhergehenden Ausgestaltungen, wobei das Protein, das zur Modifikation ausgewählt wird, bereits Insertionen, Deletionen, Substitutionen und/oder chemische Modifikationen aufweist, so dass biologische und/oder proteinchemische Funktionen des Proteins ausgeschaltet oder neu generiert wurden.

22. Protein nach Ausgestaltung 21, wobei bei dem Protein nach Modifikation insgesamt mindestens 10, bevorzugt mindestens 15 Aminosäuren des Ubiquitins ausgetauscht sind.

23. Protein nach Ausgestaltung 21 oder 22, wobei das Protein, das zur Veränderung durch Substitution, Insertion und/oder Deletion ausgewählt wird, humanes Ubiquitin ist, welches an einer oder mehreren der folgenden Positionen substituiert ist: 44, 48, 54, 70, 72, 75, und bei der die Aminosäureposition 76 deletiert ist, so dass das Ubiquitin weitgehend keine Wechselwirkung mit seinen natürlichen Bindungspartnern mehr aufweist.

24. Protein nach nach einer oder mehreren der vorhergehenden Ausgestaltungen , wobei das Protein humanes Ubiquitin ist, welches an Position 45 die Substitution Phenylalanin zu Tryptophan enthält.

25. Protein nach einer oder mehreren der vorhergehenden Ausgestaltungen,
wobei durch Random-Mutagenese ein zufälliger Austausch der ausgewählten Aminosäuren erfolgt ist.

26. Protein nach einer oder mehreren der vorhergehenden Ausgestaltungen,
wobei der Bindungspartner ein Antigen oder ein Hapten ist.

27. Protein nach einer oder mehreren der vorhergehenden Ausgestaltungen,
wobei die Bindungsaffinität, ausgedrückt in $K_D$, zu dem vorbestimmten Bindungspartner $10^{-6}$ M bis $10^{-12}$ M, weiterhin bevorzugt $10^{-8}$ bis $10^{-12}$ M oder $10^{-9}$ bis $10^{-12}$ M, beträgt.

28. Protein, das aus der Gruppe ausgewählt ist, bestehend aus Proteinen der Protein-Superfamilie "ubiquitin-like proteins", Proteinen, die ein Ubiquitin-artiges Faltungsmotiv aufweisen sowie Fragmenten oder Fusionsproteinen hiervon, die je das Ubiquitin-artige Faltungsmotiv besitzen, wobei das Protein unter Beibehaltung des Ubiquitin-artigen Faltungsmotivs, aufgrund einer oder mehrerer Modifikationen eine vorher nicht vorhandene Bindungsaffinität gegenüber einem vorbestimmten Bindungspartner aufweist, erhältlich durch folgendes Verfahren:

a) Auswählen eines zu modifizierenden Proteins;
b) Bestimmen eines Bindungspartners;
c) Auswählen von Aminosäuren in einem oberflächenexponierten Bereich des Proteins, der mindestens einen beta-Faltblattstrang des Beta-Faltblattbereichs und wahlweise Nicht-Beta-Faltblattbereiche beinhaltet;
d) Modifizieren der ausgewählten Aminosäuren bevorzugt durch Substitution, Insertion, Deletion und/oder chemische Modifikation, unter Beibehaltung des Ubiquitin-artigen Faltungsmotivs;
e) Inkontaktbringen des modifizierten Proteins mit dem in Schritt b) bestimmten Bindungspartner;
f)Ermitteln derjenigen Proteine, die eine Bindungsaffinität gegenüber dem in Schritt b) vorbestimmten Bindungspartner aufweisen.

29. Protein nach einer oder mehreren der vorhergehenden Ausgestaltungen,
wobei das Protein mit einem Protein gleicher Spezifität oder unterschiedlicher Spezifität ortsgerichtet und kovalent verknüpft ist und so bivalente bzw. bispezifische Bindungseigenschaften aufweist

30. Verfahren zur Herstellung eines Proteins nach einer oder mehreren der vorhergehenden Ausgestaltungen, ausgewählt aus der Gruppe, bestehend aus Proteinen der Protein-Superfamilie "ubiquitin-like proteins", Proteinen, die ein Ubiquitin-artiges Faltungsmotiv aufweisen sowie Fragmenten oder Fusionsproteinen hiervon, die je das Ubiquitinartite Faltungsmotiv besitzen, wobei das Protein unter Beibehaltung des Ubiquitin-artigen Faltungsmotivs, aufgrund einer oder mehrerer Modifikationen von Aminosäuren in zumindest einem oberflächenexponierten Bereich des Proteins, der mindestens einen beta-Faltblattstrang des Beta-Faltblattbereichs und wahlweise Nicht-Beta-Faltblattbereiche beinhaltet, eine vorher nicht vorhandene Bindungsaffinität gegenüber einem vorbestimmten Bindungspartner aufweist, mit den Schritten:

a) Auswählen eines zu modifizierenden Proteins;
b) Bestimmen eines Bindungspartners;
c) Auswählen von Aminosäuren in einem oberflächenexponierten Bereich des Proteins, der mindestens einen beta-Faltblattstrang des Beta-Faltblattbereichs und wahlweise Nicht-Beta-Faltblattbereiche beinhaltet;
d) Modifizieren der ausgewählten Aminosäuren, bevorzugt durch Substitution, Insertion, Deletion und/oder chemische Modifikation unter Beibehaltung des Ubiquitin-artigen Faltungsmotivs;
e) Inkontaktbringen des modifizierten Proteins mit dem in Schritt b) bestimmten Bindungspartner;
f) Ermitteln derjenigen Proteine, die eine Bindungsaffinität gegenüber dem in Schritt b) vorbestimmten Bindungspartner aufweisen.

31. Verfahren nach Ausgestaltung 30,
wobei die Schritte c) bis d) durch chemische Synthese des modifizierten Proteins realisiert werden.

32. Verfahren nach Ausgestaltung 31,
wobei die Modifikation in Schritt d) mittels gentechnischer Veränderung einer zu dem entsprechenden modifizierten Protein zugehörigen DNA erfolgt, und die Expression des Proteins in prokaryotischen oder eukaryotischen Wirtsorganismen oder *in vitro* erfolgt.

33. Verfahren nach Ausgestaltung 30, wobei in Schritt d) eine Genbibliothek erstellt wird.

34. Verfahren nach einer oder mehreren der vorhergehenden Ausgestaltungen, wobei in Schritt d) durch Random-Mutagenese ein zufälliger Austausch der ausgewählten Aminosäuren erfolgt ist.

35. Verfahren nach einer oder mehreren der vorhergehenden Ausgestaltungen, wobei in Schritt e) das Inkontaktbringen mit dem vorbestimmten Bindungspartner mittels eines geeigneten Selektionsverfahrens erfolgt, bevorzugt dem Phage-Display-, Ribosomal Display-, mRNA-Display-, CIS-Display- oder Cell-Surface-Display-Verfahren, Yeast Surface Display, Bacterial Surface Display, insbesondere bevorzugt mittels des Phage-Display-Verfahrens.

36. Verfahren nach einer oder mehreren der vorhergehenden Ausgestaltungen, wobei in Schritt f) die Ermittlung der Proteine mit einer Bindungsaffinität zu dem vorbestimmten Bindungspartner durch eines oder mehrere der folgenden Verfahren erfolgt: ELISA, Plasmon-Oberflächenresonanz-Spektroskopie, Fluoreszenz-Spektroskopie, FACS, Isothermale Titrationskalorimetrie oder Analytische Ultrazentrifugation.

37. Verfahren nach einer oder mehreren der vorhergehenden Ausgestaltungen, wobei sich an Schritt f) ein Schritt der Isolierung und/oder Anreicherung des ermittelten Proteins mit Bindungsaffinität zu dem vorbestimmten Bindungspartner anschließt.

38. Verfahren nach einer oder mehreren der vorhergehenden Ausgestaltungen, wobei das Protein durch an sich bekannte Verfahren hinsichtlich seiner Bindungsaffinität, seiner Bindungsspezifität und/oder anderer proteinchemischer Eigenschaften, z. B. Stabilität, Löslichkeit oder Ausbeute, maturiert wird.

39. Verfahren nach einer oder mehreren der vorhergehenden Ausgestaltungen, wobei das Protein ortsgerichtet und kovalent mit einem Protein gleicher oder unterschiedlicher Spezifität verknüpft wird, wodurch ein bivalentes oder bispezifisches Protein erhalten wird.

40. Verwendung eines Proteins nach einer oder mehreren der vorhergehenden Ausgestaltungen zum Nachweis, zur quantitativen Bestimmung, zur Trennung und/oder zur Isolierung des entsprechenden Bindungspartners mittels an sich bekannter Verfahren, wie Chromatographie oder Adsorptions-Technologie.

41. Verwendung eines Proteins nach einer oder mehreren der vorhergehenden Ausgestaltungen zur Diagnose, Prophylaxe und Behandlung von Krankheiten, an denen der entsprechende Bindungspartner direkt oder indirekt beteiligt ist.

**Patentansprüche**

1. Modifiziertes Säuger-Ubiquitin-Protein,
**dadurch gekennzeichnet, dass**
das Protein, unter Beibehaltung des ubiquitin-artigen Faltungsmotivs, auf Grund von Modifikationen eine vorher nicht vorhandene Bindungsaffinität gegenüber einem vorbestimmten Bindungspartner aufweist, wobei

    • 15% - 40% der in den Betasträngen befindlichen Aminosäuren im Beta-Faltblattbereich modifiziert sind.
    • unter einem ubiquitin-artigen Faltungsmotiv ein zu einer Oberfläche des Ubiquitinproteins exponiertes antiparalleles Beta-Faltblatt zu verstehen ist, das auf seiner Rückseite von einer senkrecht darüber liegenden alpha-Helix bedeckt wird, mit der Anordnung beta(2)-alpha-beta(2);
    • die Modifikationen ausgewählt sind aus Insertionen, Deletionen, Substitutionen und/oder chemischen Modifikationen,
    • solche Aminosäuren modifiziert sind, die einen zusammenhängenden Bereich von 5 bis 10 Aminosäuren auf der Oberfläche des Proteins bilden;
    • die Modifikationen solche Aminosäuren betreffen, die in zumindest einem oberflächenexponierten Bereich des Proteins liegen, der zumindest einen Beta-Faltblattstrang des Beta-Faltblattbereichs und wahlweise Nicht-Beta-Faltblattbereiche beinhaltet;
    • mindestens vier oberflächenexponierte Aminosäuren im Beta-Faltblattbereich modifiziert vorliegen;
    • die Bindungsaffinität, ausgedrückt in $K_D$, zum vorbestimmten Bindungspartner $10^{-5}$ M bis $10^{-12}$ M beträgt.

2. Protein nach Anspruch 1,
wobei das Protein humanes Ubiquitin ist.

3. Protein nach Anspruch 1 oder 2,
wobei der erste, und vierte beta-Faltblattstrang an seinen oberflächenexponierten Aminosäuren modifiziert ist

4. Protein nach Anspruch 3,
wobei wahlweise zusätzlich beim Säuger-Ubiquitin die Aminosäurebereiche 62 bis 65 der nicht beta-Faltblattbereiche modifiziert sind.

5. Protein nach einem oder mehreren der vorhergehenden Ansprüche,
wobei eine Modifikation von Aminosäuren auch in Loop-Bereichen des Proteins erfolgt.

6. Protein nach einem oder mehreren der vorhergehenden Ansprüche,
wobei es sich um humanes Ubiquitin oder ein hierzu homologes Protein handelt, und mindestens 8 oberflächenexponierte Aminosäuren des Ubiquitins modifiziert, bevorzugt substituiert, sind, so dass diese modifizierten Aminosäuren den Bereich mit Bindungsaffinität zu dem Bindungspartner umfassen.

7. Protein nach Anspruch 6,
wobei sich mindestens sechs der acht oberflächenexponierten Aminosäuren in einem Bereich des Proteins befinden, der dem beta-Faltblatt-Bereich zuzuordnen ist.

**8.** Protein nach einem oder mehreren der vorhergehenden Ansprüche, wobei das modifizierte Protein humanes Ubiquitin ist, welches an den Positionen 2, 4, 6, 62, 63, 64, 65 und 66 substituiert, deletiert, insertiert und/oder chemisch modifiziert, bevorzugt substituiert, wurde.

**9.** Protein nach einem oder mehreren der vorhergehenden Ansprüche, wobei der Bindungspartner ein Antigen oder ein Hapten ist.

**10.** Modifiziertes Ubiquitin-Protein,
**dadurch gekennzeichnet, dass**
das Protein, unter Beibehaltung des ubiquitin-artigen Faltungsmotivs, auf Grund von Modifikationen eine vorher nicht vorhandene Bindungsaffinität gegenüber einem vorbestimmten Bindungspartner aufweist, wobei

- 15% - 40 % der in den Betasträngen befindlichen Aminosäuren modifiziert sind;
- unter einem ubiquitin-artigen Faltungsmotiv ein zu einer Oberfläche des Ubiquitinproteins exponiertes antiparalleles Beta-Faltblatt zu verstehen ist, das auf seiner Rückseite von einer senkrecht darüber liegenden alpha-Helix bedeckt wird, mit der Anordnung beta(2)-alpha-beta(2);
- die Modifikationen ausgewählt sind aus Insertionen, Deletionen, Substitutionen und/oder chemischen Modifikationen;
- solche Aminosäuren modifiziert sind, die einen zusammenhängenden Bereich von 5 bis 10 Aminosäuren auf der Oberfläche des Proteins bilden;
- die Modifikationen solche Aminosäuren betreffen, die in zumindest einem oberflächenexponierten Bereich des Proteins liegen, der zumindest einen Beta-Faltblattstrang des Beta-Faltblattbereichs und wahlweise Nicht-Beta-Faltblattbereiche beinhaltet;
- mindestens vier oberflächenexponierte Aminosäuren im Beta-Faltblattbereich modifiziert vorliegen;
- die Bindungsaffinität, ausgedrückt in $K_D$, zum vorbestimmten Bindungspartner $10^{-5}$ M bis $10^{-12}$ M beträgt, erhältlich durch folgendes Verfahren:

  a) Auswählen des zu modifizierenden Ubiquitin-Proteins;
  b) Auswählen eines Bindungspartners;
  c) Bestimmung der oberflächenexponierten Aminosäuren des Proteins aus Schritt a);
  d) Auswählen von Aminosäurepositionen in zumindest einem oberflächenexponiertem Bereich des Proteins, der mindestens einen beta-Faltblattstrang des Beta-Faltblattbereichs und wahlweise Nicht-Beta-Faltblattbereiche beinhaltet;
  e) Modifizieren der ausgewählten Aminosäurepositionen durch Substitution, Insertion, Deletion und/oder chemische Modifikation, unter Beibehaltung des ubiquitin-artigen Faltungsmotivs;
  f) Inkontaktbringen des modifizierten Proteins mit dem in Schritt b) bestimmten Bindungspartner;
  g) Ermitteln derjenigen Proteine, die eine Bindungsaffinität, ausgedrückt in $K_D$, zum vorbestimmten Bindungspartner von $10^{-5}$ M bis $10^{-12}$ M gegenüber dem im Schritt b) vorbestimmten Bindungspartner aufweisen, und die solche Modifikationen aufweisen, die einen zusammenhängenden Bereich von 5-10 Aminosäuren auf der Oberfläche des Proteins bilden, wobei 15% - 40% der in den Betasträngen befindlichen Aminosäuren im Beta-Faltblattbereich modifiziert vorliegen.

**11.** Protein nach einem oder mehreren der vorhergehenden Ansprüche, wobei als oberflächenexponierte Aminosäuren Aminosäuren in den Positionen 2, 4, 6, 62, 63, 64, 65 und 66 ausgewählt werden.

**12.** Protein nach einem oder mehreren der vorhergehenden Ansprüche, wobei das Protein mit einem Protein gleicher Spezifität oder unterschiedlicher Spezifität ortsgerichtet und kovalent verknüpft ist und bivalente bzw. bispezifische Bindungseigenschaften aufweist.

**13.** Verwendung eines Proteins nach einem oder mehreren der vorhergehenden Ansprüche zum Nachweis, zur quantitativen Bestimmung, zur Trennung und/oder zur Isolierung des entsprechenden Bindungspartners mittels an sich bekannter Verfahren, wie Chromatographie oder Adsorptions-Technologie.

**14.** Verwendung eines Proteins nach einem oder mehreren der vorhergehenden Ansprüche zur Diagnose, Prophylaxe und Behandlung von Krankheiten, an denen der entsprechende Bindungspartner direkt oder indirekt beteiligt ist.

**15.** Fusionsprotein, umfassend ein modifiziertes Ubiquitin-Protein nach einem oder mehreren der vorhergehenden Ansprüche 1 bis 12, wobei optional das Protein mit einem Protein gleicher Spezifität oder unterschiedlicher Spezifität ortsgerichtet und kovalent verknüpft ist und bivalente Bindungseigenschaften besitzt.

**Abb. 1**

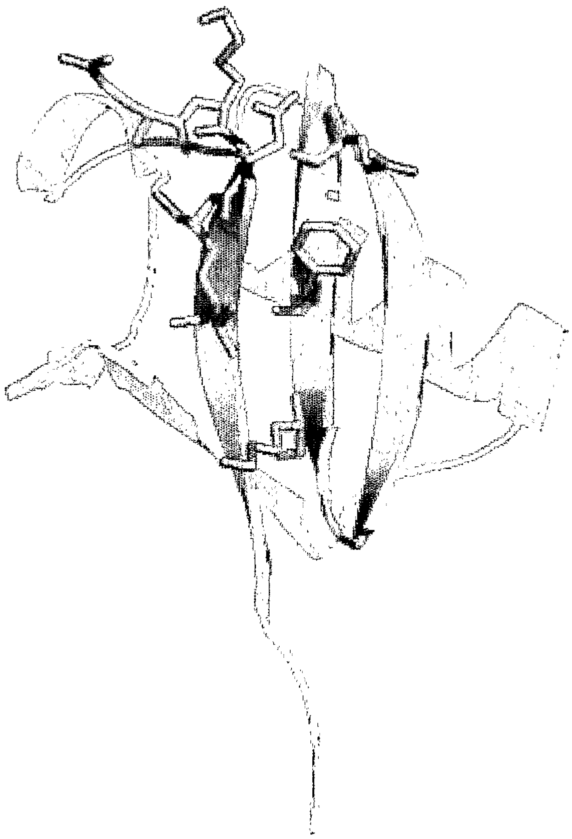

Abb. 2

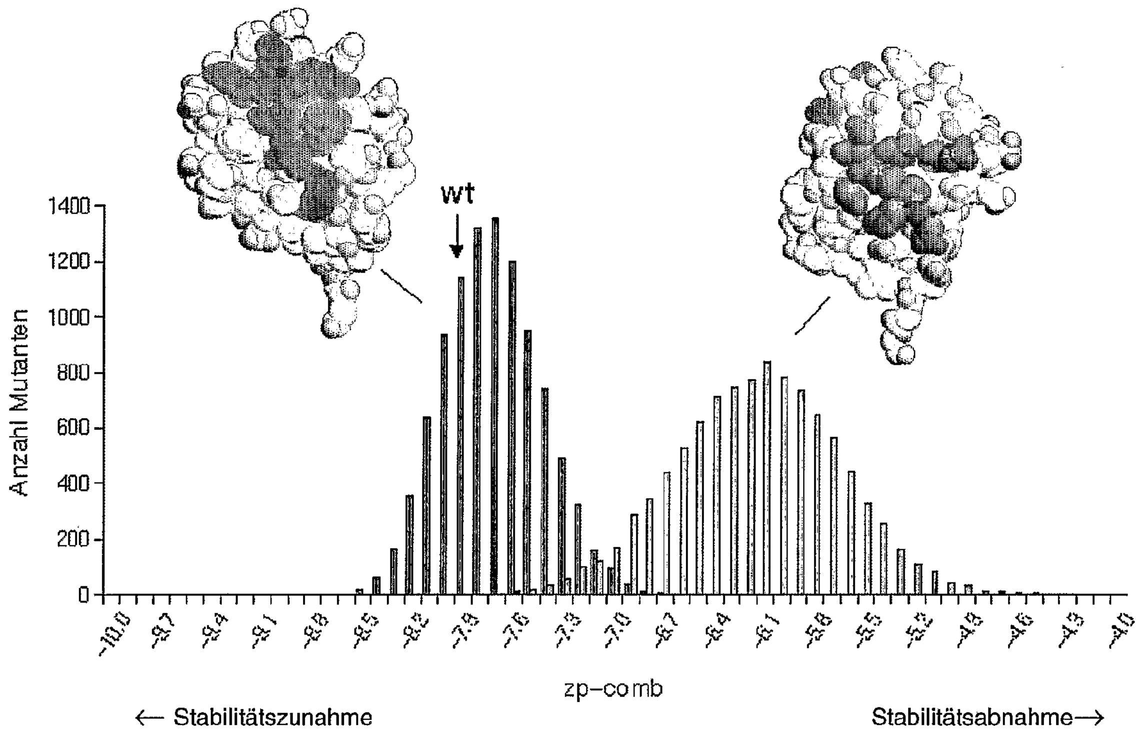
wt
Anzahl Mutanten
1400
1200
1000
800
600
400
200
0
-10.0
-9.7
-9.4
-9.1
-8.8
-8.5
-8.2
-7.9
-7.6
-7.3
-7.0
-6.7
-6.4
-6.1
-5.8
-5.5
-5.2
-4.9
-4.6
-4.3
-4.0
zp-comb
← Stabilitätszunahme
Stabilitätsabnahme→

**Abb. 3**

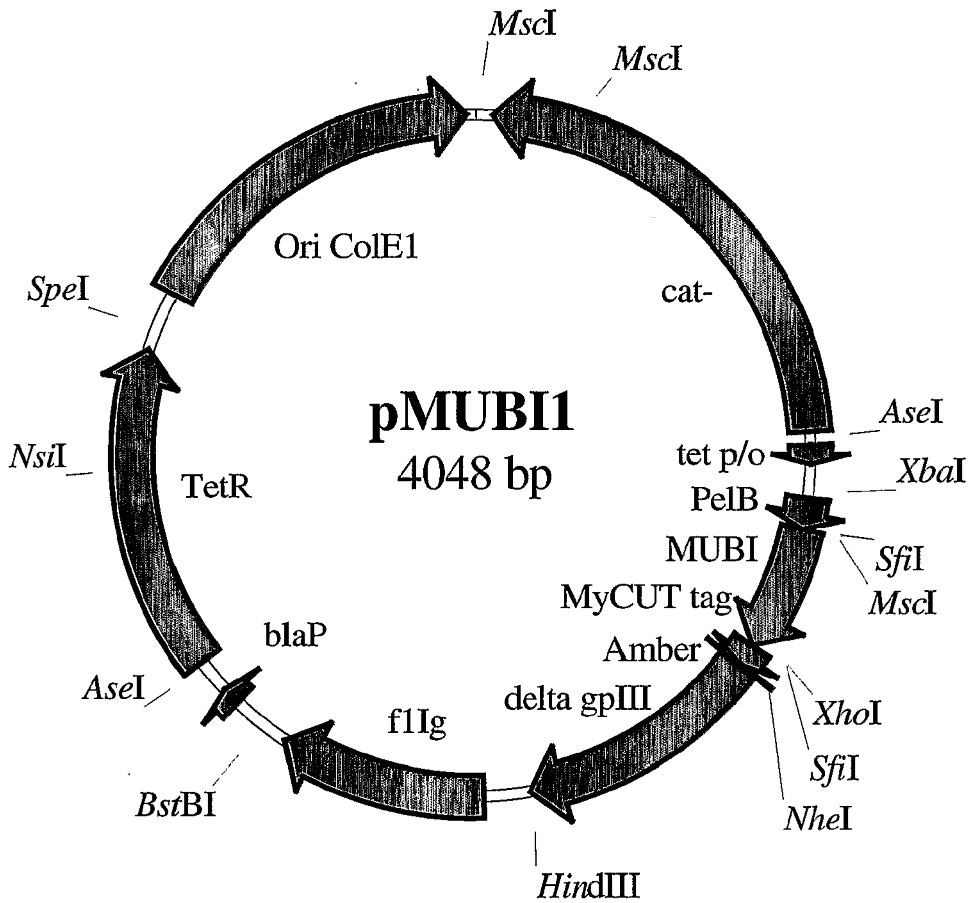
MscI
MscI
Ori ColE1
cat-
SpeI
pMUBI1
4048 bp
AseI
NsiI
TetR
tet p/o
XbaI
PelB
MUBI
SfiI
MyCUT tag
MscI
blaP
Amber
AseI
delta gpIII
XhoI
f1Ig
SfiI
BstBI
NheI
HindIII

**Abb. 4**

```
                                  ====SfiI=====
                   Seq-ID No.29 GTTATTACTCGCGGCCCAGCCGGCCATGGCCATG

                          Seq-ID No. 30 CCAGCCGGCCATGGCCATGNNKATCNNKGTTNNKACCCTGACGGGAAAGACTATC

ATGAAATACCTATTGCCTACGGCAGCCGCTGGATTGTTATTACTCGCGGCCCAGCCGGCCATGGCCATGCAAATCTTCGTTAAAACCCTGACGGGAAAGACTATCACCCTGGAGGTA
---------+---------+---------+---------+---------+---------+---------+---------+---------+---------+---------+---------+
TACTTTATGGATAACGGATGCCGTCGGCGACCTAACAATAATGAGCGCCGGGTCGGCCGGTACCGGTACGTTTAGAAGCAATTTTGGGACTGCCCTTTCTGATAGTGGGACCTCCAT

MetLysTyrLeuLeuProThrAlaAlaAlaGlyLeuLeuLeuLeuAlaAlaGlnProAlaMetAlaMetXxxIleXxxValXxxThrLeuThrGlyLysThrIleThrLeuGluVal
PelB →                                                                     Ubiquitin →


GAACCGTCCGACACCATCGAAAATGTCAAAGCTAAAATCCAAGACAAAGAAGGAATTCCACCTGACCAGCAACGCCTAGCTTTCGCAGGACGACAACTAGAGGACGGGCTCACCCTG
--+---------+---------+---------+---------+---------+---------+---------+---------+---------+---------+---------+-------
CTTGGCAGGCTGTGGTAGCTTTTACAGTTTCGATTTTAGGTTCTGTTTCTTCCTTAAGGTGGACTGGTCGTTGCGGATCGAAAGCGTCCTGCTGTTGATCTCCTGCCCGAGTGGGAC

                                                                                                                GGAC

GluProSerAspThrIleGluAsnValLysAlaLysIleGlnAspLysGluGlyIleProPpoAspGlnGlnArgLeuAlaPheAlaGlyArgGlnLeuGluAspGlyLeuThrLeu


TCTGACTACAACATCCAAAAAGAATCCACCCTCCACCTGGCACTCCTCCTGCGGGCCCTCGAGGCCGAACAAAAACTCATCTCAGAAGAGAATCTGTATTTCCAGGGCTAG Seq-ID
---+---------+---------+---------+---------+---------+---------+---------+---------+---------+---------+--------  No. 32
AGACTGATGTTGTAGGTTTTTCTTAGGTGGGAGGTGGACCGTGAGGAGGACGCCCGGGAGCTCCGGCTTGTTTTTGAGTAGAGTCTTCTCTTAGACATAAAGGTCCCGATC

SerAspTyrAsnIleXxxXxxXxxXxxXxxLeuHisLeuAlaLeuLeuLeuArgAlaLeuGluAlaGluGlnLysLeuIleSerGluGluAsnLeuTyrPheGlnGlyStp Seq-ID
No.31

AGACTGATGTTGTAGNNMNNMNNMNNMNNMGAGGTGGACCGTGAGGAGGAC Seq-ID No. 33

                                GAGGTGGACCGTGAGGAGGACGCCCGGGAGCTCCGGCTTGTTTTTGAG Seq-ID No.34
                                                   ====SfiI=====
```

**Abb. 5**

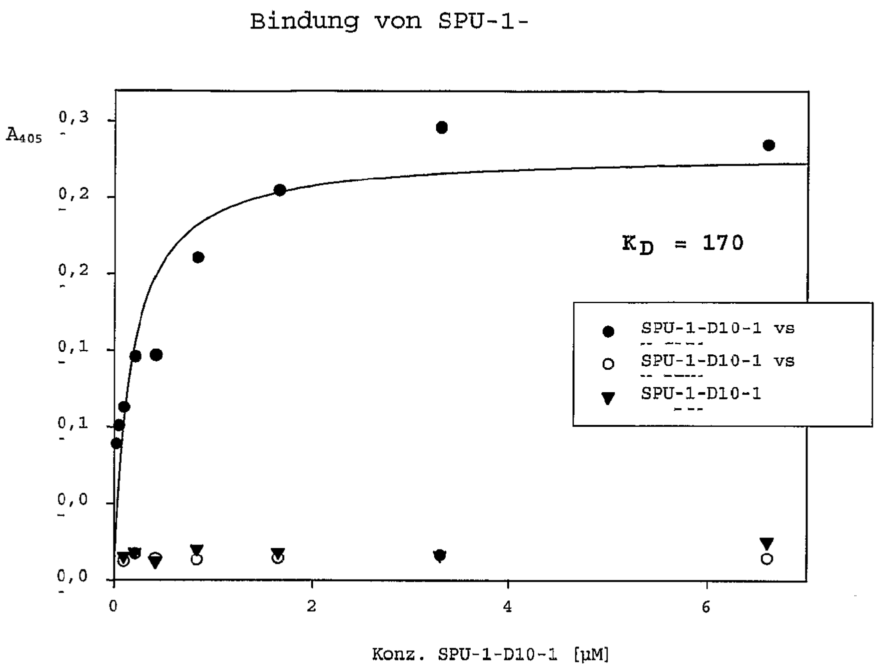
Bindung von SPU-1-
$A_{405}$
0,3
0,2
0,2
0,1
0,1
0,0
0,0
0
2
4
6
$K_D$ = 170
SPU-1-D10-1 vs
SPU-1-D10-1 vs
SPU-1-D10-1
Konz. SPU-1-D10-1 [µM]

**Abb. 6**

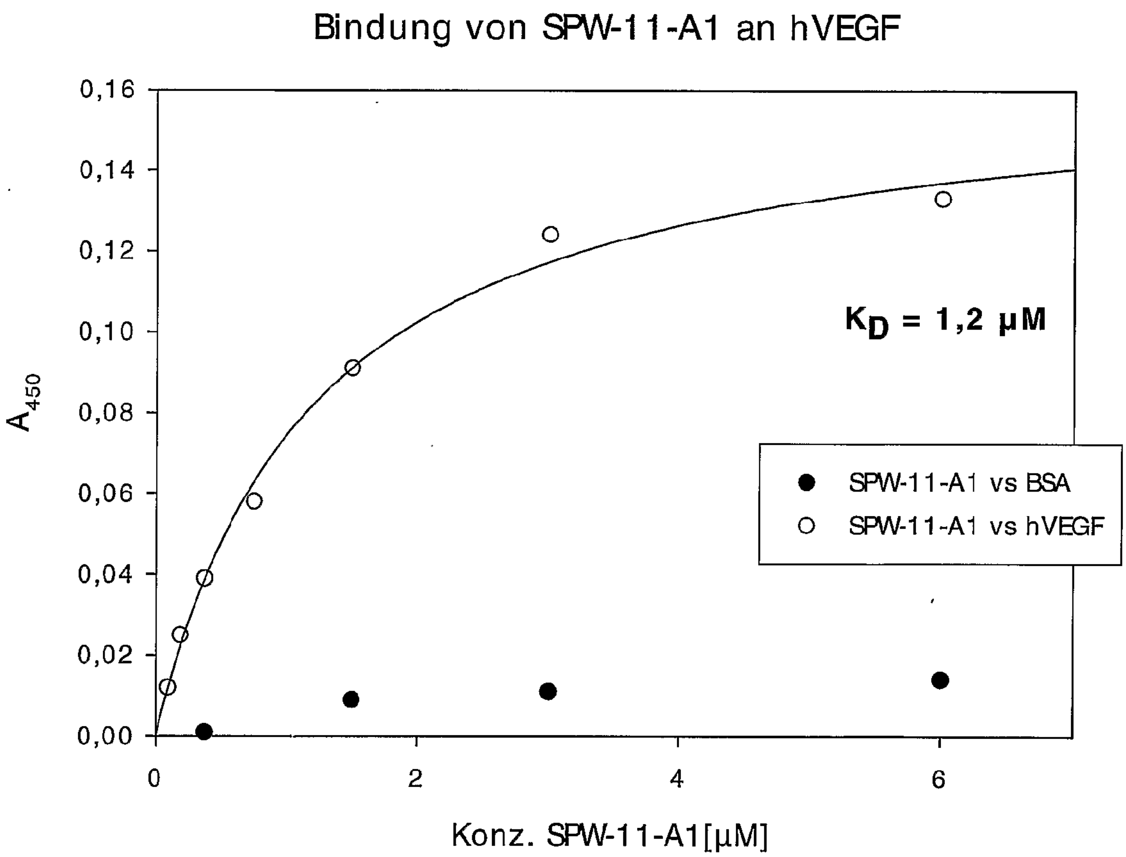
Bindung von SPW-11-A1 an hVEGF
0,16
0,14
0,12
0,10
0,08
0,06
0,04
0,02
0,00
$A_{450}$
$K_D$ = 1,2 µM
SPW-11-A1 vs BSA
SPW-11-A1 vs hVEGF
0
2
4
6
Konz. SPW-11-A1[µM]

**Abb. 7**

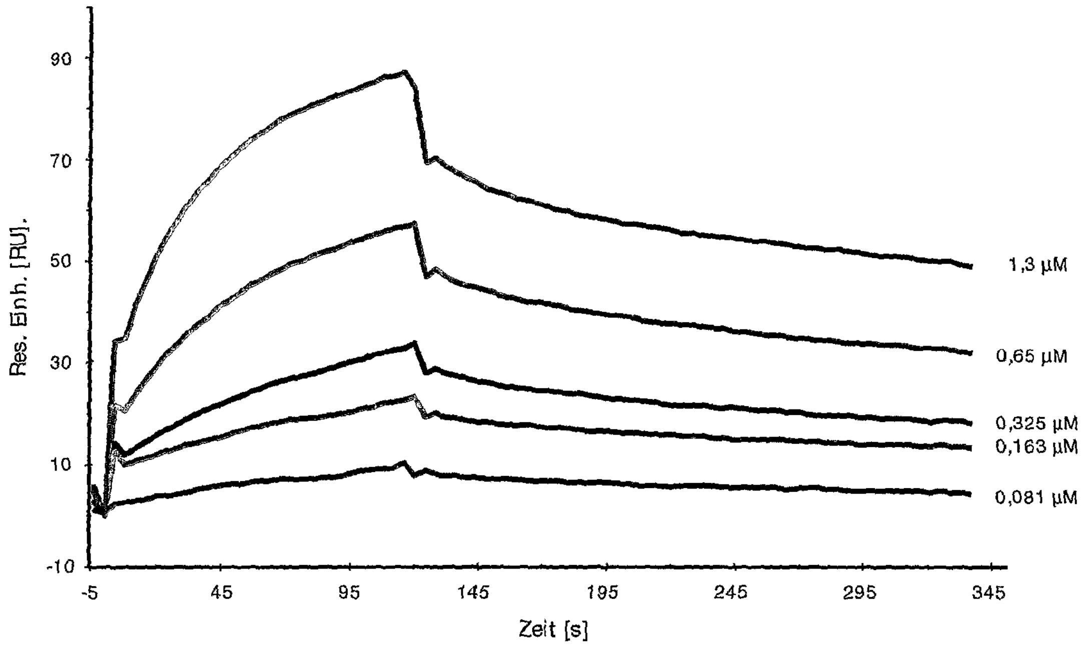
90
70
50
30
10
-10
Res. Einh. [RU].
-5
45
95
145
195
245
295
345
Zeit [s]
1,3 µM
0,65 µM
0,325 µM
0,163 µM
0,081 µM

**Abb 8**

Bindung von SPU-3-H13-O an Hydrocortison, Testosteron, Östradiol und BSA (Trägerprotein) - Nachweis mit Ni-NTA/POD

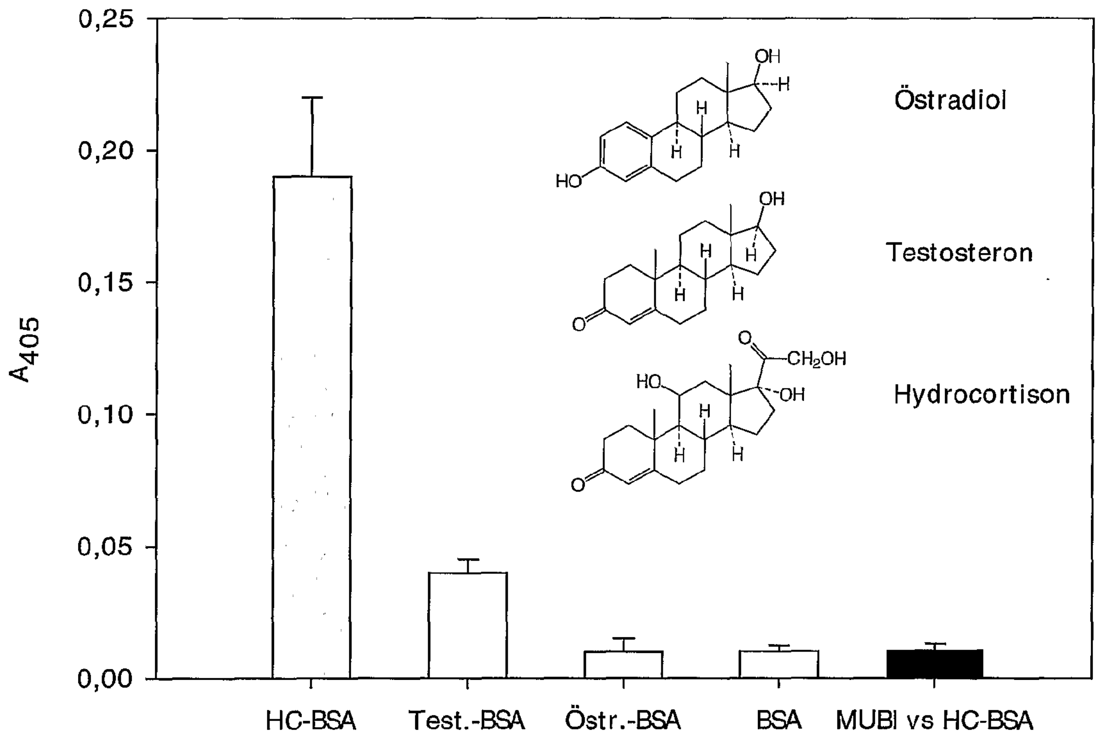

**Abb. 9**

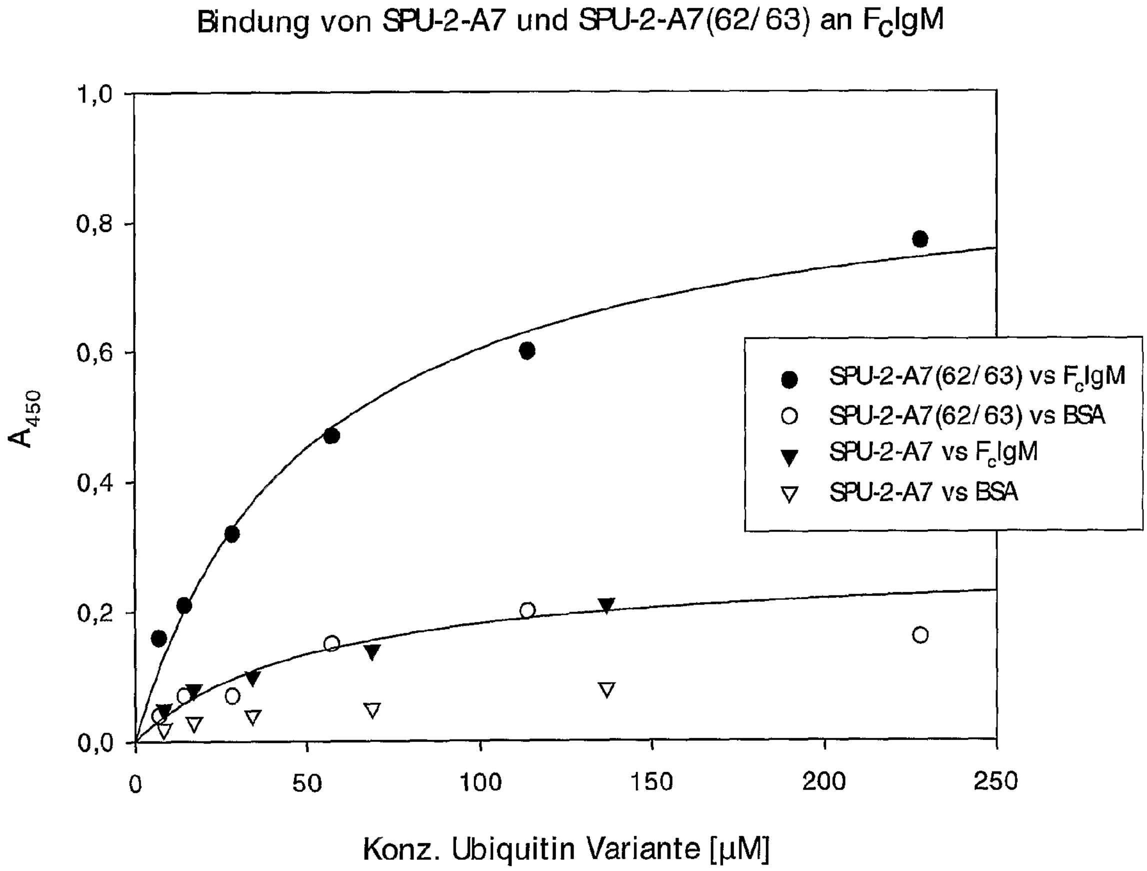
Bindung von SPU-2-A7 und SPU-2-A7(62/63) an $F_C$IgM
1,0
0,8
0,6
0,4
0,2
0,0
$A_{450}$
0
50
100
150
200
250
Konz. Ubiquitin Variante [µM]
SPU-2-A7(62/63) vs $F_c$IgM
SPU-2-A7(62/63) vs BSA
SPU-2-A7 vs $F_c$IgM
SPU-2-A7 vs BSA

Abb. 10

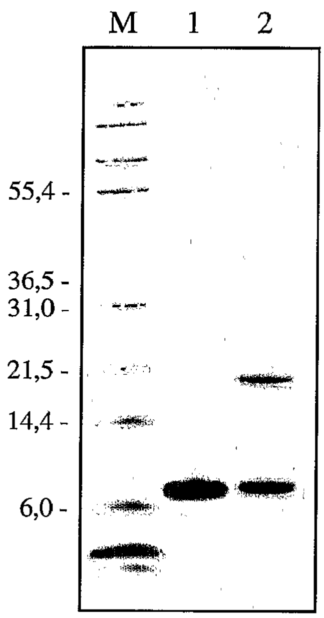

Europäisches Patentamt
European Patent Office
Office européen des brevets

# EUROPÄISCHER RECHERCHENBERICHT

Nummer der Anmeldung
EP 10 18 1802

| Kategorie | EINSCHLÄGIGE DOKUMENTE<br>Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | WO 01/04144 A (FIEDLER ULRIKE ; RUDOLPH RAINER (DE)) 18. Januar 2001 (2001-01-18)<br>* Seite 9, Zeile 8 - Seite 10, Zeile 4 *<br>----- | 1-15 | INV.<br>C07K14/435<br>C07K14/47<br>C12N15/09<br>C12N15/62 |
| Y | YEH E T H ET AL: "Ubiquitin-like proteins: new wines in new bottles",<br>GENE, ELSEVIER BIOMEDICAL PRESS. AMSTERDAM, NL,<br>Bd. 248, Nr. 1-2, Mai 2000 (2000-05), Seiten 1-14, XP004198791,<br>ISSN: 0378-1119<br>* Seite 2, rechte Spalte, Absatz 2 - Seite 3, rechte Spalte, Absatz 1 *<br>----- | 1-15 | |
| Y,D | SKERRA A: "ENGINEERED PROTEIN SCAFFOLDS FOR MOLECULAR RECOGNITION",<br>JOURNAL OF MOLECULAR RECOGNITION, HEYDEN & SON LTD., LONDON, GB,<br>Bd. 13, Nr. 4, Juli 2000 (2000-07), Seiten 167-187, XP009019725,<br>ISSN: 0952-3499<br>* Seite 168, rechte Spalte, Absatz 3; Tabelle 1 *<br>----- | 1-15 | RECHERCHIERTE SACHGEBIETE (IPC)<br>C07K<br>C12N |
| X | JENTSCH ET AL: "Ubiquitin and its kin: how close are the family ties?",<br>TRENDS IN CELL BIOLOGY, ELSEVIER SCIENCE LTD, XX,<br>Bd. 10, Nr. 8, 1. August 2000 (2000-08-01), Seiten 335-342, XP002988531,<br>ISSN: 0962-8924<br>* Seite 335, linke Spalte - Seite 336, linke Spalte; Abbildungen 1,2,4 *<br>* Seite 340, rechte Spalte, Absatz 3 *<br>-----<br>-/-- | 1-12 | |

Der vorliegende Recherchenbericht wurde für alle Patentansprüche erstellt

1

| Recherchenort | Abschlußdatum der Recherche | Prüfer |
|---|---|---|
| München | 28. Januar 2011 | Strobel, Andreas |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.......................................................................
& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 10 18 1802

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | ECKER D J ET AL: "GENE SYNTHESIS, EXPRESSION, STRUCTURES, AND FUNCTIONAL ACTIVITIES OF SITE-SPECIFIC MUTANTS OF UBIQUITIN", JOURNAL OF BIOLOGICAL CHEMISTRY, AMERICAN SOCIETY OF BIOLOCHEMICAL BIOLOGISTS, BIRMINGHAM, US, Bd. 262, Nr. 29, 15. Oktober 1987 (1987-10-15), Seiten 14213-14221, XP002974633, ISSN: 0021-9258 * Zusammenfassung * * Tabelle 1 * ----- | 1-12 | |
| X | LARSEN CHRISTOPHER N ET AL: "The ubiquitin superfamily: Members, features, and phylogenies", JOURNAL OF PROTEOME RESEARCH, Bd. 1, Nr. 5, September 2002 (2002-09), Seiten 411-419, XP002561605, ISSN: 1535-3893 * das ganze Dokument * ----- | 1-15 | RECHERCHIERTE SACHGEBIETE (IPC) |

Der vorliegende Recherchenbericht wurde für alle Patentansprüche erstellt

1

| Recherchenort | Abschlußdatum der Recherche | Prüfer |
|---|---|---|
| München | 28. Januar 2011 | Strobel, Andreas |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 10 18 1802

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

28-01-2011

| Im Recherchenbericht angeführtes Patentdokument | Datum der Veröffentlichung | Mitglied(er) der Patentfamilie | Datum der Veröffentlichung |
|---|---|---|---|
| WO 0104144 A | 18-01-2001 | AT 342359 T | 15-11-2006 |
| | | AU 5827800 A | 30-01-2001 |
| | | CA 2378871 A1 | 18-01-2001 |
| | | CN 1371415 A | 25-09-2002 |
| | | CN 101693889 A | 14-04-2010 |
| | | DE 19932688 A1 | 18-01-2001 |
| | | DK 1200583 T3 | 19-02-2007 |
| | | EP 1200583 A2 | 02-05-2002 |
| | | ES 2273705 T3 | 16-05-2007 |
| | | JP 4221174 B2 | 12-02-2009 |
| | | JP 2003504081 T | 04-02-2003 |
| | | JP 4597177 B2 | 15-12-2010 |
| | | JP 2008019276 A | 31-01-2008 |
| | | PT 1200583 E | 29-12-2006 |
| | | US 7601803 B1 | 13-10-2009 |
| | | US 2007111287 A1 | 17-05-2007 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 0104144 A
- US 5789166 A
- US 4873192 A


### In der Beschreibung aufgeführte Nicht-Patentliteratur


- **Finucane et al.** *Biochemistry,* 1999, vol. 38 (36
- **Lazar et al.** *Protein Science,* 1997, vol. 6, 1167-1178
- **Hoess.** *Curr. Opin. Struct. Biol.,* 1993, vol. 3, 572-579
- **Wells ; Lowmann.** *Curr. Opin. Strut. Biol.,* 1992, vol. 2, 597-604
- **Kay et al.** Phage Display of Peptides and Proteins - A Laboratory Manual. Academic Press, 1996
- **Daugherty et al.** *Bacterial Surface Display,* 1998
- **Kieke et al.** *Yeast Surface Display,* 1997
- **Ausuebel, F.M. ; Brent, R., Kinston, R.E. ; Moore, D.D. ; Seidmann, J.G. ; Smith, J.A. ; Struhl, K.** Current protocols in molecular biology. John Wiley & Sons, Inc, 1994
- **Bazarsuren, A. ; Grauschopf, U. ; Wozny, M. ; Reusch, D. ; Hoffmann, E. ; Schaefer, W. ; Panzner, S. ; Rudolph, R.** In vitro folding, functional characterization, and disulfide pattern of the extracellular domain of human GLP-1 receptor. *Biophys. Chem.,* 2002, vol. 96, 305-318
- **Beal, R. ; Deveraux, Q. ; Xia, G. ; Rechsteiner, M. ; Pickart, C.** Surface hydrophobic residues of multiubiquitin chains essential for proteolytic targeting. *Proc. Natl. Acad. Sci. USA,* 1996, vol. 93, 861-866
- **Berman, H. M. ; Westbrook, J. ; Feng, Z. ; Gilliland, G. ; Bhat, T. N. ; Weissig, H. ; Shindyalov, I. N. ; Bourne, P. E.** The Protein Data Bank. *Nucleic Acid Res.,* 2000, vol. 28, 235-242
- **Beste, G. ; Schmidt, F. S. ; Stibora, T. ; Skerra, A.** Small antibody-like proteins with predescribed ligand specificities derived from the lipocalin fold. *Proc. Natl. Acad. Sci. USA,* 1999, vol. 96, 1898-1903
- **Bird, R. E. ; Hardman, K. D. ; Jacobson, J. W. ; Johnson, S. ; Kaufman, R. ; Lee, S. M. ; Pope, H. S. ; Riordan, G. S. ; Whitlow, M.** Single-chain antigen-binding proteins. *Science,* 1988, vol. 242, 423-426
- **Burch, T. J. ; Haas, A. L.** Site-directed mutagenesis ofUbiquitin. Differential roles for Arginine in the interaction with Ubiquitin-activating enzyme. *Biochemistry,* 1994, vol. 33, 7300-7308
- **Brinkmann, U. ; Reiter, Y. ; Jung, S. H. ; Lee, B. ; Pastan, I.** A recombinant immunotoxin containing a disulfide-stabilized Fv-Fragment. *Proc. Natl. Acad. Sci. USA,* 1993, vol. 90, 7538-7542
- **Buchberger A ; Howard MJ ; Proctor M ; Bycroft M.** National Library of Medicine. *J Mol Bil.,* 16. Marz 2001, vol. 307 (1), 17-24
- **Carter, P. ; Kelley, R. F. ; Rodrigues, M. L. ; Snedecor, B. ; Covarrubias, M. ; Velligan, M. D. ; Wong, W. L. T. ; Rowland, Kotts, C. E. ; Carver, M. E. ; Yang, M.** Solvent-Accessible Surfaces of Proteins and Nucleic Acids. *Science,* 1983, vol. 221, 709-713
- **M. ; Henner, D.** High level Escherichia coli expression and production of a bivalent humanized antibody fragment. *Biotechnology,* 1992, vol. 10, 163-167
- **Daugherty, P. S. ; Chen, G. ; Olsen, M. J. ; Iverson, B. L. ; Georgiou, G.** Antibody affinity maturation using bacterial surface display. *Protein Eng.,* 1998, vol. 11, 825-832
- Recombinant Antibodies. **Dübel, S. ; Kontermann, R. E.** Antibody Engineering. Springer Verlag, 2001
- **Filippi, M. ; Tribioli, C. ; Toniolo, D.** Linkage and sequence conservation of the X-linked genes DX253 (P3) and DXS254E (GdX) in mouse and man. *Genomics,* 1990, vol. 7, 453-457
- **Griep, R. A. ; van Twisk, C. ; van der Wolf, J. M. ; Schots, A.** Fluobodies: green fluorescent single-chain Fv fusion proteins. *J. Immunol. Methods,* 1999, vol. 230, 121-130
- **Hanes, J. ; Jermutus, L. ; Weber-Bornhauser, S. ; Bosshard, H. R. ; Plückthun, A.** Ribosome display efficiently selects and evolves high-affinity antibodies in vitro from immune libraries. *Proc. Natl. Acad. Sci. USA,* 1998, vol. 95, 14130-14135

- **Hanes, J. ; Schaffitzel, C. ; Knappik, A. ; Plückthun, A.** Picomolar affinity antibodies from a fully synthetic naive library selected and evolved by ribosome display. *Nature Biotechnology,* 2000, vol. 18, 1287-1292
- **He, M. ; Taussig, M. J.** Antibody-ribosome-mRNA (ARM) complexes as efficient selection particles for in vitro display and evolution of antibody combining sites. *Nucleic Acids Res.,* 1997, vol. 25, 5132-5134
- **Holliger, P. ; Prospero, T. ; Winter, G.** ''Diabodies'': small bivalent and bispecific antibodies. *Proc. Natl. Sci. USA,* 1993, vol. 90, 6444-6448
- **Hoogenboom, H. R. ; de Bruine, A. P. ; Hufton, S. E. ; Hoet, R. M. ; Arends, J. W. ; Roovers, R. C.** Antibody phage display technology and its applications. *Immunotechnology,* 1998, vol. 4, 1-20
- **Jones, D. ; Candido, E. P.** Novel ubiquitin-like ribosomal protein fusion genes from the nematodes Caenorhabditis elegans and Caenorhabditis briggsae. *J. Biol. Chem.,* 1993, vol. 268, 19545-195451
- **Kieke, M. C. ; Cho, B. K. ; Boder, E. T. ; Kranz, D. M. ; Wittrup, K. D.** Isolation of anti-T cell receptor scFv mutants by yeast surface display. *Protein Eng.,* 1997, vol. 10, 1303-1310
- **Knappik, A. ; Ge, S. ; Honegger, A. ; Pack, P. ; Fischer, M. ; Wellnhofer, G. ; Hoess, A. ; Wölle, J. ; Plückthun, A. ; Virnekäs, B.** Fully synthetic human combinatorial antibody libraries (HuCAL) based on modular consensus frameworks and CDRs randomized with trinucleotides. *J. Mol. Biol.,* 2000, vol. 296, 57-86
- **Koide, A. ; Bailey, C. W. ; Huang, X. ; Koide, S.** The fibronectin type III domain as ascaffold for novel binding proteins. *J. Mol. Biol.,* 1998, vol. 284, 1141-1151
- **Kuchner, O. ; Arnold, F.H.** Directed evolution of enzyme catalysts. *TIBTECH,* 1997, vol. 15, 523-530
- **Kumar, S. ; Yoshida, Y. ; Noda, M.** Cloning of a cDNA which encodes a novel ubiquitin-like protein. *Biochem. Biophys. Res. Commun.,* 1993, vol. 195, 393-399
- **Larsen CN ; Wang H.** National Library of Medicine. *J Proteome Res.,* September 2002, vol. 1 (5), 411-9
- **Marx, J.** Ubiquitin lives up to its name. *Science,* 2002, vol. 297, 1792-1794
- **McConell S. ; Hoess R.H.** Tendamistat as a scaffold for conformationally constrained phage peptide libraries. *J. Mol. Biol.,* 1995, vol. 250, 460-470
- **Michiels, L. ; Van der Rauwelaert, E. ; Van Hasselt, F. ; Kas, K. ; Merregaert, J.** Fau cDNA encodes a ubiquitin-like-S30 fusion protein and is expressed as an antisense sequence in the Finkel-Biskis-Reilly murine sarcoma virus. *Oncogene,* 1993, vol. 8, 2537-2546
- **Miura, T. ; Klaus, W. ; Gsell, B. ; Miyamoto, C. ; Senn, H.** Characterization of the binding interface between Ubiquitin and class I human Ubiquitin-conjugating enzyme 2b by multidimensional heteronuclear NMR spectroscopy in solution. *J. Mol. Biol.,* 1999, vol. 290, 213-228
- **Muller, B.H. ; Chevrier, D. ; Boulain, J.-C ; Guesdon, J.-L.** Recombinant single-chain Fv antibody fragment-alkaline phosphatase conjugate for one-step immunodetection in molecular hybridization. *J. Immunol. Methods,* 1999, vol. 227, 177-185
- **Muller, S. ; Hoege, C. ; Pyrowolakis, G. ; Jentsch, S.** SUMO, ubiquitins mysterious cousin. *Nat. Rev. Mol. Cell Biol.,* 2001, vol. 2, 202-210
- **Murzin A. G. ; Brenner S. E. ; Hubbard T. ; Chothia C.** SCOP: a structural classification of proteins database for the investigation of sequences and structures. *J. Mol. Biol.,* 1995, vol. 247, 536-540
- **Nord K. ; Gunneriusson, E. ; Ringdahl, J. ; Stahl, S. ; Uhlen, M. ; Nygren, P.A.** Binding proteins selected from combinatorial libraries of an beta-helical bacterial receptor domain. *Nat. Biotechnol.,* 1997, vol. 8, 772-777
- **Odegrip, R. ; Coomber, D. ; Eldridge, B. ; Herderer, R. ; Kuhlman, P. A. ; Ullman, C. ; Fitz-Gerald, K. ; McGregor, D.** CIS display: In vitro selection ofpeptides from libraries ofprotein-DNA complexes. *PNAS,* 2003, vol. 101, 2806-2810
- **Pannekoek, H. ; van Meijer M. ; Schleef, R.R. ; Loskutoff, d.J. ; Barbas, C.F.** Functional display of human plasminogen-activator inhibitor 1 (PAI-1) on phages: Novel perspectives for structure-function analysis by error-prone DNA synthesis. *Gene,* 1993, vol. 128, 135-140
- **Reiter, Y. ; Pastan, I.** Recombinant Fv immunotoxins and Fv fragments as novel agents for cancer therapy and diagnosis. *Trends Biotechnol.,* 1998, vol. 16, 513-520
- **Sambrook, J. ; Maniatis, T. ; Fritsch, E.F.** Molecular Cloning: A laboratory manual. Cold Spring Harbour Laboratory Press, 1989
- **Sambrook, J. ; Fritsch, E. F. ; Maniatis, T.** Molecular Cloning: A Laboratory Manual. Cold Spring Harbor Laboratory Press, 2001
- **Shrake, A. ; Rupley, J.A.** Environment and Exposure to Solvent of Protein Atoms. Lysozyme and Insuline. *J. Mol. Biol.,* 1973, vol. 79, 351-371
- **Skerra, A. ; Plückthun, A.** Assembly of a functional immunoglobulin Fv fragment in Escherichia coli. *Science,* 1988, vol. 240, 1038-1041
- In vitro selection and evolution of protein-ligand interactions by ribosome display. **Schaffitzel, C. ; Zahnd, C. ; Amstutz, P. ; Luginbühl, B. ; Plückthun, A.** Protein-Protein Interactions, A Molecular Cloning Manual. Cold Spring Harbor Laboratory Press, 2001, 535-567

- **Skerra, A.** Engineered protein scaffolds for molecular recognition. *J. Mol. Recognit.,* 2000, vol. 13, 167-187
- **Stemmer, W.P.C.** Rapid evolution of a protein in vitro by DNA shuffling. *Nature,* 1994, vol. 370, 389-391
- **Vijay-Kumar, S. ; Bugg, C. E. ; Cook, W. J.** Structure ofUbiquitin refined at 1.8 A resolution. *J. Mol. Biol.,* 1987, vol. 194, 531-544
- **Winter, G.** Synthetic human antibodies and a strategy for protein engineering. *FEBS Lett.,* 1998, vol. 430, 92-94
- **Wintrode, P. L. ; Makhatadze, G. I. ; Privalov, P. L.** Thermodynamics ofUbiquitin unfolding. *Proteins Struct. Funct. Genet.,* 1994, vol. 18, 246-253